# EUROPEAN PATENT APPLICATION

(11) **EP 4 650 351 A1**
(43) Date of publication of application: **19.11.2025**
(21) Application number: 24741258.8
(22) Date of filing: 09.01.2024
(51) Int. Cl.: C07D 403/06, C07D 401/06, C07D 403/02, C07D 401/02, C07D 403/14, A61K 31/454, A61P 13/12

(54) **INHIBITOR OF COMPLEMENT FACTOR B**

(30) Priority: 09.01.2023 CN 202310025525
(71) Applicant: Nanjing Chia Tai Tianqing Pharmaceutical Co., Ltd., Nanjing, Jiangsu 210046 (CN)
(72) Inventor: MA, Changyou, Nanjing, Jiangsu 210046 (CN); SU, Jincai, Nanjing, Jiangsu 210046 (CN); LI, Xuefei, Nanjing, Jiangsu 210046 (CN); LIU, Jiang, Nanjing, Jiangsu 210046 (CN); ZHAO, Tingli, Nanjing, Jiangsu 210046 (CN); JI, Xiaojun, Nanjing, Jiangsu 210046 (CN); MIAO, Lei, Nanjing, Jiangsu 210046 (CN); WU, Jian, Nanjing, Jiangsu 210046 (CN); XU, Dan, Nanjing, Jiangsu 210046 (CN); ZHU, Chunxia, Nanjing, Jiangsu 210046 (CN)
(74) Representative: Elzaburu S.L.P.
(86) International application number: PCT/CN2024/071419
(87) International publication number: WO 2024/149261

(57) **Abstract**

Provided in the present disclosure is an inhibitor of the complement factor B. The structure of the inhibitor of the complement factor B is substantially as represented by general formula (I), and the definition of each substituent in the formula is as described in the description. The compound provided in the present invention has significant inhibitory activity on complement factor B, and can be used for preventing and/or treating a disease or disease state mediated by the inhibitor of the complement factor B.

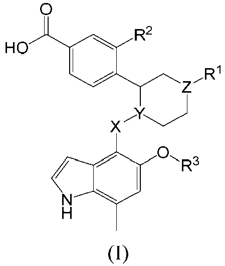

## Description

### TECHNICAL FIELD

The present disclosure belongs to the field of biomedicine and specifically relates to an inhibitor of complement factor B.

### BACKGROUND

The complement system is a part of the host's innate immune system involved in the lysis of foreign cells, enhancing antigen phagocytosis, agglutinating antigen carriers, and recruiting macrophages and neutrophils. It serves as a critical component of the innate immunity for the human body to resist infections by foreign pathogens, bacteria, parasites, etc., and meanwhile the complement system is also an important component connecting innate immunity and adaptive immunity. The complement is composed of Ife plasma proteins, including soluble proteins, membrane-bound proteins, and complement receptors, primarily produced by the liver or by membrane proteins expressed on cell surface, and functions in plasma, tissues, or cells. The complement system, an important regulator of inflammatory responses and tissue damage, is composed of more than 20 serum proteins and cell surface proteins. The complement system includes intrinsic complement components and various regulatory proteins. The intrinsic complement components include C1-C9, among which C3 has the highest content. The complement system is mainly activated through three pathways: the classical pathway (CP), the lectin pathway (LP), and the alternative pathway (AP).

In the normal physiological state of healthy individuals, the AP pathway remains in a low-level activated state to continuously monitor the invasion state of foreign pathogens. Complement proteins are distributed on the surface of apoptotic cells, and complement activation is strictly regulated, only used for clearing apoptotic cells without further activating other innate immune or adaptive immune responses. In the case of infection by foreign pathogens, the complement system is fully activated, generating inflammatory reactions, opsonization, phagocytosis, etc., destroying the pathogens and ultimately activating the adaptive immune response. Both inefficient complement function and excessive complement stimulation can be harmful to the human body, and are associated with an increased susceptibility to infections or non-communicable diseases. Complement dysfunction or excessive activation has been linked to certain autoimmune, inflammatory, and neurodegenerative diseases, as well as ischemia-reperfusion injury and cancer. For example, the activation of the alternative pathway of the complement cascade contributes to the production of C3a and C5a (both are potent anaphylatoxins), and C3a and C5a also play a role in many inflammatory diseases. Therefore, in some cases, it is desirable to reduce the response of the complement pathways (including the alternative complement pathway).

Complement factor B (Factor B, FB) is a key protein involved in the activation of AP. Inhibiting the activity of FB can prevent the activation of the AP pathway without interfering with CP and LP pathways, and can avoid the increased risk of infection due to the inhibition of the complement system. Therefore, searching for a novel and effective small-molecule inhibitor of complement factor B is an important direction in the current research on drug development for various diseases caused by complement abnormalities.

### SUMMARY

The purpose of the present disclosure is to provide a novel inhibitor compound of complement factor B or an isomer or a pharmaceutically acceptable salt thereof.

In an embodiment of the present disclosure, the present disclosure provides a compound represented by General Formula (I) or an isomer or a pharmaceutically acceptable salt thereof, wherein the structure of General Formula (I) is as follows: 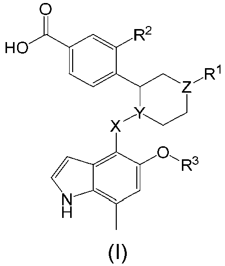 wherein:
X is selected from CH₂, NH, S, O or 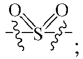
Y and Z are each independently selected from CH or N;
and when X is CH₂, Z is N;
R¹ is selected from C1-C6 alkyl, C1-C6 alkoxy, C3-C6 cycloalkyl or C3-C6 heterocyclic group, the C1-C6 alkyl or C3-C6 cycloalkyl is optionally substituted with halogen, oxo group, C1-C6 haloalkyl, C3-C6 cycloalkyl, C3-C6 heterocyclic group or C3-C6 halocycloalkyl;
R² is hydrogen, or R¹ and R² together with the atoms to which they are attached, form a C 10-15 heterocyclic group, the heterocyclic group is optionally substituted with halogen;
R³ is selected from C1-C6 alkyl or C3-C6 cycloalkyl, the C1-C6 alkyl is optionally substituted with deuterium or halogen.

In a preferred embodiment of the present disclosure, the compound represented by the General Formula (I) or isomer or pharmaceutically acceptable salt thereof is further represented by General Formula (II-1), (II-2) or (II-3): 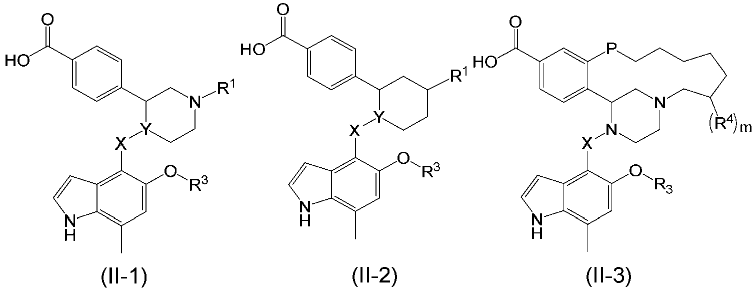 wherein:
X, Y, R¹, R³ are as defined above;
P is selected from CH₂, NH, S or O;
R⁴ is selected from hydrogen, halogen or C1-C3 alkyl;
m is 0, 1 or 2.

In a preferred embodiment of the present disclosure, the compound represented by the General Formula (I) or isomer or pharmaceutically acceptable salt thereof is further represented by General Formula (II-1-1) or (II-1-2): 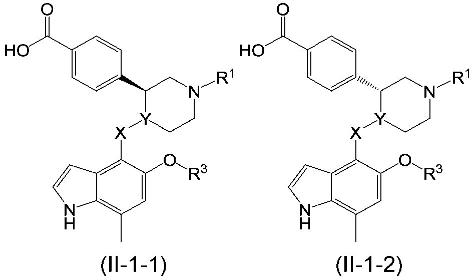 wherein:
X, Y, R¹, R³ are as defined above.

In a preferred embodiment of the present disclosure, the compound represented by the General Formula (I) or isomer or pharmaceutically acceptable salt thereof is further represented by General Formula (II-2-1) or (II-2-2): 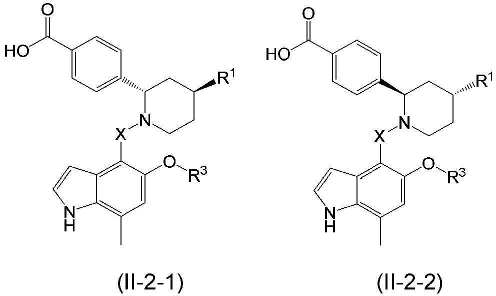 wherein:
X, Y, R¹, R³ are as defined above. In a further preferred embodiment of the present disclosure, Y is selected from CH or N; preferably CH.

In a preferred embodiment of the present disclosure, Y is selected from CH.

In a preferred embodiment of the present disclosure, Y is selected from N.

In a preferred embodiment of the present disclosure, the compound represented by the General Formula (I) or isomer or pharmaceutically acceptable salt thereof is further represented by General Formula (III-1) or (III-2): 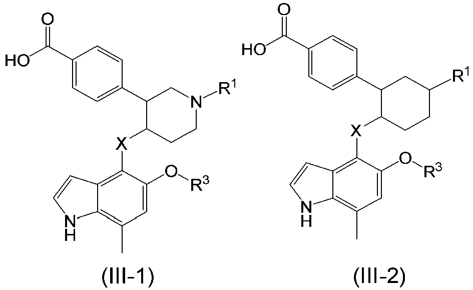 wherein:
X, R¹, R³ are as defined above.

In a preferred embodiment of the present disclosure, the compound represented by the General Formula (I) or isomer or pharmaceutically acceptable salt thereof is further represented by General Formula (III-1-1), (III-1-2), (III-1-3) or (III-1-4): 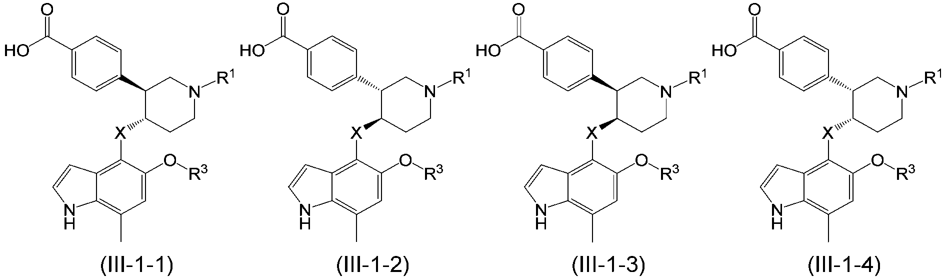 wherein:
X, R¹, R³ are as defined above.

In a preferred embodiment of the present disclosure, the compound represented by the General Formula (I) or isomer or pharmaceutically acceptable salt thereof is further represented by General Formula (III-2-1), (III-2-2), (III-2-3), (III-2-4), (III-2-5), (III-2-6), (III-2-7) or (III-2-8): 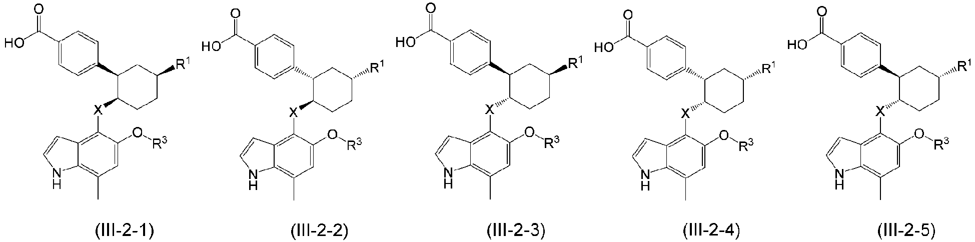 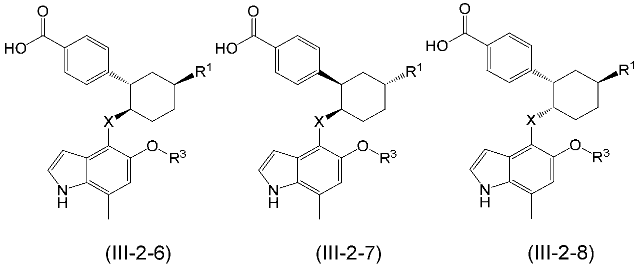 wherein:
X, R¹, R³ are as defined above.

In a further preferred embodiment of the present disclosure, X is selected from NH, S, O or

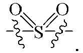

In a further preferred embodiment of the present disclosure, X is selected from CH₂, S or O; preferably S or O.

In a preferred embodiment of the present disclosure, X is selected from CH₂.

In a preferred embodiment of the present disclosure, X is selected from S.

In a preferred embodiment of the present disclosure, X is selected from O.

In a preferred embodiment of the present disclosure, X is selected from NH.

In a preferred embodiment of the present disclosure, X is selected from

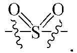

In a preferred embodiment of the present disclosure, Z is selected from CH or N.

In a preferred embodiment of the present disclosure, Z is selected from N.

In a preferred embodiment of the present disclosure, Z is selected from CH.

In a further preferred embodiment of the present disclosure, R¹ is selected from C1-C3 alkyl, C1-C3 alkoxy, C3-C4 cycloalkyl or C3-C4 heterocyclic group, the C1-C3 alkyl or C3-C4 cycloalkyl is optionally substituted with fluorine, chlorine, bromine, iodine, oxo group, C1-C3 haloalkyl, C3-C4 cycloalkyl, C3-C4 heterocyclic group or C3-C4 halocycloalkyl.

In a preferred embodiment of the present disclosure, R¹ is selected from C1-C3 alkyl, C1-C3 alkoxy, C3-C4 cycloalkyl or C3-C4 heterocyclic group, the C1-C3 alkyl or C3-C4 cycloalkyl is optionally substituted with 1, 2 or 3 substituents selected from fluorine, chlorine, bromine, iodine, oxo group, C1-C3 haloalkyl, C3-C4 cycloalkyl, C3-C4 heterocyclic group or C3-C4 halocycloalkyl.

In a preferred embodiment of the present disclosure, R¹ is selected from C1-C3 alkyl, the C1-C3 alkyl is optionally substituted with 1, 2 or 3 substituents selected from fluorine, chlorine, bromine, iodine.

In a preferred embodiment of the present disclosure, the C1-C3 fluoroalkyl is selected from fluoromethyl, difluoromethyl or trifluoromethyl, preferably trifluoromethyl.

In a preferred embodiment of the present disclosure, R¹ is selected from C3-C4 cycloalkyl, the C3-C4 cycloalkyl is optionally substituted with 1, 2 or 3 substituents selected from C1-C3 fluoroalkyl.

In a further preferred embodiment of the present disclosure, R¹ is selected from methyl, ethyl, propyl, ethoxy, cyclobutyl or oxetane, the ethyl, propyl or cyclobutyl is optionally substituted with fluorine, oxo group, trifluoromethyl, cyclopropyl, fluorocyclopropyl or oxetane.

In a further preferred embodiment of the present disclosure, R¹ is selected from methyl, ethyl, propyl, ethoxy, cyclobutyl or oxetane, the ethyl, propyl or cyclobutyl is optionally substituted with 1, 2 or 3 substituents selected from fluorine, oxo group, trifluoromethyl, cyclopropyl, fluorocyclopropyl or oxetane.

In a further preferred embodiment of the present disclosure, R¹ is selected from methyl, ethyl or propyl, the methyl, ethyl or propyl is optionally substituted with 1, 2 or 3 fluorines. In a further preferred embodiment of the present disclosure, R¹ is selected from 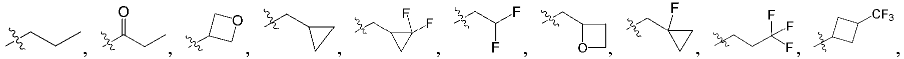 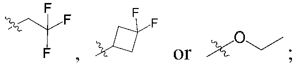 preferably 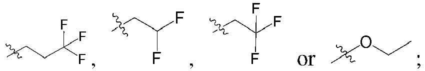 more preferably

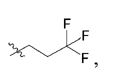

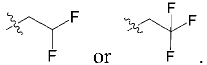

In a further preferred embodiment of the present disclosure, R¹ is selected from

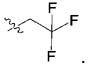

In a further preferred embodiment of the present disclosure, R³ is selected from C1-C3 alkyl or C3-C4 cycloalkyl, the C1-C3 alkyl is optionally substituted with deuterium, fluorine, chlorine, bromine or iodine.

In a further preferred embodiment of the present disclosure, R³ is selected from C1-C3 alkyl or C3-C4 cycloalkyl, the C1-C3 alkyl is optionally substituted with 1, 2 or 3 substituents selected from deuterium, fluorine, chlorine, bromine or iodine. In a further preferred embodiment of the present disclosure, R³ is selected from methyl or cyclopropyl, the methyl is optionally substituted with deuterium or fluorine.

In a further preferred embodiment of the present disclosure, R³ is selected from methyl or cyclopropyl, the methyl is optionally substituted with 1, 2 or 3 substituents selected from deuterium or fluorine.

In a further preferred embodiment of the present disclosure, R³ is selected from methyl, -CD₃, 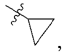 -CF₃ or 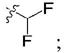 preferably methyl, -CD₃, -CF₃ or 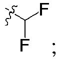 preferably methyl.

In a further preferred embodiment of the present disclosure, P is selected from CH₂, NH, S or O; preferably O.

In a further preferred embodiment of the present disclosure, R⁴ is selected from fluorine, chlorine, bromine or iodine; preferably fluorine.

In a further preferred embodiment of the present disclosure, R⁴ is selected from hydrogen.

In a further preferred embodiment of the present disclosure, m is 0, 1 or 2; preferably 2.

In a further preferred embodiment of the present disclosure, the compound or isomer or pharmaceutically acceptable salt thereof has the following structure: 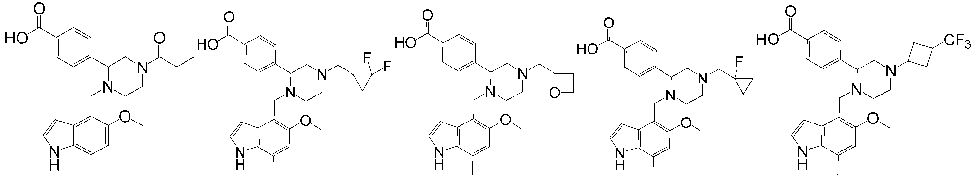 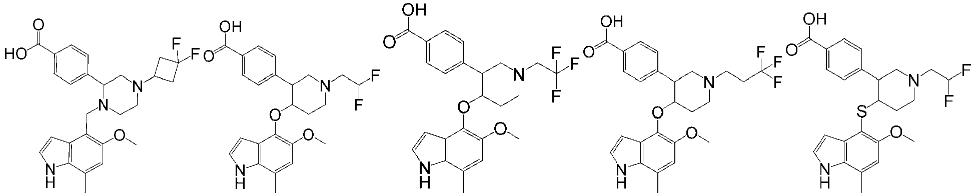 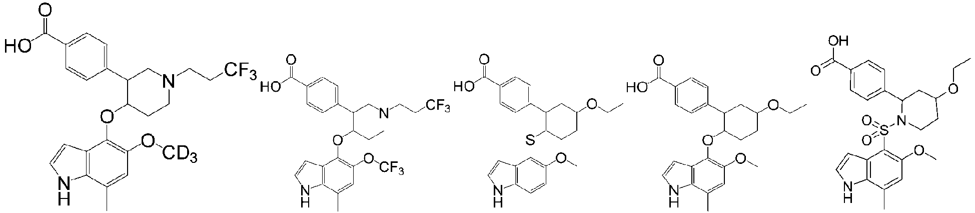 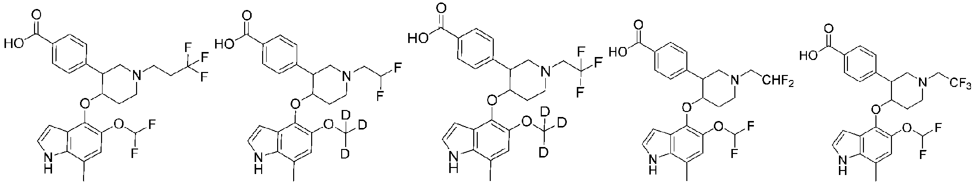 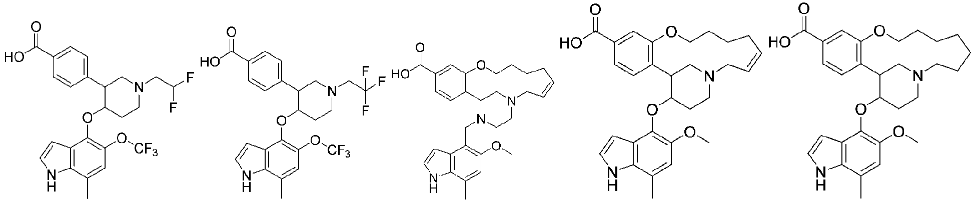 or

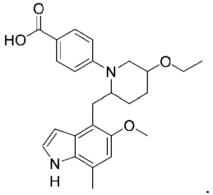

The present disclosure provides the following compound or isomer or pharmaceutically acceptable salt thereof:

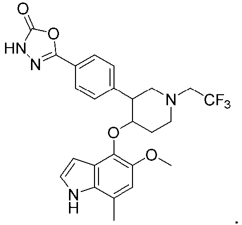

In a further preferred embodiment of the present disclosure, the compound or isomer or pharmaceutically acceptable salt thereof has the following structure: 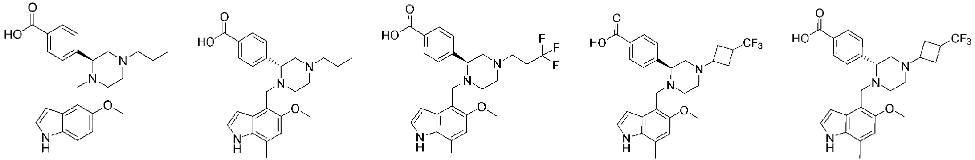 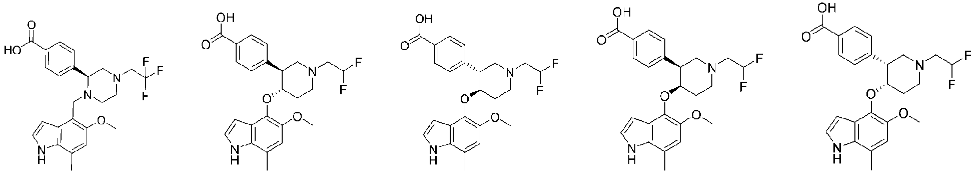 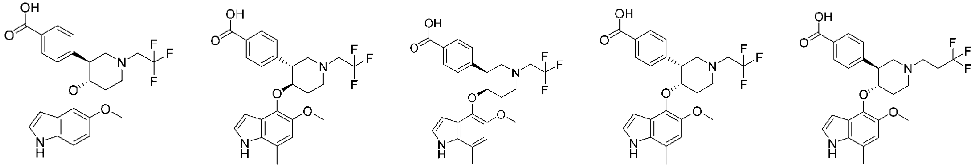 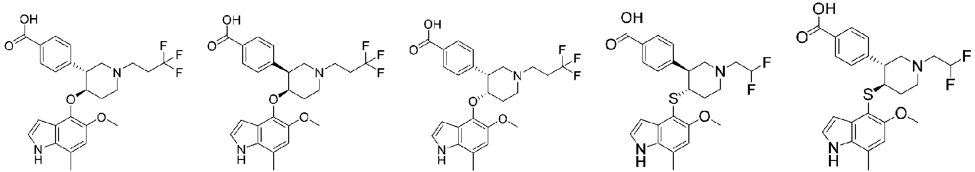 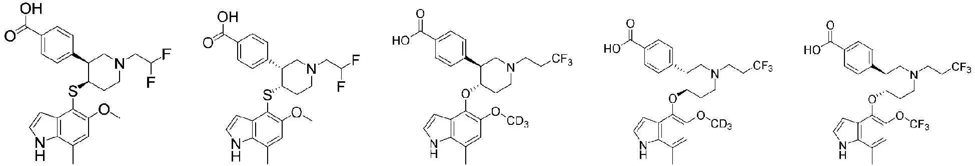 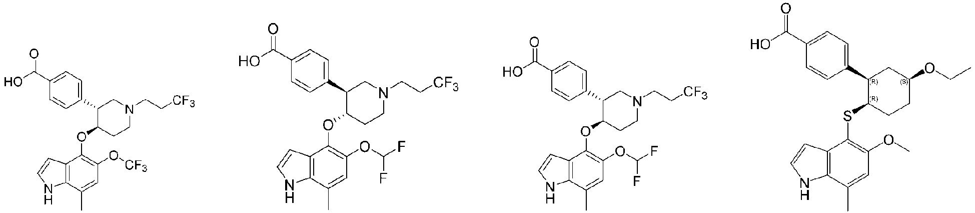 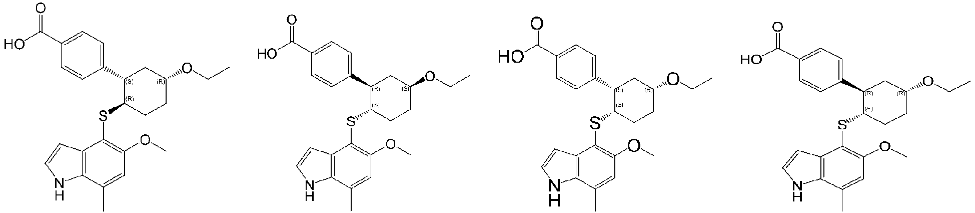 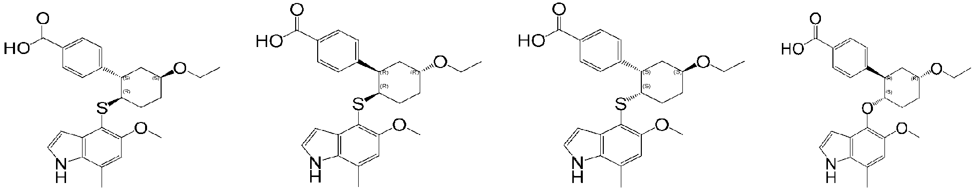 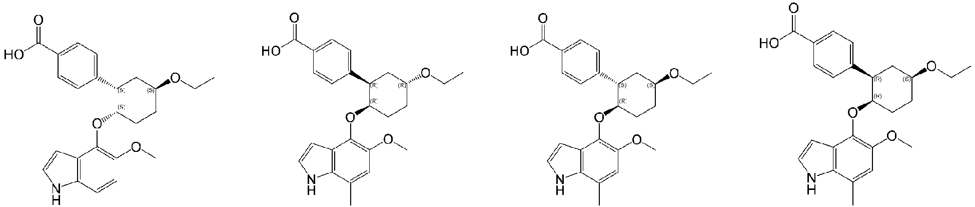 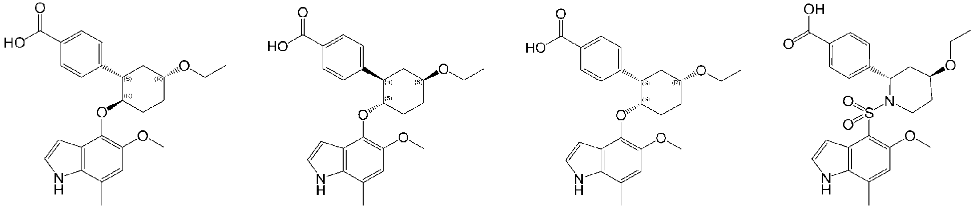 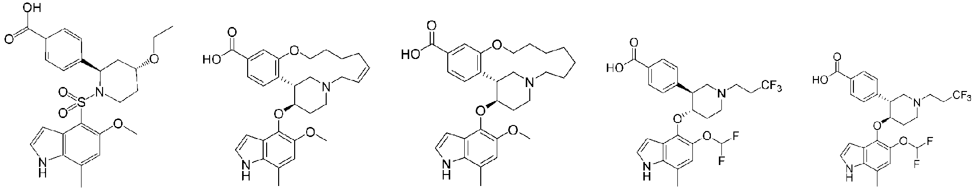 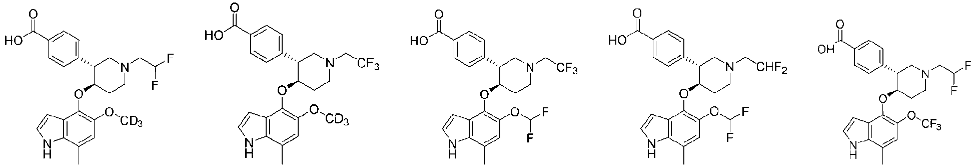 or

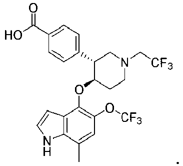

The present disclosure provides the following compound or isomer or pharmaceutically acceptable salt thereof:

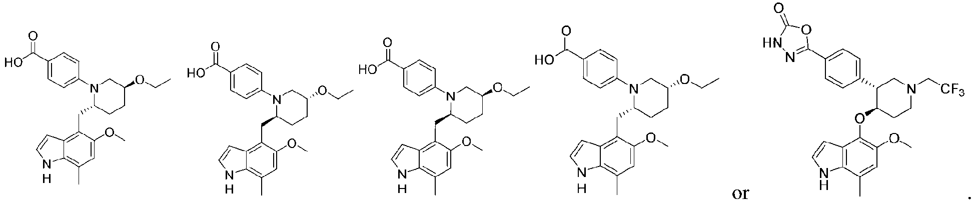

In a further preferred embodiment of the present disclosure, the compound or isomer or pharmaceutically acceptable salt thereof has the following structure:

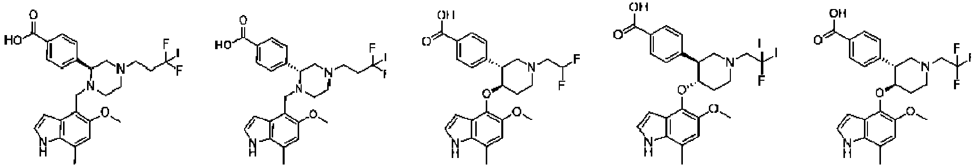

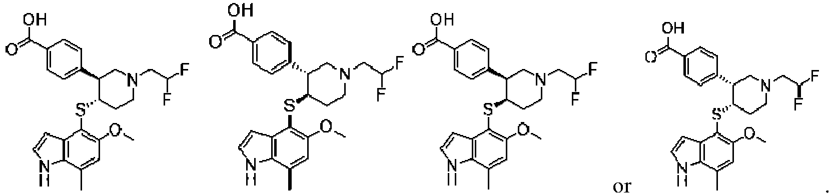

In a further preferred embodiment of the present disclosure, the compound or isomer or pharmaceutically acceptable salt thereof has the following structure:

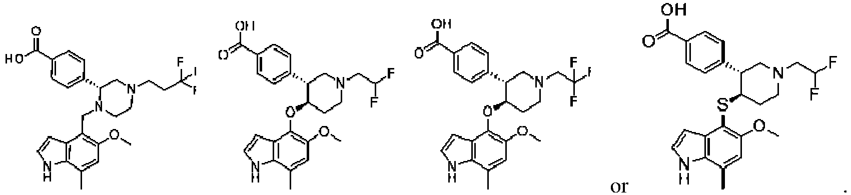

In a preferred embodiment of the present disclosure, the isomer of the compound " 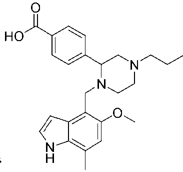 " is resolved by using chiral SFC, with a retention time of 2.06 min. The specific resolution method is as follows:
chromatographic column: Lux Cellulose-4 4.6*50 mm, 3 µm;
mobile phase A: supercritical CO₂; mobile phase B: methanol;
gradient: 10% - 50% flow for 2.0 minutes, maintained at 50% for 1.0 min;
detection wavelength: 220 nm.

In a preferred embodiment of the present disclosure, the isomer of the compound " 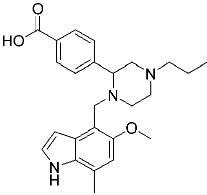 " is resolved by using chiral SFC, with a retention time of 2.34 min. The specific resolution method is as follows:
chromatographic column: Lux Cellulose-4 4.6*50 mm, 3 µm;
mobile phase A: supercritical CO₂; mobile phase B: methanol;
gradient: 10% - 50% flow for 2.0 minutes, maintained at 50% for 1.0 min;
detection wavelength: 220 nm.

In a preferred embodiment, the isomer of the compound " 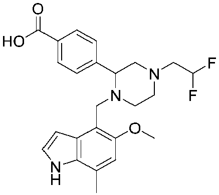 " is resolved by using chiral SFC, with a retention time of 4.90 min. The specific resolution method is as follows:
chromatographic column: DAICELCHIRALCEL^{®}OZ 4250*20 mm, 10 µm;
mobile phase A: supercritical CO₂; mobile phase B: methanol (+0.1% 7.0 mol/L ammonia in methanol );
flow rate: 70 mL/min;
mobile phase ratio: A: B = 75: 25;
detection wavelength: 214 nm.

In a preferred embodiment, the isomer of the compound " 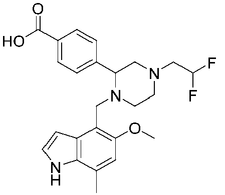 " is resolved by using chiral SFC, with a retention time of 6.20 min. The specific resolution method is as follows:
chromatographic column: DAICELCHIRALCEL^{®}OZ 4250*20 mm, 10 µm;
mobile phase A: supercritical CO₂; mobile phase B: methanol (+0.1% 7.0 mol/L ammonia in methanol);
mobile phase ratio: A: B = 75: 25;
detection wavelength: 214 nm.

In a preferred embodiment, the isomer of the compound " 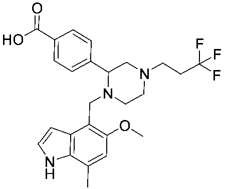 " is resolved by using chiral SFC, with a retention time of 2.31 min. The specific resolution method is as follows:
chromatographic column: CHIRALPAK IE-3 4.6*50 mm, 3 µm;
mobile phase A: (n-hexane:dichloromethane = 3:1)(0.1% diethylamine); mobile phase B: isopropanol; A:B = 80:20;
flow rate: 1.0 mL/min;
detection wavelength: dual-wavelength detection at 220/254.

In a preferred embodiment, the isomer of the compound " 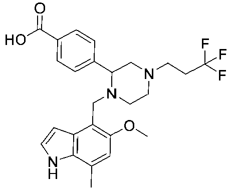 " is resolved by using chiral SFC, with a retention time of 3.57 min. The specific resolution method is as follows:
chromatographic column: CHIRALPAK IE-3 4.6*50 mm, 3 µm;
mobile phase A: (n-hexane:dichloromethane = 3:1)(0.1% diethylamine); mobile phase B: isopropanol; A:B = 80:20;
flow rate: 1.0 mL/min;
detection wavelength: dual-wavelength detection at 220/254.

In a preferred embodiment of the present disclosure, the isomer of the compound " 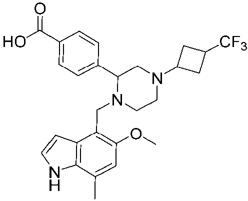 " is resolved by using chiral SFC, with a retention time of 2.12 min. The specific resolution method is as follows:
chromatographic column: DAICELCHIRALCEL^{®}OZ 250*20 mm, 10 µm;
mobile phase A: supercritical CO₂; mobile phase B: methanol (+0.1% 7.0 mol/L ammonia in methanol); A:B: 75:25;
flow rate: 70 mL/min;
detection wavelength: 214 nm.

In a preferred embodiment of the present disclosure, the isomer of the compound " 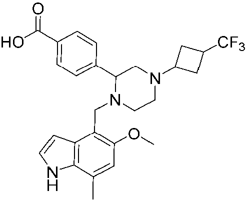 " is resolved by using chiral SFC, with a retention time of 3.03 min. The specific resolution method is as follows:
chromatographic column: DAICELCHIRALCEL^{®}OZ 250*20 mm, 10 µm;
mobile phase A: supercritical CO₂; mobile phase B: methanol (+0.1% 7.0 mol/L ammonia in methanol); A:B: 75:25;
flow rate: 70 mL/min;
detection wavelength: 214 nm.

In a preferred embodiment, the isomer of the compound " 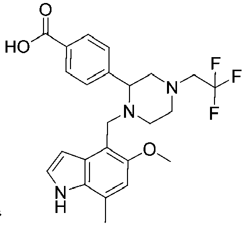 " is resolved by using chiral SFC, with a retention time of 1.08 min. The specific resolution method is as follows:
chromatographic column: DAICELCHIRALCEL^{®}OZ 250*20 mm, 10 µm;
mobile phase A: supercritical CO₂; mobile phase B: methanol (+0.1% 7.0 mol/L ammonia in methanol); A:B: 70:30;
flow rate: 100 mL/min;
detection wavelength: 214 nm.

In a preferred embodiment, the isomer of the compound " 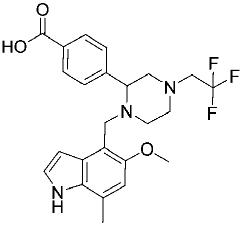 " is resolved by using chiral SFC, with a retention time of 2.24 min. The specific resolution method is as follows:
chromatographic column: DAICELCHIRALCEL^{®}OZ 250*20 mm, 10 µm;
mobile phase A: supercritical CO₂; mobile phase B: methanol (+0.1% 7.0 mol/L ammonia in methanol); A:B: 70:30;
flow rate: 100 mL/min;
detection wavelength: 214 nm.

In a preferred embodiment of the present disclosure, the isomer of the compound " 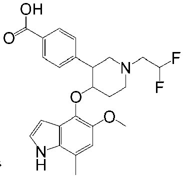 " is resolved by using chiral SFC, with a retention time of 1.19 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 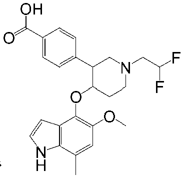 " is resolved by using chiral SFC, with a retention time of 1.95 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 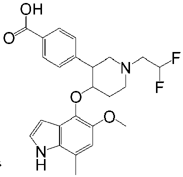 " is resolved by using chiral SFC, with a retention time of 1.29 min. The specific resolution method is as follows:
chromatographic column: DAICELCHIRALPAK^{®}IC 250*20 mm 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 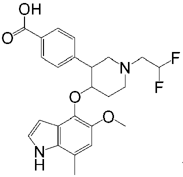 " is resolved by using chiral SFC, with a retention time of 4.00 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 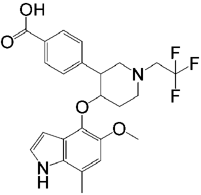 " is resolved by using chiral SFC, with a retention time of 1.87 min. The specific resolution method is as follows:
chromatographic column: DAICELCHIRALPAK^{®}OZ 250*25 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 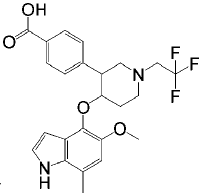 " is resolved by using chiral SFC, with a retention time of 2.73 min. The specific resolution method is as follows:
chromatographic column: DAICELCHIRALPAK^{®}OZ 250*25 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 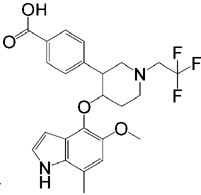 " is resolved by using chiral SFC, with a retention time of 1.06 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol ); A:B: 75:25;
flow rate: 100 mL/min;
detection wavelength: 214 nm.

In a preferred embodiment of the present disclosure, the isomer of the compound " 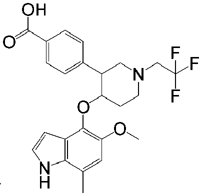 " is resolved by using chiral SFC, with a retention time of 2.37 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 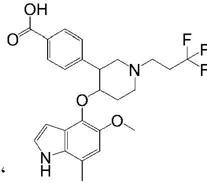 " is resolved by using chiral SFC, with a retention time of 1.25 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 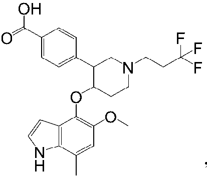 " is resolved by using chiral SFC, with a retention time of 2.00 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 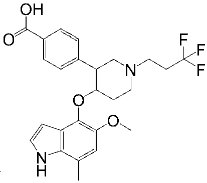 " is resolved by using chiral SFC, with a retention time of 0.91 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 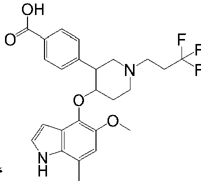 " is resolved by using chiral SFC, with a retention time of 2.35 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 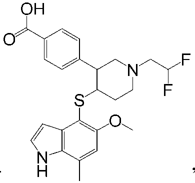 " is analyzed by using chiral SFC, with a retention time of 3.20 min. The specific analysis method is as follows:
chromatographic column: DAICELCHIRALCEL^{®}OZ 250*20 mm, 10 µm;
mobile phase A: supercritical CO₂; mobile phase B: methanol (+0.1% 7.0 mol/L ammonia in methanol); A:B: 70:30;
flow rate: 100 mL/min;
detection wavelength: 214 nm.

In a preferred embodiment of the present disclosure, the isomer of the compound " 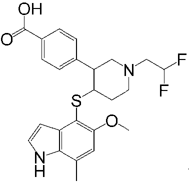 " is analyzed by using chiral SFC, with a retention time of 3.60 min. The specific analysis method is as follows:
chromatographic column: DAICELCHIRALCEL^{®}OZ 250*20 mm, 10 µm;
mobile phase A: supercritical CO₂; mobile phase B: methanol (+0.1% 7.0 mol/L ammonia in methanol); A:B: 70:30;
flow rate: 100 mL/min;
detection wavelength: 214 nm.

In a preferred embodiment of the present disclosure, the isomer of the compound " 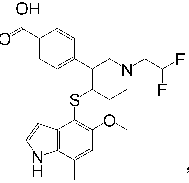 " is analyzed by using chiral SFC, with a retention time of 3.72 min. The specific analysis method is as follows:
chromatographic column: DAICELCHIRALCEL^{®}OZ 250*20 mm, 10 µm;
mobile phase A: supercritical CO₂; mobile phase B: methanol (+0.1% 7.0 mol/L ammonia in methanol); A:B: 70:30;
flow rate: 100 mL/min;
detection wavelength: 214 nm.

In a preferred embodiment of the present disclosure, the isomer of the compound " 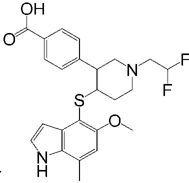 " is analyzed by using chiral SFC, with a retention time of 5.48 min. The specific analysis method is as follows:
chromatographic column: DAICELCHIRALCEL^{®}OZ 250*20 mm, 10 µm;
mobile phase A: supercritical CO₂; mobile phase B: methanol (+0.1% 7.0 mol/L ammonia in methanol ); A:B: 70:30;
flow rate: 100 mL/min;
detection wavelength: 214 nm.

In a preferred embodiment of the present disclosure, the isomer of the compound " 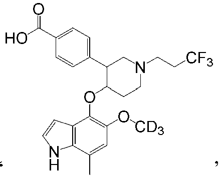 " is resolved by using chiral SFC, with a retention time of 0.88 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 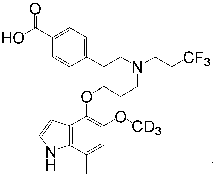 " is resolved by using chiral SFC, with a retention time of 1.39 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 75:25;
flow rate: 100 mL/min;
detection wavelength: 214 nm.

In a preferred embodiment of the present disclosure, the isomer of the compound " 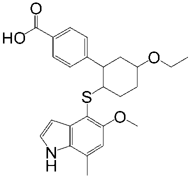 " is resolved by using chiral SFC, with a retention time of 5.45 min. The specific resolution method is as follows:
chromatographic column: DAICELCHIRALPAK^{®}IG 250*20 mm, 10 µm;
mobile phase A: supercritical CO₂; mobile phase B: methanol (+0.1% 7.0 mol/L ammonia in methanol); A:B: 50:50;
flow rate: 100 mL/min;
detection wavelength: 214 nm.

In a preferred embodiment of the present disclosure, the isomer of the compound " 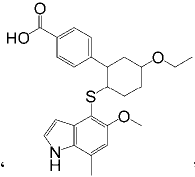 " is resolved by using chiral SFC, with a retention time of 6.03 min. The specific resolution method is as follows:
chromatographic column: DAICELCHIRALPAK^{®}IG 250*20 mm, 10 µm;
mobile phase A: supercritical CO₂; mobile phase B: methanol (+0.1% 7.0 mol/L ammonia in methanol); A:B: 50:50;
flow rate: 100 mL/min;
detection wavelength: 214 nm.

In a preferred embodiment of the present disclosure, the isomer of the compound " 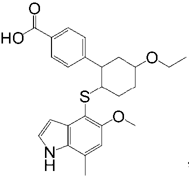 " is resolved by using chiral SFC, with a retention time of 5.33 min. The specific resolution method is as follows:
chromatographic column: DAICELCHIRALPAK^{®}IG 250*20 mm 10 µm;
mobile phase A: supercritical CO₂; mobile phase B: methanol (+0.1% 7.0 mol/L ammonia in methanol ); A:B: 50:50;
flow rate: 100 mL/min;
detection wavelength: 214 nm.

In a preferred embodiment of the present disclosure, the isomer of the compound " 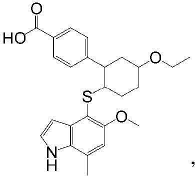 " is resolved by using chiral SFC, with a retention time of 7.81 min. The specific resolution method is as follows:
chromatographic column: DAICELCHIRALPAK^{®}IG 250*20 mm, 10 µm;
mobile phase A: supercritical CO₂; mobile phase B: methanol (+0.1% 7.0 mol/L ammonia in methanol); A:B: 50:50;
flow rate: 100 mL/min;
detection wavelength: 214 nm.

In a preferred embodiment of the present disclosure, the isomer of the compound " 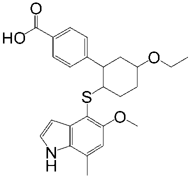 " is resolved by using chiral SFC, with a retention time of 5.14 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 55:45;
flow rate: 70 mL/min;
detection wavelength: 214 nm.

In a preferred embodiment of the present disclosure, the isomer of the compound " 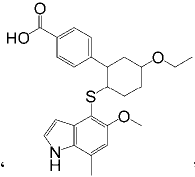 " is resolved by using chiral SFC, with a retention time of 5.73 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 55:45;
flow rate: 70 mL/min;
detection wavelength: 214 nm.

In a preferred embodiment of the present disclosure, the isomer of the compound " 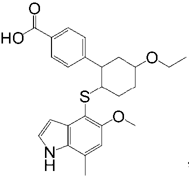 " is resolved by using chiral SFC, with a retention time of 5.20 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 55:45;
flow rate: 70 mL/min;
detection wavelength: 214 nm.

In a preferred embodiment of the present disclosure, the isomer of the compound " 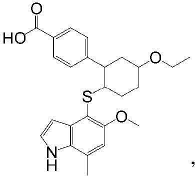 " is resolved by using chiral SFC, with a retention time of 6.30 min. The specific resolution method is as follows:
chromatographic column: DAICELCHIRALPAK^{®}IC 250*20 mm, 10 µm;
mobile phase A: supercritical CO₂; mobile phase B: methanol (+0.1% 7.0 mol/L ammonia in methanol); A:B: 55:45;
flow rate: 70 mL/min;
detection wavelength: 214 nm.

In a preferred embodiment of the present disclosure, the isomer of the compound " 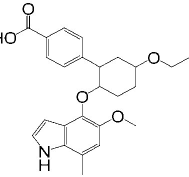 " is resolved by using chiral SFC, with a retention time of 4.70 min. The specific resolution method is as follows:
chromatographic column: DAICELCHIRALCEL^{®}AD 250*20 mm, 10 µm;
mobile phase A: supercritical CO₂; mobile phase B: methanol (+0.1% 7.0 mol/L ammonia in methanol); A:B: 70:30;
flow rate: 100 mL/min;
detection wavelength: 214 nm.
detection wavelength: 214 nm.

In a preferred embodiment of the present disclosure, the isomer of the compound " 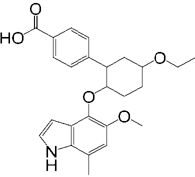 " is resolved by using chiral SFC, with a retention time of 4.80 min. The specific resolution method is as follows:
chromatographic column: DAICELCHIRALCEL^{®}AD 250*20 mm, 10 µm;
mobile phase A: supercritical CO₂; mobile phase B: methanol (+0.1% 7.0 mol/L ammonia in methanol); A:B: 70:30;
flow rate:100 mL/min;
detection wavelength: 214 nm.
detection wavelength: 214 nm.

In a preferred embodiment of the present disclosure, the isomer of the compound " 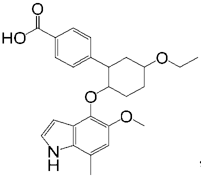 " is resolved by using chiral SFC, with a retention time of 3.43 min. The specific resolution method is as follows:
chromatographic column: DAICELCHIRALCEL^{®}AD 250*20 mm, 10 µm;
mobile phase A: supercritical CO₂; mobile phase B: methanol (+0.1% 7.0 mol/L ammonia in methanol); A:B: 70:30;
flow rate: 100 mL/min;
detection wavelength: 214 nm.
detection wavelength: 214 nm In a preferred embodiment of the present disclosure, the isomer of the compound " 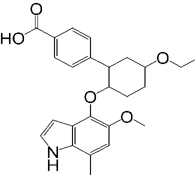 " is resolved by using chiral SFC, with a retention time of 3.59 min. The specific resolution method is as follows:
   chromatographic column: DAICELCHIRALCEL^{®}AD 250*20 mm, 10 µm;
   mobile phase A: supercritical CO₂; mobile phase B: methanol (+0.1% 7.0 mol/L ammonia in methanol); A:B: 70:30;
   flow rate: 100 mL/min;
   detection wavelength: 214 nm.

In a preferred embodiment of the present disclosure, the isomer of the compound " 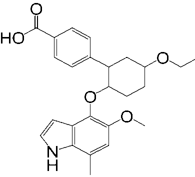 " is resolved by using chiral SFC, with a retention time of 0.89 min. The specific resolution method is as follows:
chromatographic column: DAICELCHIRALCEL^{®}AD 250*20 mm, 10 µm;
mobile phase A: supercritical CO₂; mobile phase B: methanol (+0.1% 7.0 mol/L ammonia in methanol); A:B: 75:25;
flow rate: 120 mL/min;
detection wavelength: 214 nm.

In a preferred embodiment of the present disclosure, the isomer of the compound " 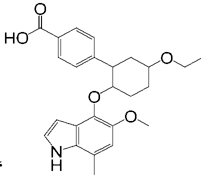 " is resolved by using chiral SFC, with a retention time of 1.22 min. The specific resolution method is as follows:
chromatographic column: DAICELCHIRALCEL^{®}AD 250*20 mm, 10 µm;
mobile phase A: supercritical CO₂; mobile phase B: methanol (+0.1% 7.0 mol/L ammonia in methanol); A:B: 75:25;
flow rate: 120 mL/min;
detection wavelength: 214 nm.
detection wavelength: 214 nm. In a preferred embodiment of the present disclosure, the isomer of the compound " 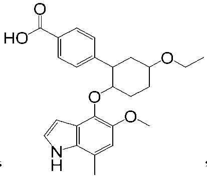 " is resolved by using chiral SFC, with a retention time of 0.92 min. The specific resolution method is as follows:
   chromatographic column: DAICELCHIRALCEL^{®}AD 250*20 mm, 10 µm;
   mobile phase A: supercritical CO₂; mobile phase B: methanol (+0.1% 7.0 mol/L ammonia in methanol); A:B: 75:25;
   flow rate: 120 mL/min;
   detection wavelength: 214 nm.
   detection wavelength: 214 nm. In a preferred embodiment of the present disclosure, the isomer of the compound " 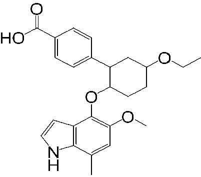 " is resolved by using chiral SFC, with a retention time of 1.46 min. The specific resolution method is as follows:
      chromatographic column: DAICELCHIRALCEL^{®}AD 250*20 mm, 10 µm;
      mobile phase A: supercritical CO₂; mobile phase B: methanol (+0.1% 7.0 mol/L ammonia in methanol); A:B: 75:25;
      flow rate: 120 mL/min;
      detection wavelength: 214 nm.

In a preferred embodiment of the present disclosure, the isomer of the compound " 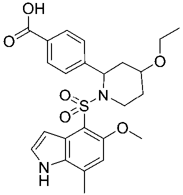 " is resolved by using chiral SFC, with a retention time of 1.34 min. The specific resolution method is as follows:
chromatographic column: DAICELCHIRALCEL^{®}AD 250*20 mm, 10 µm;
mobile phase A: supercritical CO₂; mobile phase B: methanol (+0.1% 7.0 mol/L ammonia in methanol); A:B: 55:45;
flow rate: 70 mL/min;
detection wavelength: 214 nm.

In a preferred embodiment of the present disclosure, the isomer of the compound " 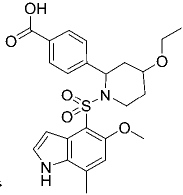 " is resolved by using chiral SFC, with a retention time of 2.68 min. The specific resolution method is as follows:
chromatographic column: DAICELCHIRALCEL^{®}AD 250*20 mm, 10 µm;
mobile phase A: supercritical CO₂; mobile phase B: methanol (+0.1% 7.0 mol/L ammonia in methanol); A:B: 55:45;
flow rate: 70 mL/min;
detection wavelength: 214 nm.

In a preferred embodiment of the present disclosure, the isomer of the compound " 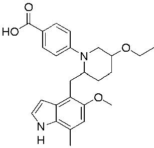 " is resolved by using chiral SFC, with a retention time of 5.47 min. The specific resolution method is as follows:
chromatographic column: CHIRALPAK-IG 2*25cm;
mobile phase A: n-hexane (0.1% formic acid); mobile phase B: ethanol:dichloromethane = 1:1; A:B = 1:1;
flow rate: 20 mL/min;
detection wavelength: 220/254nm.

In a preferred embodiment of the present disclosure, the isomer of the compound " 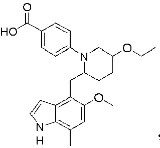 " is resolved by using chiral SFC, with a retention time of 7.57 min. The specific resolution method is as follows:
chromatographic column: CHIRALPAK-IG 2*25cm;
mobile phase A: n-hexane (0.1% formic acid); mobile phase B: ethanol:dichloromethane = 1:1; A:B = 1:1;
flow rate: 20 mL/min;
detection wavelength: 220/254nm.

In a preferred embodiment of the present disclosure, the isomer of the compound " 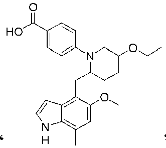 " is resolved by using chiral SFC, with a retention time of 10.6 min. The specific resolution method is as follows:
chromatographic column: CHIRALPAK-IG 2*25cm;
mobile phase A: n-hexane (0.1% formic acid); mobile phase B: ethanol:dichloromethane = 1:1; A:B = 1:1;
flow rate: 20 mL/min;
detection wavelength: 220/254nm.

In a preferred embodiment of the present disclosure, the isomer of the compound " 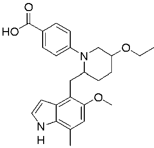 " is resolved by using chiral SFC, with a retention time of 15.2. min. The specific resolution method is as follows:
chromatographic column: CHIRALPAK-IG 2*25cm;
mobile phase A: n-hexane (0.1% formic acid); mobile phase B: ethanol:dichloromethane = 1:1; A:B = 1:1;
flow rate: 20 mL/min;
detection wavelength: 220/254nm.

An intermediate for preparing the aforementioned compound or salt thereof, which has the following structure:

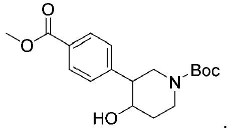

In a preferred embodiment of the present disclosure, the isomer of the intermediate " 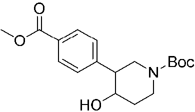 " is resolved by using chiral SFC, with a retention time of 20.33 min. The specific resolution method is as follows:
chromatographic column: DAICELCHIRALPAK^{®}IC 250*4.6mm, 5 µm;
mobile phase A: n-hexane; mobile phase B: ethanol; A:B: 90:10;
flow rate: 1 mL/min;
detection wavelength: 254nm.

In a preferred embodiment of the present disclosure, the isomer of the intermediate " 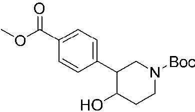 " is resolved by using chiral SFC, with a retention time of 16.51 min. The specific resolution method is as follows:
chromatographic column: DAICELCHIRALPAK^{®}IC 250*4.6mm, 5 µm;
mobile phase A: n-hexane; mobile phase B: ethanol; A:B: 90:10;
flow rate: 1 mL/min;
detection wavelength: 254nm.

In a preferred embodiment of the present disclosure, the present disclosure further provides a pharmaceutical composition, comprising a therapeutically effective amount of any of the aforementioned compound or isomer or pharmaceutically acceptable salt thereof, and one or more pharmaceutically acceptable carriers.

The pharmaceutical composition of the present disclosure further can be administered by any suitable route or method, such as oral or parenteral (e.g., intravenous) administration. For oral administration, the pharmaceutical compositions of the present disclosure are typically provided in the form of tablets, capsules, or solutions. Tablets can contain the compound of the present disclosure or isomer or pharmaceutically acceptable salt thereof, and pharmaceutically acceptable carriers. The carriers include, but are not limited to, diluents, disintegrants, binders, lubricants, colorants, or preservatives. Capsules include hard capsules and soft capsules.

For parenteral administration, the pharmaceutical compositions of the present disclosure can be administered by intravenous, intramuscular, or subcutaneous injection. They are typically provided as sterile aqueous solutions or suspensions or lyophilized powders, with appropriate pH and isotonicity adjusted accordingly.

In a preferred embodiment of the present disclosure, the present disclosure further provides a use of any one of the aforementioned compound or isomer or pharmaceutically acceptable salt thereof in the manufacture of a medicament for preventing and/or treating a disease or a disease state mediated by complement factor B.

In a preferred embodiment of the present disclosure, the present disclosure further provides a method for preventing and/or treating a disease or disease state mediated by complement factor B, comprising administering to a subject in need thereof the compound of the present disclosure or isomer or pharmaceutically acceptable salt thereof, or the pharmaceutical composition of the present disclosure.

In a preferred embodiment of the present disclosure, the disease or disease state mediated by complement factor is selected from one or more of an ophthalmic disease, an autoimmune disease (including arthritis), a disease related to the renal system, a disease of respiratory system, and a cardiovascular disease.

In a preferred embodiment of the present disclosure, the disease or disease state mediated by complement factor is arthritis.

### RELEVANT DEFINITIONS

As used herein and unless otherwise specified, the terms "comprise", "include", "have", "contain" and their grammatical equivalents shall generally be interpreted as open-ended and non-limiting, e.g., not excluding other unrecited elements or steps.

Unless otherwise specified, the following terms used in the description and claims have the following meanings:
The "compounds" of the present disclosure can be asymmetric, such as having one or more chiral centers. Unless otherwise specified, the "compounds" of the present disclosure can be any isomer or a mixture of two or more isomers. The "compounds" of the present disclosure include isomers (such as stereoisomers), enantiomers, diastereomers, racemates, or mixtures of two or more isomers of the compounds.

The term "isomer" refers to different compounds that have the same molecular formula but differ in the arrangement of atoms and configuration. An "enantiomer" is a pair of stereoisomers that are non-superimposable mirror images of each other. A 1:1 mixture of a pair of enantiomers is a "racemic" mixture, and where appropriate, the term is used to refer to such a racemic mixture. The use of "rel" indicates that the diastereomeric orientation is known, but the absolute stereochemistry is not known. For example, the designation "rel-3S, 4S" as used herein indicates that the relative stereochemistry at positions 3 and 4 is either 3S, 4S or 3R, 4R. In the present disclosure, the order of the positions on the alicyclic ring is as follows: 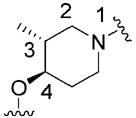 . The absolute stereochemistry has not been determined, but the optical rotation and/or the chiral chromatographic conditions will reveal which isomer is present. A "diastereoisomer" is a stereoisomer having at least two asymmetric atoms and which is not a mirror image of another stereoisomer. The absolute stereochemistry is specified according to the Cahn-Ingold-Prelog R-S system. When a compound is a pure enantiomer, the stereochemistry at each chiral carbon can be described using R or S. Compounds whose absolute configuration is undetermined after resolution can be designated as (+) or (-) according to the direction (right-handed or left-handed) in which they rotate plane-polarized light at the wavelength of the sodium D-line or according to their retention time during separation by chiral chromatography. Some of the compounds described herein contain one or more asymmetric centers or axes, and thus can generate enantiomers, diastereomers, and other stereoisomeric forms that can be defined in terms of absolute stereochemistry as (R)- or (S)- or represented by the symbols (+) or (-). The present disclosure includes all such possible isomers, including racemic mixtures, optically pure forms, and intermediate mixtures. Optically active (R)- and (S)-isomers can be prepared with chiral synthons or chiral reagents, or resolved by conventional techniques. If a compound contains a double bond, the substituents can be an E or Z configuration. If a compound contains a disubstituted cycloalkyl group, the cycloalkyl substituents can have a cis- or trans-configuration. That is, the compounds of the present disclosure include, but are not limited to, cis and trans isomers, (-)- and (+)-enantiomers, (R)- and (S)-enantiomers, diastereoisomers, (D)-isomers, (L)-isomers, and racemic mixtures thereof and other mixtures. The compounds of the present disclosure containing asymmetric carbon atoms can be isolated in the form of optically active pure forms or mixtures of two or more isomers. Optically active pure forms can be resolved from mixtures of two or more isomers, or synthesized by using chiral starting materials or chiral reagents.

The "compounds" of the present disclosure further include tautomeric forms. Tautomeric forms arise from the exchange of a single bond with an adjacent double bond accompanied by the migration of a proton. The terms "tautomer" or "tautomeric form" refer to different functional group isomers that exist in dynamic equilibrium and can rapidly interconvert at room temperature.

Those skilled in the art can separate the isomer mixtures obtainable according to the present disclosure into individual isomers by known methods; diastereomers can be separated, for example, by distribution between polyphasic solvent mixtures, recrystallization, and/or chromatographic separation such as silica gel chromatography or by medium-pressure liquid chromatography, for example, using a reversed-phase column, and racemates can be separated, for example, by forming salts with optically pure salt-forming reagents and separating mixtures of diastereomers thus obtained (for example, using fractional crystallization for separation) or by performing chromatographic processing on optically active column materials to separate. The intermediates and final products can be post-treated and/or purified according to standard methods, such as by using chromatography, distribution methods, (re)crystallization, etc. At all reaction stages, the formed isomer mixtures can be separated into individual isomers, such as diastereomers or enantiomers, or separated into any desired isomer mixtures, such as racemates or diastereomeric mixtures.

Provide absolute stereochemistry and/or optical rotation as appropriate for the embodiments of the present disclosure. The present disclosure focuses on all stereochemical forms of the compounds provided herein. In some cases, the compounds contain two or more chiral centers. The relative stereochemistry of these compounds is identified through NMR studies and/or X-ray diffraction. In such cases, the prefix "rel" is used, followed by the identification of these compounds using the R/S nomenclature. Obviously, when "rel" is used, R/S only provides relative stereochemical information (for example, trans or cis), and does not represent absolute stereochemistry. In some cases, the relative stereochemistry of the diastereomeric pairs has not been determined. Therefore, when only one isomer has been isolated and/or is available, the enantiomers are labeled/distinguished according to the retention times under the given HPLC conditions. Identical samples typically have the same retention time, but there may be certain operational errors. When samples obtained by those of ordinary skill in the art using corresponding methods are tested with the same instruments and detection methods, the retention time error is usually within ±0.2 min, preferably within ±0.1 min. Different technicians using different instruments may accidentally exhibit minor retention time errors exceeding this range, such as errors within ±0.5 min; or within ±0.3 min; or within or ±0.2 min, which shall all be considered as the same substance. Therefore, retention time errors within ±0.5 min, ±0.3 min, ±0.2 min or ±0.1 min can all be interpreted as falling within the protection scope of the present disclosure.

In cases where each diastereomer is identified as racemic but the relative stereochemistry has not been determined, if only one isomer has been isolated and/or is available, the compound is designated with the symbol "(±)" and the names "diastereomer-1" or "diastereomer-2".

In the present disclosure, " 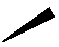 " and " 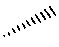 " are used to represent the absolute configuration of a stereocenter. The " 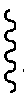 " in " 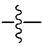 " refers to the bond connection position. When " 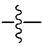 " appears in a bicyclic or polycyclic structure and the connection position is uncertain, it indicates that the connection sites are limited to any atom on the single ring where " 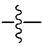 " is located, as long as the valence of the atom permits.

The structures of the compounds of the present disclosure can be confirmed by conventional methods well-known to those skilled in the art. If the present disclosure relates to the absolute configuration of the compound, the absolute configuration can be verified by conventional technical methods in the art, such as single crystal X-ray diffraction (SARD).

The term "optional" or "optionally" refers to the subsequently described event or circumstance may occur or may not occur, and the description includes the occurrence and non-occurrence of the event or circumstance.

The term "halogen" refers to fluorine, chlorine, bromine, and iodine.

The term "substituted" refers to any one or more (e.g., 1, 2, 3, or 4, etc.) hydrogen atoms on a specific group being substituted with substituents, the substituents may be the same or different, provided that the valence of the specific group is normal and the substituted compound is stable. For example, "substituted by halogen" refers to any one or more (e.g., 1, 2, 3, or 4, etc.) hydrogen atoms on a specific group being substituted with the same or different halogens, provided that the valence of the specific group is normal and the substituted compound is stable.

The numerical ranges herein refer to each integer within the given range. For example, "C1-C6" refers to the group having 1 carbon atom, 2 carbon atoms, 3 carbon atoms, 4 carbon atoms, 5 carbon atoms, or 6 carbon atoms; "C1-C3" refers to the group having 1 carbon atom, 2 carbon atoms, or 3 carbon atoms.

The term "alkyl" refers to a saturated aliphatic hydrocarbon group, including straight-chain or branched-chain saturated hydrocarbon groups, the hydrocarbon group has the indicated number of carbon atoms. For example, the term "C1-C6 alkyl" includes C1 alkyl, C2 alkyl, C3 alkyl, C4 alkyl, C5 alkyl, and C6 alkyl. Examples include, but are not limited to, methyl, ethyl, n-propyl, isopropyl, n-butyl, isobutyl, tert-butyl, n-pentyl, 2-pentyl, 3-pentyl, n-hexyl, 2-hexyl, 3-hexyl, etc.

The term "haloalkyl" refers to one or more hydrogen atoms in an alkyl are substituted with halogen atoms (fluorine, chlorine, bromine, iodine). For example, the term "C1-C6 haloalkyl" refers to a haloalkyl having 1-6 carbon atoms. Examples include, but are not limited to, trifluoromethyl and trifluoroethyl.

The term "cycloalkyl" refers to a monocyclic saturated hydrocarbon system without heteroatoms or double bonds. Examples of the term "C3-C6 cycloalkyl" include, but are not limited to, cyclopropyl, cyclobutyl, cyclopentyl, and cyclohexyl.

The term "heterocyclic group" refers to saturated or partially unsaturated monocyclic or polycyclic cyclic hydrocarbon substituents. The term "C3-C6 membered heterocyclic group" contains 3 to 6 carbon atoms as ring atoms, wherein one or more heteroatoms selected from nitrogen, oxygen, or sulfur are also included as ring atoms, preferably containing 1-3 heteroatoms. The term "C10-15 heterocyclic group" contains 10 to 15 carbon atoms as ring atoms, wherein one or more heteroatoms selected from nitrogen, oxygen, or sulfur are also included as ring atoms, preferably containing 1-3 heteroatoms; preferably includes 11 or 12 carbon atoms as ring atoms and contains 1 or 2 heteroatoms selected from oxygen or nitrogen as ring atoms.

The term "pharmaceutically acceptable" refers to a carrier, vehicle, excipient, diluent, and/or formed salt that is ordinarily chemically or physically compatible with the other components that constitute a pharmaceutical dosage form and physiologically compatible with the recipient.

The term "pharmaceutically acceptable carrier" refers to those carriers that do not cause significant irritation to the body and do not impair the biological activity and properties of the active compound. These include but are not limited to any diluents, disintegrants, binders, glidants, and wetting agents commonly used in the art for humans or animals.

The term "pharmaceutically acceptable salt" refers to salts that retain the biological efficacy of the free acids and bases of a specific compound without biological adverse effects. For example, acid (including organic acids and inorganic acids) addition salts or base (including organic bases and inorganic bases) addition salts, can also include zwitterionic salts, and also include quaternary ammonium salts, such as alkyl ammonium salts. The pharmaceutically acceptable salts of the present disclosure can be synthesized from parent compounds containing acid radicals or basic groups by conventional chemical methods. In general, the preparation method of such salts is: preparing by reacting these compounds in the form of free acids or bases with a stoichiometric amount of suitable bases or acids in water, organic solvents, or a mixture of both.

The term "effective amount" or "therapeutically effective amount" refers to a non-toxic but sufficient amount of a drug or pharmaceutical agent to achieve the desired effect. The exact dosage will vary depending on various factors, such as subject-dependent variables (e.g., age, immune system health, etc.), the disease or illness, and the treatment administered.

The term "pharmaceutical composition" refers to a composition comprising the compounds of the present disclosure or pharmaceutically acceptable salt thereof, and at least one component selected from the following pharmaceutically acceptable components, depending on the administration method and the nature of the dosage form, including but not limited to: carriers, diluents, adjuvants, excipients, preservatives, fillers, disintegrants, wetting agents, emulsifiers, suspending agents, sweeteners, flavoring agents, fragrances, antibacterial agents, antifungal agents, lubricants, dispersants, temperature-sensitive materials, temperature regulators, adhesives, stabilizers, suspending aids, etc.

The drugs or pharmaceutical compositions of the present disclosure can be administered orally, locally, parenterally, or mucosally (e.g., sublingually, by inhalation, or rectally) in dosage unit formulations comprising conventional non-toxic pharmaceutically acceptable carriers. An oral route of administration is generally preferred. The active agents can be administered orally in the form of capsules, tablets, etc.

The drugs or pharmaceutical compositions of the present disclosure can be delivered parenterally, i.e., by intravenous (i.v.), intracerebroventricular (i.c.v.), subcutaneous (s.c.), intraperitoneal (i.p.), intramuscular (i.m.), subdermal (s.d.), or intradermal (i.d.) administration, by direct injection such as bolus injection or continuous infusion. Formulations for injection can be presented in unit dosage form, such as in ampoules or multi-dose containers with added preservatives. The compositions can take the form of excipients, suspensions, solutions, or emulsions in oily or aqueous carriers, and can contain formulating agents such as antisettling agents, stabilizers, and/or dispersing agents. Alternatively, the active ingredient can be in powder form to be reconstituted with a suitable carrier (e.g., sterile pyrogen-free water) before use.

The drugs or pharmaceutical compositions of the present disclosure can also be formulated for rectal administration, for example, in the form of suppositories or retention enemas (for example, including conventional suppository bases such as cocoa butter or other glycerides).

The term "treatment" includes inhibiting, alleviating, preventing, or eliminating one or more symptoms or side effects associated with the disease, condition or disorder being treated.

The abbreviations used in the claims and the description have the following meanings:
M: mol/L
DMSO: dimethyl sulfoxide
DMSO-d6: deuterated dimethyl sulfoxide
DEA: diethylamine
DCM: dichloromethane
IPA: isopropanol
FA: formic acid
¹H NMR: proton nuclear magnetic resonance spectroscopy
HPLC: high performance liquid chromatography
LCMS: liquid chromatography-mass spectrometry
M: molarity (mol/L)
MEOH: methanol
m/z: mass-to-charge ratio
SFC: supercritical fluid chromatography
V/V: volume/volume

### BRIEF DESCRIPTION OF THE FIGURES

Figure 1 is the arthritis score graph for Example 13a-2 in the collagen antibody-induced mouse arthritis experiment of Experimental Example 4;
Figure 2 is the paw pad thickness change graph for Example 13a-2 in the collagen antibody-induced mouse arthritis experiment of Experimental Example 4;
Figure 3 is the AUC change graph for the efficacy study in the mouse CAIA model of Experimental Example 5;
Figure 4 is the crystal structure of the compound 4-((3R,4R)-1-(2,2-difluoroethyl)-4-((5-methoxy-7-methyl-1H-indol-4 -yl)oxy)piperidin-3-yl)benzoic acid in Experimental Example 6;
Figure 5 is the crystal structure of the compound 4-((3R,4R)-4-((5-methoxy-7-methyl-1H-indol-4-yl)oxy)-1-(2,2,2-trifluoroethyl)piperidin-3-yl)benzoic acid in Experimental Example 7;
Figure 6 is the crystal structure of the compound (3S,4R)-tert-butyl 4-hydroxy-3-(4-(methoxycarbonyl)phenyl)piperidine-1-carboxylate in Experimental Example 8.

### DETAILED DESCRIPTION OF THE EMBODIMENTS

The preparation methods of the compounds of the present disclosure are described in more detail below, but these specific preparation methods do not constitute any limitation to the scope of the present disclosure. Additionally, reaction conditions such as reactants, solvents, bases, amounts of compounds used, reaction temperature, reaction time, etc., are not limited to the examples below.

The compounds of the present disclosure can also be conveniently prepared by optionally combining various synthetic methods described in the specification or known in the art, and such combinations can be readily carried out by those skilled in the art.

Unless otherwise specified, raw materials and equipment used in the specific embodiments of the present disclosure are known products and can be obtained by purchasing commercially available products.

### Example 1: (S)-4-(1-((5-methoxy-7-methyl-1H-indol-4-yl)methyl)-4-propylpiperazin-2-yl)benzoic acid and (R)-4-(1-((5-methoxy-7-methyl-1H-indol-4-yl)methyl)-4-propylpiperazin-2-yl)benzoic acid

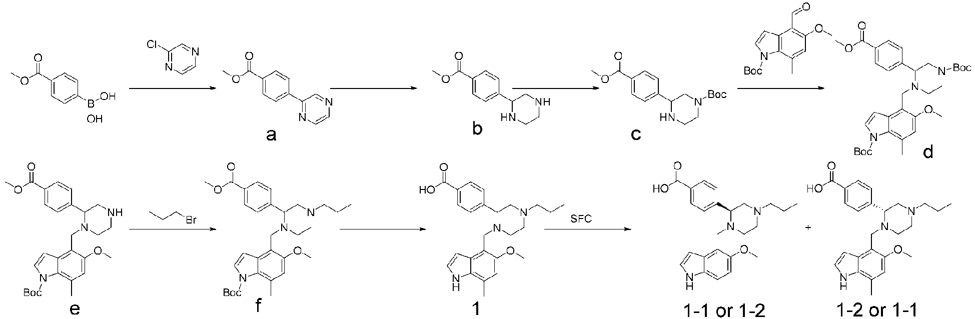

### a) Preparation of methyl 4-(pyrazin-2-yl)benzoate

(4-(Methoxycarbonyl)phenyl)boronic acid (2 g), 1,4-dioxane (100 mL), and water (20 mL) were added to a two-necked flask (250 mL). Then sodium carbonate (3.53 g) and tetrakis(triphenylphosphine)palladium (1.28 g) were added. The mixture was purged with nitrogen for protection. Then 2-chloropyrazine (1.91 g) was added, and the mixture was stirred at 100 °C for 4.5 hours. The reaction solution was cooled to room temperature and filtered. The filtrate was concentrated under reduced pressure to yield a powdery residue, which was purified by column chromatography (mobile phase: dichloromethane/methanol = 10/1 (V/V)) to obtain 2.04 g of the title compound.

### b) Preparation of (±)methyl 4-(piperazin-2-yl)benzoate

Methyl 4-(pyrazin-2-yl)benzoate (1 g) was dissolved in a mixed solvent of acetic acid/methanol (28 mL/14 mL). Palladium acetate (105 mg) and palladium on carbon (350 mg) were added. The mixture was purged with hydrogen then stirred overnight at room temperature. The mixture was filtered through diatomaceous earth, and the filtrate was evaporated to dryness to obtain 0.9 g of the title compound.

### c) Preparation of (±)tert-butyl 3-(4-(methoxycarbonyl)phenyl)piperazine-1-carboxylate

(±)Methyl 4-(piperazin-2-yl)benzoate (480 mg) was added to a 50 mL two-necked flask. Dichloromethane (30 mL) and triethylamine (661.54 mg) were added. The mixture was purged with nitrogen for protection. Then di-tert-butyl dicarbonate (475.59 mg) was added, and the mixture was stirred at room temperature for 1 hour. After quenching with water, the mixture was extracted with ethyl acetate. The organic phase was washed with saturated brine, dried over anhydrous sodium sulfate, and filtered. The filtrate was concentrated under reduced pressure to yield a powdery residue, which was purified by column chromatography (mobile phase: dichloromethane/methanol = 20/1 (V/V)) to obtain 300 mg of the title compound.

### d) Preparation of (±)tert-butyl 4-((4-(tert-butoxycarbonyl)-2-(4-(methoxycarbonyl)phenyl)piperazin-1-yl)methyl)-5-methoxy-7-methyl-1H-indole-1-carboxylate

Tert-butyl 4-formyl-5-methoxy-7-methyl-1H-indole-1-carboxylate (270.9 mg) was added to a 50 mL two-necked flask. 1,2-Dichloroethane (10 mL) was added, followed by the addition of (±)tert-butyl 3-(4-(methoxycarbonyl)phenyl)piperazine-1-carboxylate (300 mg). The mixture was purged with nitrogen for protection. The mixture was cooled to 0 °C in an ice-water bath, and sodium triacetoxyborohydride (396.9 mg) was added. The mixture was stirred overnight at room temperature. After quenching with water, the mixture was extracted with ethyl acetate. The organic phase was washed with saturated brine, dried over anhydrous sodium sulfate, and filtered. The filtrate was concentrated under reduced pressure to yield a powdery residue, which was purified by column chromatography (mobile phase: petroleum ether/ethyl acetate = 10/1 (V/V)) to obtain 380 mg of the title compound.

### e) Preparation of (±)tert-butyl 5-methoxy-4-((2-(4-(methoxycarbonyl)phenyl)piperazin-1-yl)methyl)-7-methyl-1H-indole-1-carboxylate

(±)Tert-butyl 4-((4-(tert-butoxycarbonyl)-2-(4-(methoxycarbonyl)phenyl)piperazin-1-yl)methyl)-5-methoxy-7-methyl-1H-indole-1-carboxylate (150 mg) was added to a 25 mL two-necked flask. Methanol (5 mL) and a 4 M solution of hydrogen chloride in 1,4-dioxane (5 mL) were added. The mixture was purged with nitrogen for protection. The mixture was stirred at room temperature for 2 h. The mixture was concentrated under reduced pressure to obtain 120 mg of the title compound.

### f) Preparation of (±)tert-butyl 5-methoxy-4-((2-(4-(methoxycarbonyl)phenyl)-4-propylpiperazin-1-yl)methyl)-7-methyl -1H-indole-1-carboxylate

(±)Tert-butyl 5-methoxy-4-((2-(4-(methoxycarbonyl)phenyl)piperazin-1-yl)methyl)-7-methyl-1H-indole-1-carboxylate (70 mg) was added to a 15 mL sealed tube. Acetonitrile (8 mL), 1-bromopropane (52 mg), and triethylamine (86.1 mg) were added. The mixture was stirred at 50 °C overnight. The reaction solution was concentrated under reduced pressure to yield a powdery residue, which was purified by column chromatography (mobile phase: dichloromethane/methanol = 20/1 (V/V)) to obtain 40 mg of the title compound.

### g) Preparation of (±)-4-(1-((5-methoxy-7-methyl-1H-indol-4-yl)methyl)-4-propylpiperazin-2-yl)benzoic acid

(±)Tert-butyl 5-methoxy-4-((2-(4-(methoxycarbonyl)phenyl)-4-propylpiperazin-1-yl)methyl)-7-methyl-1H-indole-1-carboxylate (40 mg) was dissolved in a mixed solvent of dichloromethane/methanol (2mL/2mL). A 1 N aqueous solution of lithium hydroxide (1 mL) was added. The mixture was stirred at 70 °C for 1 hour. The reaction solution was directly concentrated by rotary evaporation. After dissolving the residue in water, the pH was adjusted to neutral with dilute hydrochloric acid. The mixture was extracted with ethyl acetate. The organic phase was dried over anhydrous sodium sulfate and filtered. The filtrate was concentrated under reduced pressure to yield a powdery residue, which was purified by column chromatography (mobile phase: dichloromethane/methanol = 20/1 (V/V)) to obtain 9 mg of the title compound.

¹HNMR (400 MHz, DMSO-*d6*) δ 10.82 (s, 1H), 7.96 (s, 2H), 7.62 (s, 2H), 7.25 (t, *J =* 2.7 Hz, 1H), 6.65 (s, 1H), 6.44 (s, 1H), 3.70 (s, 3H), 3.56 (d, *J* = 11.9 Hz, 2H), 3.25 - 3.13 (m, 2H), 2.70 (t, *J* = 9.2 Hz, 2H), 2.41 (s, 3H), 2.18 (dd, *J =* 18.8, 11.5 Hz, 2H), 1.98 (d, *J =* 7.5 Hz, 2H), 1.87 (t, *J =* 10.9 Hz, 1H), 1.37 (dt, *J =* 12.0, 6.1 Hz, 2H), 0.85 - 0.80 (m, 3H).

LCMS m/z = 422.2 [M+1]⁺

The enantiomers of (±)4-(1-((5-methoxy-7-methyl-1H-indol-4-yl)methyl)-4-propylpiperazin-2-yl)benzoic acid were resolved by chiral SFC, obtaining 1-1, tᵣ = 2.06 min and 1-2, tᵣ = 2.34 min. (The specific resolution method was as follows: chromatographic column: Lux Cellulose-4 4.6*50 mm, 3 µm; mobile phase A: supercritical CO₂; mobile phase B: methanol; gradient: from 10% to 50% flow for 2.0 minutes, then maintain at 50% for 1.0 minute; detection wavelength: 220 nm)

1-1, tᵣ = 2.06 min

¹HNMR (400 MHz, DMSO-*d6*) δ 10.82 (s, 1H), 7.97 (d, J = 8.3 Hz, 2H), 7.69 (d, J = 6.7 Hz, 2H), 7.25 (t, J = 2.7 Hz, 1H), 6.66 (s, 1H), 6.53 - 6.35 (m, 1H), 3.71 (s, 3H), 3.56 (d, J = 11.8 Hz, 1H), 3.38 (s, 1H), 3.23 (d, J = 11.8 Hz, 1H), 2.71 (t, J = 10.0 Hz, 2H), 2.63 (d, J = 11.4 Hz, 1H), 2.42 (s, 3H), 2.26 - 2.10 (m, 3H), 1.93 (dt, J = 21.0, 10.9 Hz, 2H), 1.47 - 1.30 (m, 2H), 0.82 (t, J = 7.3 Hz, 3H).

1-2, tᵣ = 2.34 min

¹HNMR (400 MHz, DMSO-*d6*) δ 10.82 (s, 1H), 7.97 (d, J = 8.3 Hz, 2H), 7.69 (d, J = 6.7 Hz, 2H), 7.25 (s, 1H), 6.66 (s, 1H), 6.52 - 6.35 (m, 1H), 3.71 (s, 3H), 3.56 (d, J = 11.8 Hz, 1H), 3.39 (d, J = 10.4 Hz, 1H), 3.23 (d, J = 11.8 Hz, 1H), 2.71 (t, J = 10.2 Hz, 2H), 2.63 (d, J = 11.4 Hz, 1H), 2.42 (s, 3H), 2.28 - 2.09 (m, 3H), 1.93 (dt, J = 21.2, 11.1 Hz, 2H), 1.46 - 1.29 (m, 2H), 0.82 (t, J = 7.3 Hz, 3H).

### Example 2: (±)4-(1-((5-methoxy-7-methyl-1H-indol-4-yl)methyl)-4-propionylpiperazin-2-yl)benzoic acid

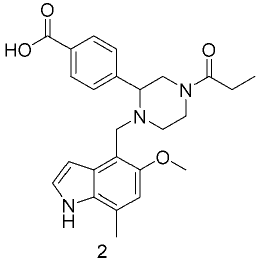

It was prepared by referring to the preparation method of Example 1, and 1-bromopropane in step f) was replaced with propionyl chloride.

¹HNMR (400 MHz, DMSO-*d6*) δ 12.94 (s, 1H), 10.85 (s, 1H), 8.01 (d, J = 6.4 Hz, 2H), 7.73 (dd, J = 16.2, 7.7 Hz, 2H), 7.27 (s, 1H), 6.67 (s, 1H), 6.42 (d, J = 8.7 Hz, 1H), 4.26 (s, 1H), 3.72 (s, 3H), 3.57 (s, 2H), 3.23 (d, J = 11.9 Hz, 2H), 3.04 - 2.92 (m, 1H), 2.67 - 2.61 (m, 2H), 2.43 (s, 3H), 2.26 (s, 1H), 1.99 (s, 2H), 0.98 (t, J = 7.4 Hz, 3H).

LCMS m/z = 436.2 [M+1]⁺

### Example 3: (±)-4-(1-((5-methoxy-7-methyl-1H-indol-4-yl)methyl)-4-(oxetan-3-yl)piperazin-2-yl)benzoic acid

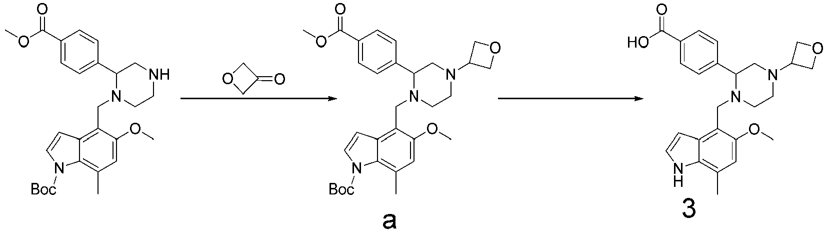

### a) Preparation of (±)tert-butyl 5-methoxy-4-((2-(4-(methoxycarbonyl)phenyl)-4-(oxetan-3-yl)piperazin-1-yl)methyl)-7-methyl-1H-indole-1-carboxylate

(±)Tert-butyl 5-methoxy-4-((2-(4-(methoxycarbonyl)phenyl)piperazin-1-yl)methyl)-7-methyl-1H-indole-1-carboxylate (70 mg) was dissolved in 1,2-dichloroethane (8 mL) and added to a 25 mL two-necked flask. Sodium triacetoxyborohydride (120.2 mg) was then added. The mixture was cooled to 0 °C in an ice bath. The mixture was purged with nitrogen for protection. Oxetan-3-one (20.44 mg) was added, and the mixture was stirred overnight. The reaction solution was concentrated under reduced pressure to yield a powdery residue, which was purified by column chromatography (mobile phase: dichloromethane/methanol = 20/1 (V/V)) to obtain 50 mg of the title compound.

### b) Preparation of (±)4-(1-((5-methoxy-7-methyl-1H-indol-4-yl)methyl)-4-(oxetan-3-yl)piperazin-2-yl)benzoic acid

(±)Tert-butyl 5-methoxy-4-((2-(4-(methoxycarbonyl)phenyl)-4-(oxetan-3-yl)piperazin-1-yl)methyl)-7-methyl-1H-indole-1-carboxylate (50 mg) was dissolved in a mixed solution of tetrahydrofuran/methanol (2 mL/2 mL). A 1 N aqueous solution of lithium hydroxide (1 mL) was added. The mixture was stirred at 70 °C for 3 hours. The reaction solution was directly concentrated by rotary evaporation. After dissolving the residue in water, the pH was adjusted to neutral with dilute hydrochloric acid. The mixture was extracted with ethyl acetate. The organic phase was dried over anhydrous sodium sulfate, and filtered. The filtrate was concentrated under reduced pressure to yield a powdery residue, which was purified by column chromatography (mobile phase: dichloromethane/methanol = 20/1 (V/V)) to obtain 22 mg of the title compound.

¹HNMR (400 MHz, DMSO-*d6*) δ 10.83 (s, 1H), 7.97 (d, *J =* 8.0 Hz, 2H), 7.68 (d, *J =* 6.1 Hz, 2H), 7.25 (s, 1H), 6.66 (s, 1H), 6.43 (s, 1H), 4.51 - 4.43 (m, 2H), 4.40 (t, *J =* 5.8 Hz, 2H), 3.71 (s, 3H), 3.58 (d, *J =* 11.8 Hz, 2H), 3.24 (d, *J* = 11.8 Hz, 2H), 2.67 (s, 1H), 2.66 - 2.55 (m, 2H), 2.42 (s, 3H), 2.24 (t, *J* = 10.6 Hz, 1H), 1.92 - 1.81 (m, 2H).

LCMS m/z = 436.2 [M+1]⁺.

### Example 4: (±)4-(4-(cyclopropylmethyl)-1-((5-methoxy-7-methyl-1H-indol-4-yl)methyl)piperazin-2-yl)benzoic acid

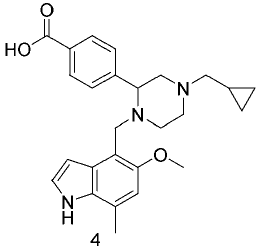

It was prepared by referring to the preparation method of Example 1, and 1-bromopropane in step f) was replaced with (bromomethyl)cyclopropane.

¹HNMR (400 MHz, DMSO-*d6*) δ 10.82 (s, 1H), 7.97 (d, *J =* 8.2 Hz, 2H), 7.69 (s, 2H), 7.26 (s, 1H), 6.66 (s, 1H), 6.45 (s, 1H), 3.71 (s, 3H), 3.56 (d, *J =* 11.8 Hz, 1H), 3.23 (d, *J =* 11.7 Hz, 1H), 2.84 (dd, *J =* 19.7, 10.8 Hz, 2H), 2.64 (d, *J =* 11.5 Hz, 1H), 2.42 (s, 3H), 2.23 (t, *J =* 10.7 Hz, 1H), 2.14 (d, *J =* 6.4 Hz, 2H), 1.99 (dt, *J =* 22.0, 11.1 Hz, 2H), 1.24 (s, 1H), 0.78 (s, 1H), 0.40 (d, *J* = 7.8 Hz, 2H), 0.04 (d, *J =* 7.8 Hz, 2H).

LCMS m/z = 434.3 [M+1]⁺.

### Example 5: (±)4-(4-((2,2-difluorocyclopropyl)methyl)-1-((5-methoxy-7-methyl-1H-indol-4-yl)methyl)piperazin-2 -yl)benzoic acid

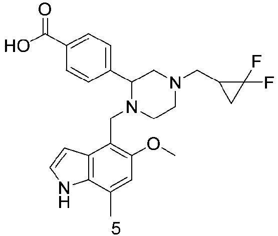

It was prepared by referring to the preparation method of Example 1, and 1-bromopropane in step f) was replaced with 2-(bromomethyl)-1,1-difluorocyclopropane.

¹HNMR (400 MHz, DMSO-*d6*) δ 12.87 (s, 1H), 10.84 (s, 1H), 7.98 (d, J = 7.7 Hz, 2H), 7.71 (s, 2H), 7.26 (t, J = 2.5 Hz, 1H), 6.66 (s, 1H), 6.45 (d, J = 2.0 Hz, 1H), 3.72 (s, 3H), 3.57 (d, J = 11.1 Hz, 1H), 3.41 (s, 1H), 3.24 (d, J = 11.8 Hz, 2H), 2.78 (d, J = 15.2 Hz, 2H), 2.66 (d, J = 11.2 Hz, 1H), 2.43 (s, 3H), 2.37-2.15 (m, 2H), 2.15-1.93 (m, 2H), 1.79 (d, J = 6.3 Hz, 1H), 1.60-1.46 (m, 1H), 1.12 (s, 1H).

LCMS m/z = 470.3 [M+1]⁺.

### Example 6: (S)-4-(4-(2,2-difluoroethyl)-1-((5-methoxy-7-methyl-1H-indol-4-yl)methyl)piperazin-2-yl)benzoic acid and (R)-4-(4-(2,2-difluoroethyl)-1-((5-methoxy-7-methyl-1H-indol-4-yl)methyl)piperazin-2-yl)benzoic acid

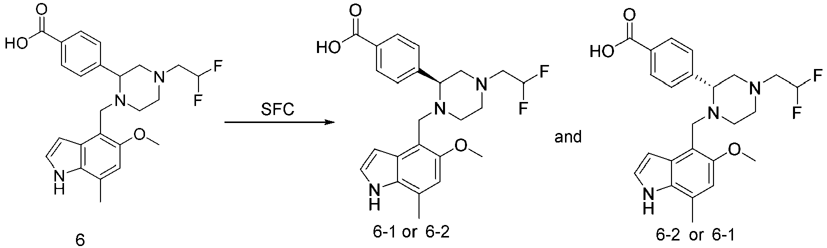

It was prepared by referring to the preparation method of Example 1, and 1-bromopropane in step f) was replaced with 1,1-difluoro-2-iodoethane.

¹HNMR (400 MHz, DMSO-*d6*) δ 10.83 (s, 1H), 7.97 (d, J = 8.2 Hz, 2H), 7.68 (d, J = 6.5 Hz, 2H), 7.25 (t, J = 2.7 Hz, 1H), 6.66 (s, 1H), 6.47 - 6.40 (m, 1H), 6.26 - 5.96 (m, 1H), 3.71 (s, 3H), 3.65 - 3.48 (m, 1H), 3.41 (d, J = 10.6 Hz, 1H), 3.22 (d, J = 11.9 Hz, 1H), 2.78 (dd, J = 21.1, 10.5 Hz, 2H), 2.69 - 2.57 (m, 3H), 2.42 (s, 3H), 2.26 (d, J = 10.9 Hz, 3H).

LCMS m/z = 444.2 [M+1]⁺.

The enantiomers of (±)4-(4-(2,2-difluoroethyl)-1-((5-methoxy-7-methyl-1H-indol-4-yl)methyl)piperazin-2-yl)benzoic acid were resolved by chiral SFC, obtaining 6-1, tᵣ = 4.90 min and 6-2, tᵣ = 6.20 min. (The resolution method: chromatographic column: DAICELCHIRALCEL^{®}OZ 4250*20 mm, 10 µm; mobile phase A: supercritical CO₂; mobile phase B: methanol (+0.1% 7.0 mol/L ammonia in methanol); flow rate: 70 mL/min; mobile phase ratio: A: B = 75: 25; detection wavelength: 214 nm).

6-1, tᵣ = 4.90 min

¹HNMR (400 MHz, DMSO-*d6*) δ 10.82 (s, 1H), 7.96 (d, J = 8.0 Hz, 2H), 7.65 (s, 2H), 7.25 (t, J = 2.7 Hz, 1H), 6.66 (s, 1H), 6.43 (s, 1H), 6.31 - 5.91 (m, 1H), 3.71 (s, 3H), 3.57 (d, J = 12.0 Hz, 1H), 3.40 (s, 1H), 3.22 (d, J = 11.8 Hz, 1H), 2.78 (dd, J = 21.5, 10.3 Hz, 2H), 2.72 - 2.58 (m, 3H), 2.42 (s, 3H), 2.30 - 2.17 (m, 3H).

LCMS m/z = 444.2 [M+1]⁺.

6-2, tᵣ = 6.20 min

¹HNMR (400 MHz, DMSO-d6) δ12.90 (s, 1H), 10.83 (s, 1H), 7.99 (d, J = 8.4 Hz, 2H), 7.70 (d, J = 7.1 Hz, 2H), 7.26 (t, J = 2.7 Hz, 1H), 6.66 (s, 1H), 6.44 (dd, J = 2.8, 2.0 Hz, 1H), 6.11 (tt, J = 55.7, 4.3 Hz, 1H), 3.71 (s, 3H), 3.57 (d, J = 11.8 Hz, 1H), 3.42 (d, J = 8.2 Hz, 1H), 3.24 (d, J = 11.8 Hz, 1H), 2.82 (d, J = 10.8 Hz, 1H), 2.79 - 2.59 (m, 4H), 2.42 (s, 3H), 2.31 - 2.14 (m, 3H).

LCMS m/z = 444.2 [M+1]⁺.

### Example 7: (±)4-(1-((5-methoxy-7-methyl-1H-indol-4-yl)methyl)-4-(oxetan-2-ylmethyl)piperazin-2-yl)benzoic acid

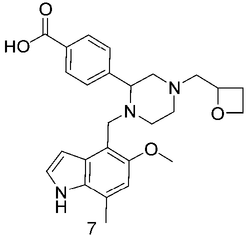

It was prepared by referring to the preparation method of Example 1, and 1-bromopropane in step f) was replaced with 2-(iodomethyl)oxetane.

¹HNMR (400 MHz, DMSO) δ 8.01 (d, J = 8.2 Hz, 2H), 7.68 (d, J = 7.8 Hz, 2H), 7.29 (s, 1H), 6.68 (s, 1H), 6.47 (s, 1H), 5.00 (s, 1H), 4.64 (d, J = 5.0 Hz, 1H), 4.56 - 4.46 (m, 2H), 4.34 (s, 1H), 3.78 (s, 3H), 3.62 - 3.52 (m, 2H), 3.29 (d, J = 8.4 Hz, 2H), 2.86 (d, J = 11.9 Hz, 2H), 2.73 (s, 1H), 2.66 (d, J = 4.4 Hz, 3H), 2.39 (s, 2H), 2.13 (s, 1H), 1.30 (s, 1H).

LCMS m/z = 450 [M+1]⁺.

### Example 8: (±)4-(4-((1-fluorocyclopropyl)methyl)-1-((5-methoxy-7-methyl-1H-indol-4-yl)methyl)piperazin-2-yl) benzoic acid

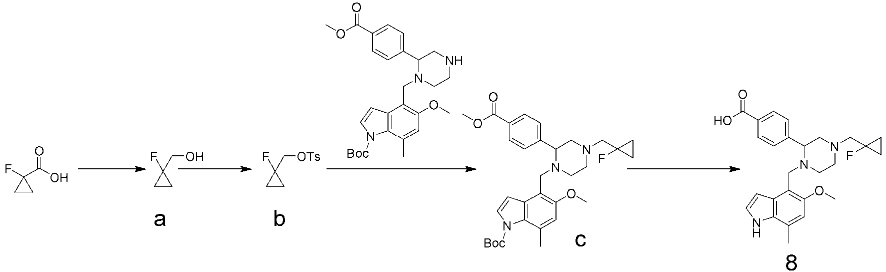

### a) Preparation of (1-fluorocyclopropyl)methanol

1-Fluorocyclopropane-1-carboxylic acid (520 mg) was dissolved in tetrahydrofuran (20 mL). The mixture was purged with nitrogen for protection. Lithium aluminum hydride (209 mg) was added at 0 °C, and the mixture was stirred at room temperature for 2 hours. The reaction was quenched with ice-water, and the mixture was extracted with ethyl acetate, washed with saturated brine. The organic phase was dried over anhydrous sodium sulfate, and filtered. The filtrate was concentrated under reduced pressure to obtain 420 mg of the title compound.

### b) Preparation of (1-fluorocyclopropyl)methyl 4-methylbenzenesulfonate

(1-Fluorocyclopropyl)methanol (420 mg) was dissolved in dichloromethane (20 mL). p-Toluenesulfonyl chloride (209 mg) and triethylamine (209 mg) were added in sequence, and the mixture was stirred overnight at room temperature. The reaction solution was concentrated under reduced pressure to yield a powdery residue, which was purified by column chromatography (mobile phase: petroleum ether/ethyl acetate = 20/1 (V/V)) to obtain 230 mg of the title compound.

### c) Preparation of (±)tert-butyl 4-((4-((1-fluorocyclopropyl)methyl)-2-(4-(methoxycarbonyl)phenyl)piperazin-1-yl) methyl)-5-methoxy-7-methyl-1H-indole-1-carboxylate

Tert-butyl 5-methoxy-4-((2-(4-(methoxycarbonyl)phenyl)piperazin-1-yl)methyl)-7-methyl-1H-indole-1-carboxylate (50 mg) and (1-fluorocyclopropyl)methyl 4-methylbenzenesulfonate (230 mg, 0.9 mmol) were added to a sealed tube. Then, N,N-dimethylformamide (10 mL) and sodium hydride (20 mg) were added. The mixture was stirred at 70 °C overnight. The reaction solution was cooled to room temperature and concentrated under reduced pressure to yield a powdery residue, which was purified by column chromatography (mobile phase: dichloromethane/methanol = 20/1 (V/V)) to obtain 12 mg of the title compound.

### d) Preparation of (±)4-(4-((1-fluorocyclopropyl)methyl)-1-((5-methoxy-7-methyl-1H-indol-4-yl)methyl)piperazin-2-yl) benzoic acid

(±)Tert-butyl 4-((4-((1-fluorocyclopropyl)methyl)-2-(4-(methoxycarbonyl)phenyl)piperazin-1-yl)methyl)-5-methoxy-7 -methyl-1H-indole-1-carboxylate (12 mg) was dissolved in a mixed solution of anhydrous tetrahydrofuran (3 mL) and methanol (3 mL). A 1 N aqueous solution of lithium hydroxide (0.5 mL) was added, and the mixture was heated to 70 °C and reacted for 3 h. After dissolving the residue in water, the pH was adjusted to neutral with dilute hydrochloric acid. The mixture was extracted with ethyl acetate. The organic phase was dried over anhydrous sodium sulfate, and filtered. The filtrate was concentrated under reduced pressure to yield a powdery residue, which was purified by column chromatography (mobile phase: dichloromethane/methanol = 20/1 (V/V)) to obtain 3.45 mg of the title compound.

¹HNMR (400 MHz, DMSO-*d6*) δ 7.97 (d, *J =* 7.6 Hz, 2H), 7.64 (s, 2H), 7.32 (d, *J =* 3.1 Hz, 1H), 6.64 (s, 1H), 6.44 (s, 1H), 4.72 (d, *J =* 20.5 Hz, 2H), 3.73 (s, 3H), 3.52 (d, *J =* 10.7 Hz, 2H), 3.25 - 3.12 (m, 2H), 2.80 (s, 1H), 2.66 (s, 3H), 2.33 (s, 1H), 2.12 - 1.91 (m, 2H), 1.46 (s, 1H), 1.04 (d, *J =* 19.0 Hz, 2H), 0.87 (d, *J* = 7.0 Hz, 2H).

LCMS m/z = 452.2 [M+1]⁺.

### Example 9: (R)-4-(1-((5-methoxy-7-methyl-1H-indol-4-yl)methyl)-4-(3,3,3-trifluoropropyl)piperazin-2-yl) benzoic acid and (S)-4-(1-((5-methoxy-7-methyl-1H-indol-4-yl)methyl)-4-(3,3,3-trifluoropropyl)piperazin-2-yl) benzoic acid

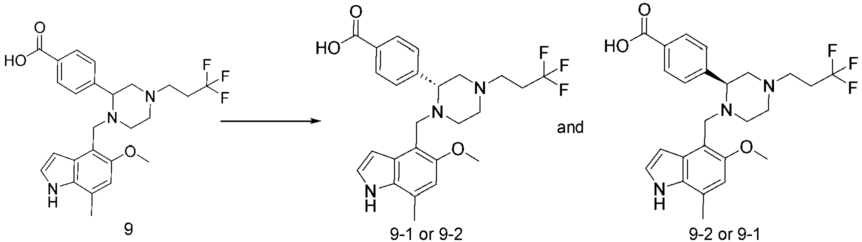

It was prepared by referring to the preparation method of Example 8, and the (1-fluorocyclopropyl)methyl 4-methylbenzenesulfonate in step c) was replaced with 3,3,3-trifluoropropyl 4-methylbenzenesulfonate.

¹HNMR (400 MHz, DMSO-*d*₆) δ 12.87 (s, 1H), 10.83 (s, 1H), 7.98 (d, *J* = 8.1 Hz, 2H), 7.71 (s, 2H), 7.28 (s, 1H), 6.66 (s, 1H), 6.44 (s, 1H), 3.71 (s, 3H), 3.57 (d, *J* = 11.8 Hz, 1H), 3.49 - 3.39 (m, 2H), 3.23 (d, *J =* 11.8 Hz, 2H), 2.77 (dd, *J =* 19.2, 10.5 Hz, 2H), 2.69 - 2.59 (m, 1H), 2.46 (d, *J* = 10.1 Hz, 1H), 2.42 (s, 3H), 2.21 (t, *J* = 10.9 Hz, 1H), 2.10 - 1.91 (m, 3H).

LCMS m/z = 476.2 [M+1]⁺.

The enantiomers of (±)4-(1-((5-methoxy-7-methyl-1H-indol-4-yl)methyl)-4-(3,3,3-trifluoropropyl)piperazin-2-yl) benzoic acid were resolved by chiral SFC, obtaining 9-1, tᵣ = 2.31 min and 9-2, tᵣ = 3.57 min. (The resolution method: chromatographic column: CHIRALPAK IE-3 4.6*50 mm, 3 µm; mobile phase A: (n-Hexane:dichloromethane = 3:1)(0.1% diethylamine); mobile phase B: isopropanol; A:B = 80:20; detection wavelength: dual-wavelength detection at 220/254; flow rate: 1.0 mL/min)

9-1, tr = 2.31 min

¹HNMR (400 MHz, DMSO-*d6*) δ10.82 (s, 1H), 7.94 (d, *J* = 8.3 Hz, 2H), 7.62 (d, *J* = 6.6 Hz, 2H), 7.25 (t, *J* = 2.7 Hz, 1H), 6.66 (s, 1H), 6.43 (s, 1H), 3.71 (s, 3H), 3.57 (d, *J* = 11.8 Hz, 2H), 3.22 (d, *J* = 11.8 Hz, 2H), 2.84 (q, *J* = 7.2 Hz, 2H), 2.76 (dd, *J* = 17.2, 11.2 Hz, 2H), 2.63 (d, *J* = 11.5 Hz, 1H), 2.45 (d, *J* = 9.8 Hz, 1H), 2.42 (s, 3H), 2.20 (t, *J* = 10.5 Hz, 1H), 2.10 - 1.92 (m, 2H).

9-2, tᵣ = 3.57 min

¹HNMR (400 MHz, DMSO-d6) δ10.82 (s, 1H), 7.94 (d, *J* = 8.3 Hz, 2H), 7.61 (d, *J* = 6.3 Hz, 2H), 7.25 (t, *J* = 2.7 Hz, 1H), 6.66 (s, 1H), 6.47 - 6.38 (m, 1H), 3.71 (s, 3H), 3.57 (d, *J* = 11.8 Hz, 2H), 3.21 (d, *J* = 11.8 Hz, 2H), 2.82 (dd, *J* = 14.5, 7.2 Hz, 2H), 2.77 - 2.69 (m, 2H), 2.63 (d, *J* = 11.5 Hz, 1H), 2.45 (d, *J* = 9.7 Hz, 1H), 2.42 (s, 3H), 2.19 (t, *J* = 10.4 Hz, 1H), 2.01 (dt, *J* = 20.7, 10.2 Hz, 2H).

### Example 10: (S)-4-(1-((5-methoxy-7-methyl-1H-indol-4-yl)methyl)-4-(3-(trifluoromethyl)cyclobutyl)piperazin-2-yl)benzoic acid and (R)-4-(1-((5-methoxy-7-methyl-1H-indol-4-yl)methyl)-4-(3-(trifluoromethyl)cyclobutyl)piperazin-2-yl)benzoic acid

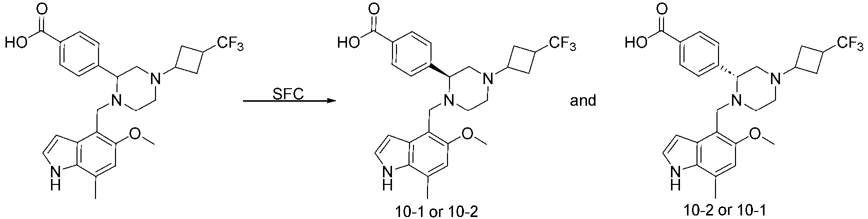

It was prepared by referring to the preparation method of Example 3 and oxetan-3-one in step a) was replaced with 3-(trifluoromethyl)cyclobutan-1-one.

¹HNMR (400 MHz, DMSO-*d*₆) δ 10.83 (s, 1H), 7.98 (d, J = 8.1 Hz, 2H), 7.68 (d, J = 7.3 Hz, 2H), 7.26 (s, 1H), 6.66 (s, 1H), 6.44 (s, 1H), 3.71 (s, 3H), 3.57 (d, J = 11.9 Hz, 1H), 3.43 (d, J = 8.7 Hz, 2H), 3.24 (d, J = 12.0 Hz, 3H), 3.20 - 3.07 (m,3H), 2.79 (t, J = 11.7 Hz, 2H), 2.62 (d, J = 11.1 Hz, 1H), 2.46 - 2.37 (m, 4H), 2.35 (d, J = 11.6 Hz, 1H), 2.28 - 2.19 (m, 1H).

LCMS m/z = 502.2 [M+1]⁺.

The enantiomers of (±)4-(1-((5-methoxy-7-methyl-1H-indol-4-yl)methyl)-4-(3-(trifluoromethyl)cyclobutyl)piperazin-2 -yl)benzoic acid were resolved by chiral SFC, obtaining 10-1, tᵣ = 2.12 min and 10-2, tᵣ = 3.03 min. (The resolution method: chromatographic column: DAICELCHIRALCEL^{®}OZ 250*20 mm, 10µm; mobile phase A: supercritical CO₂; mobile phase B: methanol (+0.1% 7.0 mol/L ammonia in methanol); A:B: 75:25; flow rate: 70 mL/min; detection wavelength: 214 nm)

10-1, tᵣ = 2.12 min

¹HNMR (400 MHz, DMSO-*d6*) δ 10.82 (s, 1H), 7.96 (d, J = 8.2 Hz, 2H), 7.67 (s, 2H), 7.25 (s, 1H), 6.65 (s, 1H), 6.43 (s, 1H), 3.70 (s, 3H), 3.57 (d, J = 11.7 Hz, 2H), 3.23 (d, J = 11.7 Hz, 2H), 2.84 - 2.74 (m, 1H), 2.65 (d, J = 13.1 Hz, 3H), 2.42 (s, 3H), 2.18 (dd, J = 18.3, 9.4 Hz, 3H), 2.03 - 1.93 (m, 1H), 1.83 (dd, J = 19.4, 8.6 Hz, 3H). LCMS m/z = 502.2 [M+1]⁺.

10-2, tᵣ = 3.03 min

¹HNMR (400 MHz, DMSO-*d6*) δ 10.82 (s, 1H), 7.95 (d, J = 8.0 Hz, 2H), 7.65 (s, 2H), 7.25 (s, 1H), 6.65 (s, 1H), 6.43 (s, 1H), 3.70 (s, 3H), 3.57 (d, J = 11.8 Hz, 2H), 3.22 (d, J = 11.9 Hz, 2H), 2.83 (d, J = 9.1 Hz, 1H), 2.65 (d, J = 12.1 Hz, 3H), 2.42 (s, 3H), 2.18 (d, J = 26.6 Hz, 3H), 2.03 - 1.93 (m, 1H), 1.83 (dd, J = 19.6, 9.5 Hz, 3H). LCMS m/z = 502.2 [M+1]⁺.

### Example 11: (S)-4-(1-((5-methoxy-7-methyl-1H-indol-4-yl)methyl)-4-(2,2,2-trifluoroethyl)piperazin-2-yl)benzoic acid and (R)-4-(1-((5-methoxy-7-methyl-1H-indol-4-yl)methyl)-4-(2,2,2-trifluoroethyl)piperazin-2-yl)benzoic acid.

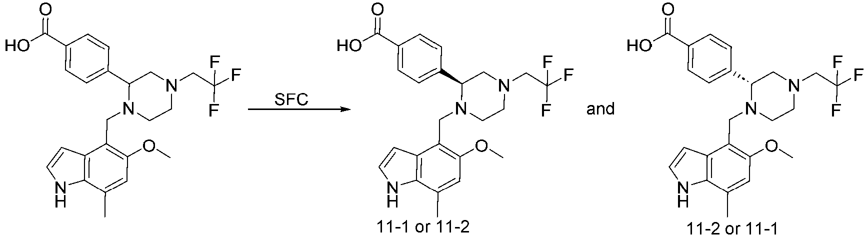

It was prepared by referring to the preparation method of Example 8, and the (1-fluorocyclopropyl)methyl 4-methylbenzenesulfonate in step c) was replaced with 2,2,2-trifluoroethyl trifluoromethanesulfonate.

¹HNMR (400 MHz, DMSO-*d*₆) δ 10.83 (s, 1H), 7.96 (d, J = 8.4 Hz, 2H), 7.66 (d, J = 7.5 Hz, 2H), 7.26 (t, J = 2.7 Hz, 1H), 6.66 (s, 1H), 6.44 (dd, J = 2.8, 2.0 Hz, 1H), 3.71 (s, 3H), 3.57 (d, J = 11.8 Hz, 1H), 3.47 - 3.40 (m, 1H), 3.22 (s, 1H), 3.19 - 3.07 (m, 2H), 2.79 (t, J = 12.1 Hz, 2H), 2.67 (d, J = 11.5 Hz, 1H), 2.46 - 2.31 (m, 5H), 2.23 (t, J = 10.7 Hz, 1H).

LCMS m/z = 462.2 [M+1]⁺.

The enantiomers of (±)4-(1-((5-methoxy-7-methyl-1H-indol-4-yl)methyl)-4-(2,2,2-trifluoroethyl)piperazin-2-yl) benzoic acid were resolved by chiral SFC, obtaining 11-1, tᵣ = 1.08 min and 11-2, tᵣ = 2.24 min. (The resolution method: chromatographic column: DAICELCHIRALPAK^{®}IG 250*20 mm, 10 µm; mobile phase A: supercritical CO₂; mobile phase B: MEOH (+0.1% 7.0 mol/L ammonia in methanol); A:B: 70:30; flow rate: 100 mL/min; detection wavelength: 214 nm)

11-1, tᵣ = 1.08 min

LCMS m/z = 462.2[M+1]⁺.

¹HNMR (400 MHz, DMSO-d6) δ 10.83 (s, 1H), 7.98 (d, J = 8.4 Hz, 2H), 7.68 (d, J = 7.5 Hz, 2H), 7.26 (t, J = 2.7 Hz, 1H), 6.66 (s, 1H), 6.44 (dd, J = 2.8, 2.0 Hz, 1H), 3.71 (s, 3H), 3.57 (d, J = 11.8 Hz, 1H), 3.47 - 3.40 (m, 1H), 3.26 - 3.20 (m, 1H), 3.19 - 3.07 (m, 2H), 2.79 (t, J = 12.1 Hz, 2H), 2.62 (d, J = 11.5 Hz, 1H), 2.46 - 2.31 (m, 5H), 2.23 (t, J = 10.7 Hz, 1H).

11-2, tᵣ = 2.24 min

¹HNMR (400 MHz, DMSO-*d6*) δ 10.83 (s, 1H), 7.98 (d, *J* = 8.4 Hz, 2H), 7.69 (d, *J* = 7.3 Hz, 2H), 7.26 (t, *J =* 2.7 Hz, 1H), 6.66 (s, 1H), 6.47 - 6.37 (m, 1H), 3.71 (s, 3H), 3.57 (d, *J* = 11.8 Hz, 1H), 3.46 - 3.40 (m, 1H), 3.24 (d, *J* = 11.9 Hz, 1H), 3.19 - 3.08 (m, 2H), 2.79 (t, *J* = 11.8 Hz, 2H), 2.62 (d, *J* = 11.4 Hz, 1H), 2.46 - 2.31 (m, 5H), 2.23 (t, *J =* 10.5 Hz, 1H).

LCMS m/z = 462.2 [M+1]⁺.

### Example 12: (±)4-(4-(3,3-difluorocyclobutyl)-1-((5-methoxy-7-methyl-1H-indol-4-yl)methyl)piperazin-2-yl) benzoic acid

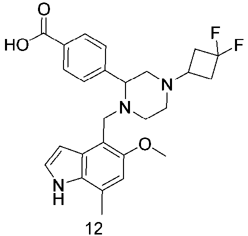

It was prepared by referring to the preparation method of Example 3 and oxetan-3-one in step a) was replaced with 3,3-difluorocyclobutan-1-one.

¹HNMR (400 MHz, DMSO-*d*₆) δ 10.82 (s, 1H), 7.97 (d, J = 7.8 Hz, 2H), 7.69 (s, 2H), 7.19 (s, 1H), 6.66 (s, 1H), 6.43 (s, 1H), 3.71 (s, 3H), 3.58 (d, J = 12.4 Hz, 1H), 3.35 (s, 1H), 3.23 (d, J = 11.9 Hz, 1H), 2.67 (s, 3H), 2.61 (s, 1H), 2.42 (s, 2H), 2.33 (s, 1H), 2.21 (s, 2H), 2.07 - 1.94 (m, 2H), 1.45 (s, 3H).

LCMS m/z = 470.2[M+1]⁺.

### Example 13: 4-((3S,4S)-1-(2,2-difluoroethyl)-4-((5-methoxy-7-methyl-1H-indol-4-yl)oxy)piperidin-3-yl)benzoic acid and 4-((3R,4R)-1-(2,2-difluoroethyl)-4-((5-methoxy-7-methyl-1H-indol-4-yl)oxy)piperidin-3-yl)benzoic acid and 4-((3S,4R)-1-(2,2-difluoroethyl)-4-((5-methoxy-7-methyl-1H-indol-4-yl)oxy)piperidin-3-yl)benzoic acid and 4-((3R,4S)-1-(2,2-difluoroethyl)-4-((5-methoxy-7-methyl-1H-indol-4-yl)oxy)piperidin-3-yl)benzoic acid

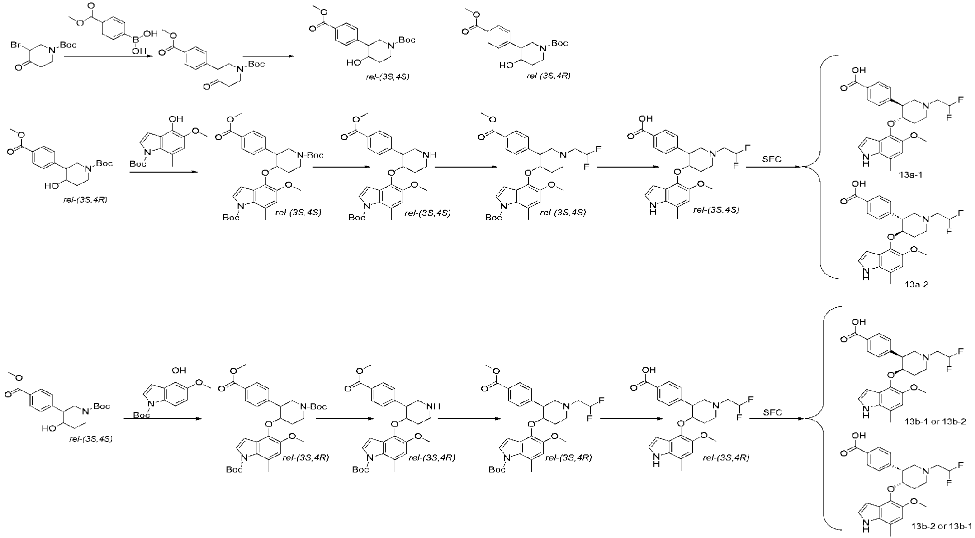

### a) Preparation of (±)tert-butyl 3-(4-(methoxycarbonyl)phenyl)-4-oxopiperidine-1-carboxylate

Tert-butyl 3-bromo-4-oxopiperidine-1-carboxylate (50 g), (4-(methoxycarbonyl)phenyl)boronic acid (48.52 g), nickel(II) trifluoromethanesulfonate (3.21 g), 1,10-phenanthroline (1.62 g), potassium carbonate (49.62 g), and 1,4-dioxane (500 mL) were added to a reaction flask. The mixture was purged with nitrogen for protection. The mixture was stirred at 80 °C for 16 h. The reaction solution was diluted with water (500 mL) and extracted with ethyl acetate (3 × 500 mL). The layers were separated, and the organic phases were combined, dried over anhydrous sodium sulfate, and filtered. The filtrate was concentrated under reduced pressure to yield a powdery residue, which was purified by column chromatography (mobile phase: petroleum ether/ethyl acetate = 5/1 (V/V)) to obtain 32 g of the title compound.

### b) Preparation of (±)-rel-(3S,4R)-tert-butyl 4-hydroxy-3-(4-(methoxycarbonyl)phenyl)piperidine-1-carboxylate and (±)-rel-(3S,4S)-tert-butyl 4-hydroxy-3-(4-(methoxycarbonyl)phenyl)piperidine-1-carboxylate

(±)Tert-butyl 3-(4-(methoxycarbonyl)phenyl)-4-oxopiperidine-1-carboxylate (30 g) was added to a reaction flask. Methanol (300 mL) was added. Under nitrogen protection, sodium borohydride (10.21 g) was added in batches at 0 °C. The mixture was stirred at room temperature for 1 hour. Under an ice-bath, water (500 mL) was added to quench the reaction. The mixture was extracted with ethyl acetate (3 × 500 mL). The layers were separated, and the combined organic phases were dried over anhydrous sodium sulfate and filtered. The filtrate was concentrated under reduced pressure to yield a powdery residue, which was purified by column chromatography (mobile phase: petroleum ether/ethyl acetate = 3/1 (V/V)). According to the corresponding elution gradient, 8.9 g and 21.3 g of the title compounds were obtained respectively.

(±)-rel-(3S,4R)-tert-butyl 4-hydroxy-3-(4-(methoxycarbonyl)phenyl)piperidine-1-carboxylate

¹HNMR (400 MHz, DMSO-*d₆*) δ 7.88 (d, *J =* 8.4 Hz, 2H), 7.43 (d, *J =* 8.4 Hz, 2H), 4.75 (d, *J* = 4.2 Hz, 1H), 3.99 (d, *J* = 7.8 Hz, 1H), 3.84 (s, 3H), 3.77 (d, *J* = 12.4 Hz, 2H), 3.49 (d, *J* = 14.2 Hz, 1H), 3.16 (s, 1H), 2.83 (d, *J* = 11.2 Hz, 1H), 1.71 - 1.64 (m, 2H), 1.39 (s, 9H).

(±)-rel-(3S,4S)-tert-butyl 4-hydroxy-3-(4-(methoxycarbonyl)phenyl)piperidine-1-carboxylate

¹HNMR (400 MHz, DMSO-*d₆*) δ 7.90 (d, *J =* 8.3 Hz, 2H), 7.42 (d, *J =* 8.3 Hz, 2H), 4.71 (d, *J* = 6.0 Hz, 1H), 4.03 (q, *J* = 7.1 Hz, 1H), 3.97 (d, *J =* 11.8 Hz, 1H), 3.85 (s, 3H), 3.83 - 3.72 (m, 2H), 2.89 (s, 2H), 1.99 (s, 1H), 1.89 (dd, *J* = 12.4, 3.8 Hz, 1H), 1.40 (s, 9H).

Example 13a: 4-((3S,4S)-1-(2,2-difluoroethyl)-4-((5-methoxy-7-methyl-1H-indol-4-yl)oxy)piperidin-3-yl)benzoic acid and 4-((3R,4R)-1-(2,2-difluoroethyl)-4-((5-methoxy-7-methyl-1H-indol-4-yl)oxy)piperidin-3-yl)benzoic acid

### a) Preparation of (±)-rel-(3S,4S)-tert-butyl 4-((1-(tert-butoxycarbonyl)-3-(4-(methoxycarbonyl)phenyl)piperidin-4-yl) oxy)-5-methoxy-7-methyl-1H-indole-1-carboxylate

Tert-butyl 4-hydroxy-5-methoxy-7-methyl-1H-indole-1-carboxylate (10.06 g), (±)-rel-(3S,4R)-tert-butyl 4-hydroxy-3-(4-(methoxycarbonyl)phenyl)piperidine-1-carboxylate (8.4 g), triphenylphosphine (23.82 g), and anhydrous tetrahydrofuran (200 mL) were added to a three-necked flask. The mixture was purged with nitrogen for protection. Diisopropyl azodicarboxylate (18.33 g) was added dropwise at 0 °C. The reaction system was warmed to room temperature and reacted for 16 hours. The reaction was diluted with water (500 mL) and extracted with ethyl acetate (3 × 200 mL). The layers were separated, and the combined organic phases were dried over anhydrous sodium sulfate and filtered. The filtrate was concentrated under reduced pressure to yield a powdery residue, which was purified by column chromatography (mobile phase: petroleum ether/ethyl acetate = 5/1 (V/V)) to obtain 22 g of the title compound.

### b) Preparation of (±)-rel-(3S,4S)-tert-butyl 5-methoxy-4-((3-(4-(methoxycarbonyl)phenyl)piperidin-4-yl)oxy)-7-methyl-1H-indole-1-carboxylate

(±)-Rel-(3S,4S)-tert-butyl 4-((1-(tert-butoxycarbonyl)-3-(4-(methoxycarbonyl)phenyl)piperidin-4-yl)oxy)-5-methoxy-7 -methyl-1H-indole-1-carboxylate (10 g) was dissolved in 4 M hydrochloric acid in 1,4-dioxane (70 mL). The mixture was stirred at room temperature for 4 hours, and then concentrated under reduced pressure to obtain 16 g of the title compound as a residue.

LCMS m/z = 495.4[M+1]⁺.

### c) Preparation of (±)-rel-(3S,4S)-tert-butyl 4-((1-(2,2-difluoroethyl)-3-(4-(methoxycarbonyl)phenyl)piperidin-4-yl)oxy) -5-methoxy-7-methyl-1H-indole-1-carboxylate

(±)-Rel-(3S,4S)-tert-butyl 5-methoxy-4-((3-(4-(methoxycarbonyl)phenyl)piperidin-4-yl)oxy)-7-methyl-1H-indole-1-carboxylate (8 g) and N,N-diisopropylethylamine (8.4 mL) were added to a reaction flask. Tetrahydrofuran (80 mL) was added, followed by the addition of 2,2-difluoroethyl trifluoromethanesulfonate (10.4 g). The mixture was purged with nitrogen for protection. The mixture was stirred at 70 °C for 2 hours. The reaction solution was diluted with water (500 mL) and extracted with ethyl acetate (3 × 200 mL). The layers were separated, and the combined organic phases were dried over anhydrous sodium sulfate and filtered. The filtrate was concentrated under reduced pressure to yield a powdery residue, which was purified by column chromatography (mobile phase: petroleum ether/ethyl acetate = 5/1 (V/V)) to obtain 12.4 g of the title compound.

### d) Preparation of 4-((3S,4S)-1-(2,2-difluoroethyl)-4-((5-methoxy-7-methyl-1H-indol-4-yl)oxy)piperidin-3-yl)benzoic acid and 4-((3R,4R)-1-(2,2-difluoroethyl)-4-((5-methoxy-7-methyl-1H-indol-4-yl)oxy)piperidin-3-yl)benzoic acid

(±)-Rel-(3S,4S)-tert-butyl 4-((1-(2,2-difluoroethyl)-3-(4-(methoxycarbonyl)phenyl)piperidin-4-yl)oxy)-5-methoxy-7-methyl-1H-indole-1-carboxylate (6.2 g) was added to a reaction flask. Methanol (120 mL), lithium hydroxide (7.8 g), and water (40 mL) were added. The mixture was purged with nitrogen for protection. The mixture was stirred at 70 °C for 16 hours. The reaction solution was adjusted to pH = 6 with dilute hydrochloric acid (3 M), diluted with water (100 mL), and extracted with ethyl acetate (3 × 500 mL). The layers were separated, and the combined organic phases were dried over anhydrous sodium sulfate and filtered. The filtrate was concentrated under reduced pressure to yield a powdery residue, which was purified by column chromatography (mobile phase: dichloromethane/methanol = 5/1 (V/V)) to obtain 7.6 g of the enantiomer of the title compound.

LCMS m/z = 445[M+1]⁺.

The enantiomers of (±)-rel-(3S,4S)-4-(1-(2,2-difluoroethyl)-4-((5-methoxy-7-methyl-1H-indol-4-yl)oxy)piperidin-3-yl) benzoic acid were resolved by chiral SFC, obtaining 4-((3S,4S)-1-(2,2-difluoroethyl)-4-((5-methoxy-7-methyl-1H-indol-4-yl)oxy)piperidin-3-yl)benzoic acid (13a-1, tᵣ = 1.19 min) and 4-((3R,4R)-1-(2,2-difluoroethyl)-4-((5-methoxy-7-methyl-1H-indol-4-yl)oxy)piperidin-3-yl)benzoic acid (13a-2, tᵣ = 1.95 min). The absolute stereochemical configuration of 4-((3R,4R)-1-(2,2-difluoroethyl)-4-((5-methoxy-7-methyl-1H-indol-4-yl)oxy)piperidin-3-yl)benzoic acid was confirmed by X-ray single crystal diffraction in Experimental Example 6. (The resolution method: chromatographic column: DAICELCHIRALPAK^{®}IC 250*20 mm, 10 µm; mobile phase A: supercritical CO₂; mobile phase B: methanol (+0.1% 7.0 mol/L ammonia in methanol); A:B: 75:25;flow rate: 100 mL/min; detection wavelength: 214 nm).

4-((3S,4S)-1-(2,2-difluoroethyl)-4-((5-methoxy-7-methyl-1H-indol-4-yl)oxy)piperidin-3-yl)benzoic acid

¹HNMR (400 MHz, DMSO-*d₆*) δ 10.83 (s, 1H), 7.90 (d, *J =* 8.2 Hz, 2H), 7.56 (d, *J =* 8.1 Hz, 2H), 7.20 - 7.13 (m, 1H), 6.62 (s, 1H), 6.30 - 5.96 (m, 2H), 4.48 - 4.38 (m, 1H), 3.60 (s, 3H), 3.29 (s, 1H), 3.12 (s, 1H), 2.93 (dd, *J =* 23.7, 11.6 Hz, 2H), 2.78 (t, *J =* 15.5 Hz, 2H), 2.34 (d, *J =* 8.9 Hz, 3H), 2.28 (s, 1H), 1.70 (s, 2H).

LCMS m/z = 445.1[M+1]⁺.

4-((3R,4R)-1-(2,2-difluoroethyl)-4-((5-methoxy-7-methyl-1H-indol-4-yl)oxy)piperidin-3-yl)benzoic acid

¹HNMR (400 MHz, DMSO-d6) δ 10.83 (s, 1H), 7.88 (d, *J =* 8.1 Hz, 2H), 7.51 (d, *J =* 8.1 Hz, 2H), 7.16 (t, *J =* 2.7 Hz, 1H), 6.62 (s, 1H), 6.29 - 5.97 (m, 2H), 4.51 - 4.39 (m, 1H), 3.60 (s, 3H), 3.38 (d, *J =* 3.5 Hz, 1H), 3.09 (s, 1H), 2.95 - 2.87 (m, 2H), 2.75 (d, *J =* 15.6 Hz, 2H), 2.36 (s, 3H), 2.26 (s, 1H), 1.69 (s, 2H).

LCMS m/z = 445.1[M+1]⁺.

Example 13b: 4-((3S,4R)-1-(2,2-difluoroethyl)-4-((5-methoxy-7-methyl-1H-indol-4-yl)oxy)piperidin-3-yl)benzoic acid and 4-((3R,4S)-1-(2,2-difluoroethyl)-4-((5-methoxy-7-methyl-1H-indol-4-yl)oxy)piperidin-3-yl)benzoic acid

It was prepared by referring to the preparation method of Example 13a, and (±)-rel-(3S,4R)-tert-butyl 4-hydroxy-3-(4-(methoxycarbonyl)phenyl)piperidine-1-carboxylate in step a) was replaced with (±)-rel-(3S,4S)-tert-butyl 4-hydroxy-3-(4-(methoxycarbonyl)phenyl)piperidine-1-carboxylate.

The enantiomers of (±)-rel-(3S,4R)-4-(1-(2,2-difluoroethyl)-4-((5-methoxy-7-methyl-1H-indol-4-yl)oxy)piperidin-3-yl) benzoic acid were resolved by chiral SFC, obtaining 13b-1, tᵣ = 1.29 min and 13b-2, tᵣ = 4.00 min (The resolution method: chromatographic column: DAICELCHIRALPAK^{®}IC 250*20 mm 10 µm; mobile phase A: supercritical CO₂; mobile phase B: methanol (+0.1% 7.0 mol/L ammonia in methanol); A:B: 75:25;flow rate:100 mL/min; detection wavelength: 214 nm)

13b-1, tᵣ = 1.29 min

¹HNMR (400 MHz, DMSO-*d6*) δ 10.83 (s, 1H), 7.89 (d, *J =* 8.3 Hz, 2H), 7.57 (d, *J =* 8.4 Hz, 2H), 7.09 (t, *J =* 2.7 Hz, 1H), 6.61 (s, 1H), 6.38 - 5.99 (m, 1H), 5.73 (s, 1H), 4.65 (s, 1H), 3.55 (s, 3H), 3.18 (dd, *J =* 22.7, 10.9 Hz, 2H), 2.88 (dd, *J* = 17.7, 12.0 Hz, 4H), 2.67 (s, 1H), 2.34 (s, 3H), 1.66 (s, 2H).

LCMS m/z = 445.1[M+1]⁺.

13b-2, tᵣ = 4.00 min

¹HNMR (400 MHz, DMSO-d6) δ 13.09 - 12.27 (m, 1H), 10.83 (s, 1H), 7.90 (d, J = 8.3 Hz, 2H), 7.58 (d, J = 8.3 Hz, 2H), 7.09 (t, J = 2.8 Hz, 1H), 6.61 (s, 1H), 6.37 - 6.02 (m, 1H), 5.77 - 5.71 (m, 1H), 4.66 (s, 1H), 3.55 (s, 3H), 3.21 (dd, J = 24.5, 13.9 Hz, 2H), 2.96 - 2.83 (m, 4H), 2.66 (d, J = 10.6 Hz, 1H), 2.34 (s, 3H), 1.66 (s, 2H).

LCMS m/z = 445.1[M+1]⁺.

### Example 14:

### 4-((3S,4S)-4-((5-methoxy-7-methyl-1H-indol-4-yl)oxy)-1-(2,2,2-trifluoroethyl)piperidin-3-yl)benzoic acid and 4-((3R,4R)-4-((5-methoxy-7-methyl-1H-indol-4-yl)oxy)-1-(2,2,2-trifluoroethyl)piperidin-3-yl)benzoic acid and 4-((3S,4R)-4-((5-methoxy-7-methyl-1H-indol-4-yl)oxy)-1-(2,2,2-trifluoroethyl)piperidin-3-yl)benzoic acid and 4-((3R,4S)-4-((5-methoxy-7-methyl-1H-indol-4-yl)oxy)-1-(2,2,2-trifluoroethyl)piperidin-3-yl)benzoic acid

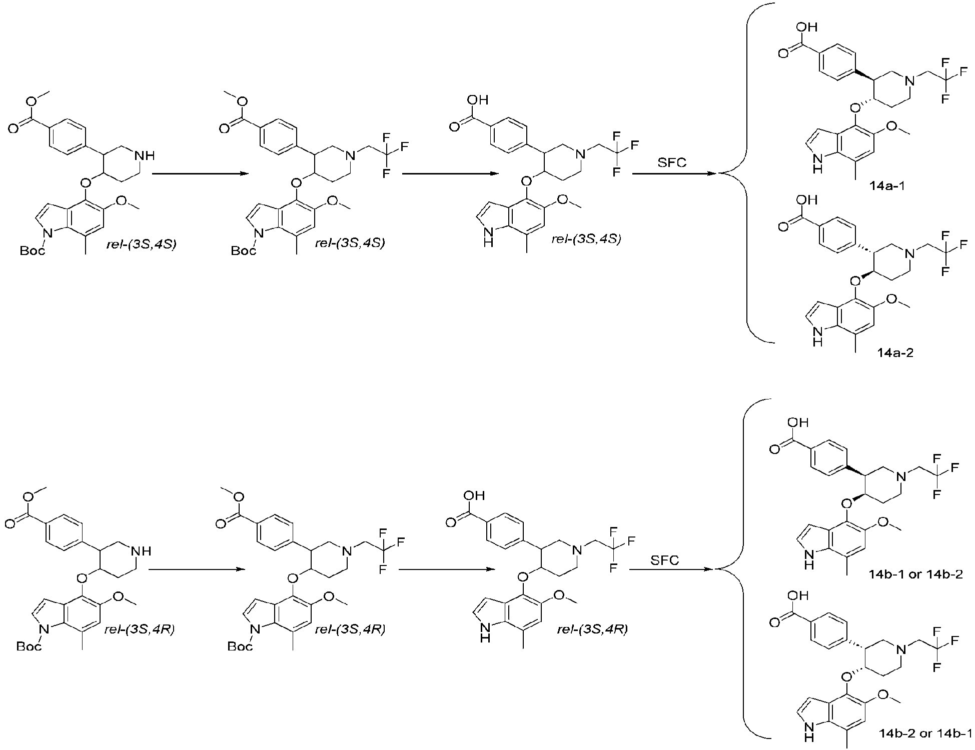

### Example 14a: 4-((3S,4S)-4-((5-methoxy-7-methyl-1H-indol-4-yl)oxy)-1-(2,2,2-trifluoroethyl)piperidin-3-yl)benzoic acid and 4-((3R,4R)-4-((5-methoxy-7-methyl-1H-indol-4-yl)oxy)-1-(2,2,2-trifluoroethyl)piperidin-3-yl)benzoic acid

### a) Preparation of (±)-rel-(3S,4S)-tert-butyl 5-methoxy-4-((3-(4-(methoxycarbonyl)phenyl)-1-(2,2,2-trifluoroethyl) piperidin-4-yl)oxy)-7-methyl-1H-indole-1-carboxylate

(±)-Rel-(3S,4S)-tert-butyl 4-((3-(4-(methoxycarbonyl)phenyl)piperidin-4-yl)oxy)-5-methoxy-7 -methyl-1H-indole-1-carboxylate was prepared by referring to Example 13. (±)-Rel-(3S,4S)-tert-butyl 4-((3-(4-(methoxycarbonyl)phenyl)piperidin-4-yl)oxy)-5-methoxy-7-methyl-1H-indole-1-carboxylate (6.3 g) and N,N-diisopropylethylamine (6.6 mL) were added to a reaction flask. Tetrahydrofuran (80 mL) was added, followed by the addition of 2,2,2-trifluoroethyl trifluoromethanesulfonate (8.9 g). The mixture was purged with nitrogen for protection. The mixture was stirred at 70 °C for 2 hours. The reaction solution was diluted with water (500 mL) and extracted with ethyl acetate (3 × 200 mL). The layers were separated, and the combined organic phases were dried over anhydrous sodium sulfate and filtered. The filtrate was concentrated under reduced pressure to yield a powdery residue, which was purified by column chromatography (mobile phase: petroleum ether/ethyl acetate = 5/1 (V/V)) to obtain 5 g of the title compound.

LCMS m/z = 577.1 [M+H]⁺.

### b) Preparation of 4-((3S,4S)-4-((5-methoxy-7-methyl-1H-indol-4-yl)oxy)-1-(2,2,2-trifluoroethyl)piperidin-3-yl) benzoic acid and 4-((3R,4R)-4-((5-methoxy-7-methyl-1H-indol-4-yl)oxy)-1-(2,2,2-trifluoroethyl)piperidin-3-yl)benzoic acid

(±)-Rel-(3S,4S)-tert-butyl 5-methoxy-4-((3-(4-(methoxycarbonyl)phenyl)-1-(2,2,2-trifluoroethyl)piperidin-4-yl)oxy)-7 -methyl-1H-indole-1-carboxylate (5 g) was added to a reaction flask. Methanol (120 mL), lithium hydroxide (6.25 g), and water (100 mL) were added. The mixture was purged with nitrogen for protection. The mixture was stirred at 70 °C for 16 hours. The reaction solution was adjusted to pH = 6 with dilute hydrochloric acid (3 M), diluted with water (100 mL), and extracted with ethyl acetate (3 × 500 mL). The layers were separated, and the combined organic phases were dried over anhydrous sodium sulfate and filtered. The filtrate was concentrated under reduced pressure to yield a powdery residue, which was purified by column chromatography (mobile phase: dichloromethane/methanol = 5/1 (V/V)) to obtain 1.7 g of the enantiomer of the title compound.

The enantiomers of (±)-rel-(3S,4S)-4-(4-((5-methoxy-7-methyl-1H-indol-4-yl)oxy)-1-(2,2,2-trifluoroethyl)piperidin-3-yl)benzoic acid were resolved by chiral SFC, obtaining 4-((3S,4S)-4-((5-methoxy-7-methyl-1H-indol-4-yl)oxy)-1-(2,2,2-trifluoroethyl)piperidin-3-yl)benzoic acid (14a-1, tᵣ = 1.87 min) and 4-((3R,4R)-4-((5-methoxy-7-methyl-1H-indol-4-yl)oxy) -1-(2,2,2-trifluoroethyl)piperidin-3-yl)benzoic acid (14a-2, tᵣ = 2.73 min). The absolute stereochemical configuration of 4-((3R,4R)-4-((5-methoxy-7-methyl-1H-indol-4-yl)oxy)-1-(2,2,2-trifluoroethyl)piperidin-3-yl)benzoic acid was confirmed by X-ray single crystal diffraction in Experimental Example 7. (The resolution method: chromatographic column: DAICELCHIRALCEL^{®}OZ 250*25 mm, 10 µm; mobile phase A: supercritical CO₂; mobile phase B: methanol (+0.1% 7.0 mol/L ammonia in methanol); A:B: 75:25; flow rate:100 mL/min; detection wavelength: 214 nm)

14a-1, tᵣ = 1.87 min

¹HNMR (400 MHz, DMSO-*d6*) δ 10.84 (s, 1H), 7.89 (d, *J =* 8.2 Hz, 2H), 7.55 (d, *J* = 8.2 Hz, 2H), 7.17 (t, *J* = 2.8 Hz, 1H), 6.63 (s, 1H), 6.21 - 6.16 (m, 1H), 4.47 (dd, *J* = 16.1, 7.7 Hz, 1H), 3.60 (s, 3H), 3.24 (d, *J* = 3.5 Hz, 2H), 3.16 - 3.08 (m, 1H), 2.94 (t, *J* = 12.7 Hz, 2H), 2.67 (t, *J* = 11.2 Hz, 1H), 2.47 - 2.41 (m, 1H), 2.34 (d, *J* = 11.4 Hz, 3H), 1.70 (d, *J* = 3.6 Hz, 2H).

LCMS m/z = 463.1 [M+H]⁺.

14a-2, tᵣ = 2.73 min

¹HNMR (400 MHz, DMSO-*d6*) δ 10.81 (s, 1H), 7.86 (d, *J =* 8.2 Hz, 2H), 7.51 (d, *J* = 8.2 Hz, 2H), 7.14 (t, *J* = 2.8 Hz, 1H), 6.59 (s, 1H), 6.15 (dd, *J* = 3.0, 2.0 Hz, 1H), 4.44 (dd, *J* = 16.1, 7.8 Hz, 1H), 3.57 (s, 3H), 3.20 (d, *J* = 3.6 Hz, 2H), 3.12 - 3.05 (m, 1H), 2.89 (d, *J* = 13.0 Hz, 2H), 2.64 (t, *J* = 11.3 Hz, 1H), 2.42 (s, 1H), 2.32 (s, 3H), 1.67 (d, *J* = 3.5 Hz, 2H).

LCMS m/z = 463.1 [M+H]⁺.

### Example 14b: (±)-rel-(3S,4R)-4-(4-((5-methoxy-7-methyl-1H-indol-4-yl)oxy)-1-(2,2,2-trifluoroethyl)piperidin-3-yl) benzoic acid

It was prepared by referring to the preparation method of Example 14a, and (±)-rel-(3S,4S)-tert-butyl 4-((1-(tert-butoxycarbonyl)-3-(4-(methoxycarbonyl)phenyl)piperidin-4-yl)oxy)-5-methoxy-7-methyl-1H-indole-1-carboxylate in step a) was replaced with (±)-rel-(3S,4R)-tert-butyl 4-((1-(tert-butoxycarbonyl)-3-(4-(methoxycarbonyl)phenyl) piperidin-4-yl)oxy)-5-methoxy-7-methyl-1H-indole-1-carboxylate.

The enantiomers of (±)-rel-(3S,4R)-4-(4-((5-methoxy-7-methyl-1H-indol-4-yl)oxy)-1-(2,2,2-trifluoroethyl)piperidin-3-yl)benzoic acid were resolved by chiral SFC, obtaining 14b -1, tᵣ = 1.06 min and 14b -2, tᵣ = 2.37 min (The resolution method: chromatographic column: DAICELCHIRALPAK^{®}IC 250*20 mm, 10 µm; mobile phase A: supercritical CO₂; mobile phase B: methanol (+0.1% 7.0 mol/L ammonia in methanol); A:B: 75:25; flow rate:100 mL/min; detection wavelength: 214 nm)

14b -1, tᵣ = 1.06 min

¹HNMR (400 MHz, DMSO-*d6*) δ 10.83 (s, 1H), 7.89 (d, *J =* 8.3 Hz, 2H), 7.56 (d, *J* = 8.3 Hz, 2H), 7.09 (t, *J* = 2.8 Hz, 1H), 6.61 (s, 1H), 5.71 (dd, *J* = 3.0, 2.0 Hz, 1H), 4.65 (d, *J* = 2.5 Hz, 1H), 3.55 (s, 3H), 3.42 - 3.36 (m, 2H), 3.30 - 3.25 (m, 2H), 3.14 - 3.07 (m, 1H), 2.89 (d, *J* = 7.6 Hz, 1H), 2.68 (d, *J* = 10.9 Hz, 1H), 2.34 (s, 3H), 1.65 (s, 2H).

LCMS m/z = 463.1 [M+H]⁺.

14b -2, tᵣ = 2.37 min

¹HNMR (400 MHz, DMSO-*d6*) δ 10.84 (s, 1H), 7.90 (d, *J* = 8.3 Hz, 2H), 7.58 (d, *J* = 8.3 Hz, 2H), 7.09 (t, *J* = 2.7 Hz, 1H), 6.61 (s, 1H), 5.72 (dd, *J* = 2.9, 2.0 Hz, 1H), 4.66 (d, *J* = 2.4 Hz, 1H), 3.55 (s, 3H), 3.44 - 3.36 (m, 2H), 3.29 (d, *J* = 5.9 Hz, 2H), 3.12 (td, *J* = 10.6, 5.6 Hz, 1H), 2.89 (d, *J* = 7.8 Hz, 1H), 2.69 (d, *J* = 11.1 Hz, 1H), 2.34 (s, 3H), 1.66 (s, 2H).

LCMS m/z = 463.1 [M+H]⁺.

### Example 15:

### 4-((3S,4S)-4-((5-methoxy-7-methyl-1H-indol-4-yl)oxy)-1-(3,3,3-trifluoropropyl)piperidin-3-yl)benzoic acid and 4-((3R,4R)-4-((5-methoxy-7-methyl-1H-indol-4-yl)oxy)-1-(3,3,3-trifluoropropyl)piperidin-3-yl)benzoic acid and 4-((3S,4R)-4-((5-methoxy-7-methyl-1H-indol-4-yl)oxy)-1-(3,3,3-trifluoropropyl)piperidin-3-yl)benzoic acid and 4-((3R,4S)-4-((5-methoxy-7-methyl-1H-indol-4-yl)oxy)-1-(3,3,3-trifluoropropyl)piperidin-3-yl)benzoic acid

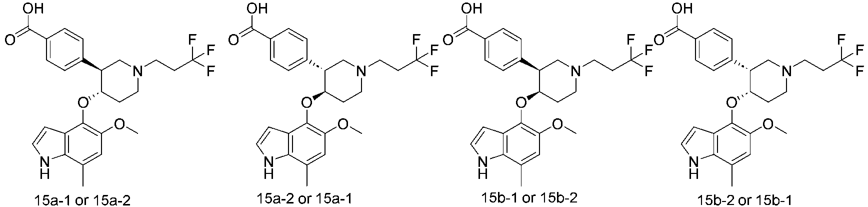

### Example 15a: (±)-rel-(3S,4S)-4-(4-((5-methoxy-7-methyl-1H-indol-4-yl)oxy)-1-(3,3,3-trifluoropropyl)piperidin-3-yl) benzoic acid

It was prepared by referring to the preparation method of Example 13a, and 2,2-difluoroethyl trifluoromethanesulfonate in step c) was replaced with 3,3,3-trifluoropropyl trifluoromethanesulfonate.

The enantiomers of (±)-rel-(3S,4S)4-(4-((5-methoxy-7-methyl-1H-indol-4-yl)oxy)-1-(3,3,3-trifluoropropyl)piperidin-3 -yl)benzoic acid were resolved by chiral SFC, obtaining 15a-1, tᵣ = 1.25 min and 15a-2, tᵣ = 2.00 min(The resolution method: chromatographic column: DAICELCHIRALPAK^{®}IC 250*20 mm,10µm; mobile phase A: supercritical CO₂; mobile phase B: methanol (ammonia in methanol); A:B: 75:25;flow rate: 100 mL/min; detection wavelength: 214 nm)

15a-1, tᵣ = 1.25 min

¹HNMR (400 MHz, DMSO-*d6*) δ 10.83 (s, 1H), 7.91 (d, *J =* 8.1 Hz, 2H), 7.58 (d, *J =* 8.1 Hz, 2H), 7.17 (t, *J =* 2.4 Hz, 1H), 6.63 (s, 1H), 6.17 (s, 1H), 4.46 (td, *J* = 9.5, 4.8 Hz, 1H), 3.60 (s, 3H), 3.12 (td, *J* = 10.2, 3.6 Hz, 1H), 2.89 (dd, *J* = 24.1, 10.3 Hz, 2H), 2.57 (dd, *J =* 9.6, 6.7 Hz, 2H), 2.49 (d, *J =* 13.4 Hz, 2H), 2.36 (s, 3H), 2.30 (d, *J =* 11.0 Hz, 1H), 2.07 (t, *J =* 10.1 Hz, 1H), 1.79 - 1.64 (m, 2H).

LCMS m/z = 475.0 [M-H]⁺.

15a-2, tᵣ = 2.00 min

¹HNMR (400 MHz, DMSO-*d6*) δ 10.83 (s, 1H), 7.91 (d, *J =* 8.1 Hz, 2H), 7.58 (d, *J =* 8.2 Hz, 2H), 7.17 (t, *J =* 2.6 Hz, 1H), 6.63 (s, 1H), 6.17 (s, 1H), 4.46 (td, *J =* 9.5, 4.8 Hz, 1H), 3.60 (s, 3H), 3.12 (td, *J* = 10.2, 3.7 Hz, 1H), 2.93 - 2.83 (m, 2H), 2.62 - 2.53 (m, 2H), 2.52 - 2.45 (m, 2H), 2.36 (s, 3H), 2.31 (t, *J =* 11.1 Hz, 1H), 2.05 (dt, *J* = 15.0, 7.5 Hz, 1H), 1.80 - 1.62 (m, 2H).

LCMS m/z = 475.0[M-H]⁺.

### Example 15b: (±)-rel-(3S,4R)-4-(4-((5-methoxy-7-methyl-1H-indol-4-yl)oxy)-1-(3,3,3-trifluoropropyl)piperidin-3-yl) benzoic acid

It was prepared by referring to the preparation method of Example 13b, and 2,2-difluoroethyl trifluoromethanesulfonate in step c) was replaced with 2,2,2-trifluoropropyl trifluoromethanesulfonate.

The enantiomers of (±)-rel-(3S,4R)-4-(4-((5-methoxy-7-methyl-1H-indol-4-yl)oxy)-1-(3,3,3-trifluoropropyl)piperidin -3-yl)benzoic acid were resolved by chiral SFC, obtaining 15b-1, tᵣ = 0.91 min and 15b-2, tᵣ = 2.35 min (The resolution method: chromatographic column: DAICELCHIRALPAK^{®}IC 250*20 mm, 10µm; mobile phase A: supercritical CO₂; mobile phase B: methanol (+0.1% 7.0 mol/L ammonia in methanol); A:B: 75:25; flow rate:100 mL/min; detection wavelength: 214 nm)

15b-1, tᵣ = 0.91 min

¹HNMR (400 MHz, DMSO-*d6*) δ 12.88 - 12.26 (m, 1H), 10.83 (s, 1H), 7.90 (d, *J* = 8.3 Hz, 2H), 7.60 (d, *J* = 8.3 Hz, 2H), 7.10 (t, *J* = 2.7 Hz, 1H), 6.61 (s, 1H), 5.76 (s, 1H), 4.67 (d, *J* = 2.5 Hz, 1H), 3.54 (s, 3H), 3.29 (s, 1H), 3.24 (s, 1H), 3.00 (t, *J* = 10.8 Hz, 1H), 2.84 (d, *J* = 9.4 Hz, 1H), 2.73 - 2.54 (m, 5H), 2.33 (dd, *J* = 5.6, 1.0 Hz, 3H), 1.67 (t, *J* = 14.4 Hz, 2H).

15b-2, tr = 2.35 min

¹HNMR (400 MHz, DMSO-*d6*) δ 12.66 - 11.86 (m, 1H), 10.83 (s, 1H), 7.90 (d, *J* = 7.9 Hz, 2H), 7.60 (d, *J* = 8.3 Hz, 2H), 7.10 (t, *J* = 2.7 Hz, 1H), 6.61 (s, 1H), 5.76 (s, 1H), 4.67 (s, 1H), 3.55 (s, 3H), 3.29 (s, 1H), 3.28 - 3.21 (m, 1H), 3.00 (s, 1H), 2.85 (s, 1H), 2.64 (dd, *J* = 24.2, 22.4 Hz, 5H), 2.37 - 2.32 (m, 3H), 1.68 (s, 2H).

### Example 16: 4-((3S,4S)-1-(2,2-difluoroethyl)-4-((5-methoxy-7-methyl-1H-indol-4-yl)thio)piperidin-3-yl)benzoic acid and 4-((3R,4R)-1-(2,2-difluoroethyl)-4-((5-methoxy-7-methyl-1H-indol-4-yl)thio)piperidin-3-yl)benzoic acid and 4-((3S,4R)-1-(2,2-difluoroethyl)-4-((5-methoxy-7-methyl-1H-indol-4-yl)thio)piperidin-3-yl)benzoic acid and 4-((3R,4S)-1-(2,2-difluoroethyl)-4-((5-methoxy-7-methyl-1H-indol-4-yl)thio)piperidin-3-yl)benzoic acid

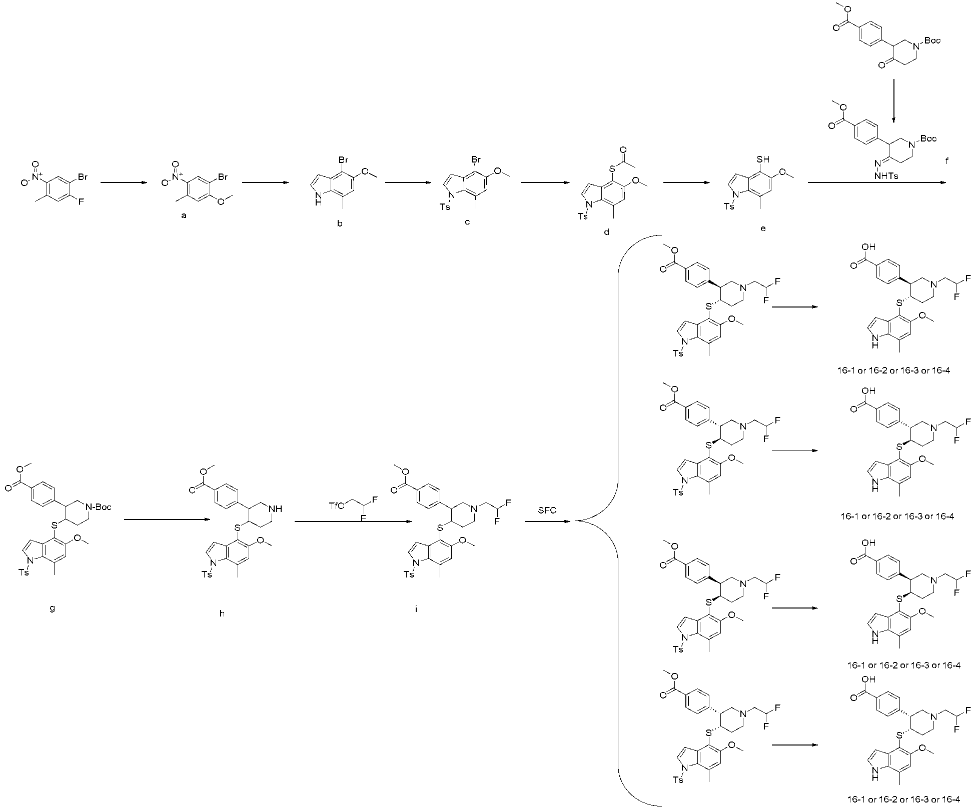

### a) Preparation of 1-bromo-2-methoxy-4-methyl-5-nitrobenzene

1-Bromo-2-fluoro-4-methyl-5-nitrobenzene (100 g) was dissolved in methanol (1 L), and sodium methoxide (25.6 g) was added. The mixture was purged with nitrogen for protection. The mixture was stirred at 30 °C for 16 hours. The reaction solution was filtered, and the filtrate was concentrated under reduced pressure to obtain 73 g of the title compound.

### b) Preparation of 4-bromo-5-methoxy-7-methyl-1H-indole

1-Bromo-2-methoxy-4-methyl-5-nitrobenzene (73 g) was added to a 2 L three-necked flask. Tetrahydrofuran (730 mL) was added. Under nitrogen protection, the temperature was lowered to -40 °C. A solution of vinylmagnesium bromide in tetrahydrofuran (1750 mL, 1.3 M) was slowly added dropwise. The reaction solution was stirred at -40 °C for 3 hours. The reaction was quenched with aqueous ammonium chloride solution (200 mL) and extracted with ethyl acetate (3*200 mL). The organic phase was dried over anhydrous sodium sulfate and filtered. The filtrate was concentrated under reduced pressure to yield a powdery residue, which was purified by column chromatography (mobile phase: petroleum ether/ethyl acetate = 20/1 (V/V)) to obtain 8.5 g of the title compound.

### c) Preparation of 4-bromo-5-methoxy-7-methyl-1-tosyl-1H-indole

4-Bromo-5-methoxy-7-methyl-1H-indole (8.5 g), potassium hydroxide (6.0 g), and N,N-dimethylformamide (100 mL) were added to a reaction flask. The mixture was cooled to 0 °C, and p-toluenesulfonyl chloride (10.1 g) was added. The mixture was warmed to room temperature and stirred for 16 hours, then quenched with water (50 mL) and extracted with ethyl acetate (100 mL). The organic phase was dried over anhydrous sodium sulfate, filtered, and the filtrate was concentrated under reduced pressure to yield a powdery residue, which was purified by column chromatography (mobile phase: petroleum ether/ethyl acetate = 5/1 (V/V)) to obtain 8.1 g of the title compound.

### d) Preparation of S-(5-methoxy-7-methyl-1-tosyl-1H-indol-4-yl) ethanethioate

A solution of S-(5-methoxy-7-methyl-1-tolyl-1H-indol-4-yl) ethanethioate (8 g), potassium thioacetate (2.55 g), tris(dibenzylideneacetone)dipalladium (9.29 g), 4,5-bis(diphenylphosphino)-9,9-dimethylxanthene (17.6 g), N,N-diisopropylethylamine (7.87 g), and 1,4-dioxane (120 mL) was added to a reaction flask. The mixture was heated in a microwave at 140 °C for 2 hours. The reaction mixture was quenched with water (50 mL) and extracted with ethyl acetate (300 mL). The layers were separated, and the organic phase was dried over anhydrous sodium sulfate and filtered. The filtrate was concentrated under reduced pressure to yield a powdery residue, which was purified by column chromatography (mobile phase: petroleum ether/ethyl acetate = 5/1 (V/V)) to obtain 2.6 g of the title compound.

### e) Preparation of 5-methoxy-7-methyl-1-tosyl-1H-indole-4-thiol

S-(5-Methoxy-7-methyl-1-tosyl-1H-indol-4-yl) ethanethioate (300 mg) was dissolved in a mixed solution of tetrahydrofuran/methanol (1.5 mL/3.0 mL). 1 N lithium hydroxide (1 mL) was added. The mixture was purged with nitrogen for protection. The mixture was stirred at room temperature for 1 hour. The reaction solution was diluted with 1 N dilute hydrochloric acid (10 mL) and water (30 mL), and then extracted with ethyl acetate (60 mL). The layers were separated, and the organic phase was dried over anhydrous sodium sulfate and filtered. The filtrate was concentrated under reduced pressure to obtain 260 mg of the title compound.

LCMS m/z = 348 [M+1]⁺.

### f) Preparation of (±)tert-butyl 3-(4-(methoxycarbonyl)phenyl)-4-(2-tosylhydrazineylidene)piperidine-1-carboxylate

(±)Tert-butyl 3-(4-(methoxycarbonyl)phenyl)-4-oxopiperidine-1-carboxylate (2.194 g) and 4-methylbenzenesulfonyl hydrazide (1.838 g) were added to a reaction flask, followed by the addition of methanol (35 mL). The mixture was purged with nitrogen for protection and stirred at room temperature for 16 hours. The mixture was concentrated under reduced pressure to dryness, and the dried residue was purified by reverse-phase column chromatography (WelFlash C18-I, Regular C18 20-40 µm, 330 g; mobile phase A: 10 mmol formic acid/H₂O, B: MeCN; flow rate: 150 mL/min; isocratic elution: 77.5% MeCN) to obtain 0.9 g of the title compound.

### g) Preparation of (±)tert-butyl 4-((5-methoxy-7-methyl-1-tolyl-1H-indol-4-yl)thio)-3-(4-(methoxycarbonyl)phenyl) piperidine-1-carboxylate

5-Methoxy-7-methyl-1-tosyl-1H-indole-4-thiol (260 mg), cesium carbonate (856 mg), 1,4-dioxane (10 mL), and (±)tert-butyl 3-(4-(methoxycarbonyl)phenyl)-4-(2-tosylhydrazineylidene)piperidine-1-carboxylate (590 mg) were added to a reaction flask. Under nitrogen protection, the mixture was stirred at 110 °C for 1 hour. The reaction was diluted with water (30 mL) and extracted with ethyl acetate (3 × 30 mL). The layers were separated, and the combined organic layers were dried over anhydrous sodium sulfate and filtered. The filtrate was concentrated under reduced pressure to yield a powdery residue, which was purified by column chromatography (mobile phase: petroleum ether/ethyl acetate = 5/1 (V/V)) to obtain 470 mg of the title compound.

### h) Preparation of (±)methyl 4-(4-((5-methoxy-7-methyl-1-tolyl-1H-indol-4-yl)thio)piperidin-3-yl)benzoate

(±)-Tert-butyl 4-((5-methoxy-7-methyl-1-tolyl-1H-indol-4-yl)thio)-3-(4-(methoxycarbonyl)phenyl)piperidine-1-carboxylate (470 mg) was dissolved in a 4 M hydrochloric acid solution in 1,4-dioxane (5.0 mL). The mixture was stirred at room temperature for 1 hour, and the reaction solution was concentrated under reduced pressure to obtain 220 mg of the title compound.

### i) Preparation of (±)methyl 4-(1-(2,2-difluoroethyl)-4-((5-methoxy-7-methyl-1-tolyl-1H-indol-4-yl)thio)piperidin-3-yl) benzoate

(±)Methyl 4-(4-((5-methoxy-7-methyl-1-tolyl-1H-indol-4-yl)thio)piperidin-3-yl)benzoate (200 mg), N,N-diisopropylethylamine (226 mg), anhydrous tetrahydrofuran (10 mL), and 2,2-difluoroethyl trifluoromethanesulfonate (227 mg) were added to a reaction flask. The mixture was purged with nitrogen for protection, stirred at 70 °C for 2 hours, and the reaction solution was concentrated under reduced pressure to yield a powdery residue which was purified by column chromatography (mobile phase: petroleum ether/ethyl acetate = 5/1 (V/V)) to obtain 190 mg of a mixture of diastereomers.

The enantiomers of (±)-methyl 4-(1-(2,2-difluoroethyl)-4-((5-methoxy-7-methyl-1-tolyl-1H-indol-4-yl)thio)piperidin-3 -yl)benzoate were resolved by chiral SFC, obtaining peak-1, tr = 2.24 min, 64 mg, peak-2, tr = 2.65 min, 61 mg, peak-3, tr = 2.55 min, 60 mg, and peak-4, tr = 3.38 min, 80 mg. (The resolution method: chromatographic column: DAICEL CHIRALCEL^{®} OZ 250*20 mm, 10 µm; mobile phase A: supercritical CO₂; mobile phase B: methanol (+0.1% 7.0 mol/L ammonia in methanol); A:B = 70:30; flow rate: 100 mL/min; detection wavelength: 214 nm)

Peak-1, tᵣ = 2.24 min

¹HNMR (400 MHz, CDCl₃) δ 7.86 (d, J = 8.4 Hz, 2H), 7.64 (d, J = 3.8 Hz, 1H), 7.48 (d, J = 8.4 Hz, 2H), 7.25 - 7.16 (m, 4H), 6.66 (d, J = 3.8 Hz, 1H), 6.50 (s, 1H), 5.98 - 5.70 (m, 1H), 3.91 (s, 3H), 3.81 (s, 3H), 3.34 - 3.29 (m, 1H), 2.97 - 2.92 (m, 3H), 2.72 (td, J = 15.0, 4.4 Hz, 2H), 2.51 (s, 3H), 2.34 (s, 3H), 2.32 - 2.22 (m, 2H), 1.85 - 1.74 (m, 2H).

Peak-2, tᵣ = 2.65 min

¹HNMR (400 MHz, CDCl₃) δ 7.86 (d, *J =* 8.2 Hz, 2H), 7.64 (d, *J =* 3.8 Hz, 1H), 7.48 (d, *J =* 8.2 Hz, 2H), 7.24 (d, *J* = 8.2 Hz, 2H), 7.19 (d, *J =* 8.2 Hz, 2H), 6.66 (d, *J* = 3.8 Hz, 1H), 6.50 (s, 1H), 6.01 - 5.66 (m, 1H), 3.91 (s, 3H), 3.81 (s, 3H), 3.31 (dt, *J =* 11.0, 5.6 Hz, 1H), 2.97 - 2.92 (m, 3H), 2.72 (td, *J =* 15.0, 4.4 Hz, 2H), 2.51 (s, 3H), 2.34 (s, 3H), 2.32 - 2.20 (m, 2H), 1.85 - 1.74 (m, 2H).

Peak-3, tᵣ = 2.55 min

¹HNMR (400 MHz, CDCl₃) δ 7.90 (d, *J =* 8.4 Hz, 2H), 7.60 (d, *J =* 3.8 Hz, 1H), 7.46 (d, *J =* 8.4 Hz, 2H), 7.33 (d, *J* = 8.2 Hz, 2H), 7.19 (d, *J =* 8.2 Hz, 2H), 6.49 (d, *J =* 3.8 Hz, 2H), 5.90 (tt, *J =* 55.8, 4.4 Hz, 1H), 3.92 (s, 3H), 3.78 - 3.68 (m, 4H), 3.39 - 3.31 (m, 1H), 3.11 (t, *J* = 10.6 Hz, 1H), 2.99 - 2.81 (m, 4H), 2.67 - 2.64 (m, 1H), 2.50 (s, 3H), 2.35 (s, 3H), 1.96 - 1.90 (m, 1H), 1.78 - 1.74 (m, 1H).

Peak-4, tᵣ = 3.38 min

¹HNMR (400 MHz, CDCl₃) δ 7.89 (d, *J =* 8.4 Hz, 2H), 7.60 (d, *J =* 3.8 Hz, 1H), 7.46 (d, *J =* 8.4 Hz, 2H), 7.33 (d, *J* = 8.2 Hz, 2H), 7.19 (d, *J =* 8.2 Hz, 2H), 6.49 (d, *J =* 3.8 Hz, 2H), 5.90 (tt, *J =* 55.8, 4.4 Hz, 1H), 3.92 (s, 3H), 3.78 - 3.69 (m, 4H), 3.40- 3.31 (m, 1H), 3.12 (t, *J* = 10.6 Hz, 1H), 2.99 - 2.82(m, 4H), 2.67 - 2.64 (m, 1H), 2.50 (s, 3H), 2.35 (s, 3H), 1.96 - 1.90 (m, 1H), 1.78 - 1.74 (m, 1H).

### j) Preparation of 4-((3S,4S)-1-(2,2-difluoroethyl)-4-((5-methoxy-7-methyl-1H-indol-4-yl)thio)piperidin-3-yl)benzoic acid and 4-((3R,4R)-1-(2,2-difluoroethyl)-4-((5-methoxy-7-methyl-1H-indol-4-yl)thio)piperidin-3-yl)benzoic acid and 4-((3S,4R)-1-(2,2-difluoroethyl)-4-((5-methoxy-7-methyl-1H-indol-4-yl)thio)piperidin-3-yl)benzoic acid and 4-((3R,4S)-1-(2,2-difluoroethyl)-4-((5-methoxy-7-methyl-1H-indol-4-yl)thio)piperidin-3-yl)benzoic acid

Following the method in step d) of Example 13a, the four title compounds were respectively obtained. Retention times were analyzed by chiral HPLC as follows: 16-1, tr = 3.20 min; 16-2, tr = 3.60 min; 16-3, tr = 3.72 min; 16-4, tr = 5.48 min.

16-1, tr = 3.20 min

¹HNMR (400 MHz, DMSO-*d6*) δ 11.00 (s, 1H), 7.86 (d, *J =* 8.2 Hz, 2H), 7.40 (d, *J =* 8.0 Hz, 2H), 7.29 (t, *J =* 2.8 Hz, 1H), 6.71 (s, 1H), 6.32 - 6.29 (m, 1H), 6.08 (dd, *J* = 57.9, 53.6 Hz, 1H), 3.75 (s, 3H), 2.85 (dd, *J* = 27.0, 11.8 Hz, 4H), 2.75 - 2.62 (m, 2H), 2.44 (s, 3H), 2.30 (dd, *J* = 19.8, 8.3 Hz, 1H), 2.15 (t, *J* = 11.6 Hz, 1H), 1.67 - 1.47 (m, 2H).

16-2, tr = 3.60 min

¹HNMR (400 MHz, DMSO-*d6*) δ 11.01 (s, 1H), 7.86 (d, *J =* 8.1 Hz, 2H), 7.38 (d, *J =* 8.0 Hz, 2H), 7.30 (t, *J =* 2.8 Hz, 1H), 6.71 (s, 1H), 6.32 - 6.29 (m, 1H), 6.23 - 5.91 (m, 1H), 3.76 (s, 3H), 2.92 - 2.78 (m, 4H), 2.74 - 2.63 (m, 2H), 2.45 (s, 3H), 2.30 (dd, *J* = 20.7, 9.9 Hz, 1H), 2.14 (t, *J* = 10.7 Hz, 1H), 1.57 (dd, *J* = 35.0, 9.4 Hz, 2H).

16-3, tr = 3.72 min

¹HNMR (400 MHz, DMSO-*d6*) δ 10.98 (s, 1H), 7.85 (dd, *J* = 14.8, 8.2 Hz, 2H), 7.43 (d, *J* = 8.1 Hz, 2H), 7.26 (t, *J =* 2.8 Hz, 1H), 6.67 (s, 1H), 6.34 - 5.95 (m, 2H), 3.74 (d, *J* = 3.9 Hz, 1H), 3.69 (s, 3H), 3.03 (t, *J* = 10.8 Hz, 2H), 2.92 (d, *J =* 8.7 Hz, 2H), 2.86 - 2.77 (m, 2H), 2.54 (d, *J* = 12.8 Hz, 1H), 2.41 (d, *J* = 8.5 Hz, 3H), 1.77 (d, *J =* 6.6 Hz, 1H), 1.48 (d, *J =* 11.0 Hz, 1H).

16-4, tr = 5.48 min

¹HNMR (400 MHz, DMSO-*d6*) δ 10.98 (s, 1H), 7.84 (dd, *J* = 14.4, 8.3 Hz, 2H), 7.42 (d, *J* = 8.0 Hz, 2H), 7.26 (t, *J =* 2.8 Hz, 1H), 6.67 (s, 1H), 6.34 - 5.99 (m, 2H), 3.74 (d, *J =* 3.8 Hz, 1H), 3.70 (s, 3H), 3.03 (t, *J* = 10.7 Hz, 2H), 2.93 (t, *J* = 10.0 Hz, 2H), 2.87 - 2.76 (m, 2H), 2.54 (d, *J =* 11.2 Hz, 1H), 2.41 (d, *J* = 9.8 Hz, 3H), 1.77 (d, *J* = 8.3 Hz, 1H), 1.48 (d, *J =* 10.5 Hz, 1H).

### Example 17:

### 4-((3S,4S)-4-((5-(methoxy-d3)-7-methyl-1H-indol-4-yl)oxy)-1-(3,3,3-trifluoropropyl)piperidin-3-yl)benzoic acid and 4-((3R,4R)-4-((5-(methoxy-d3)-7-methyl-1H-indol-4-yl)oxy)-1-(3,3,3-trifluoropropyl)piperidin-3-yl)benzoic acid

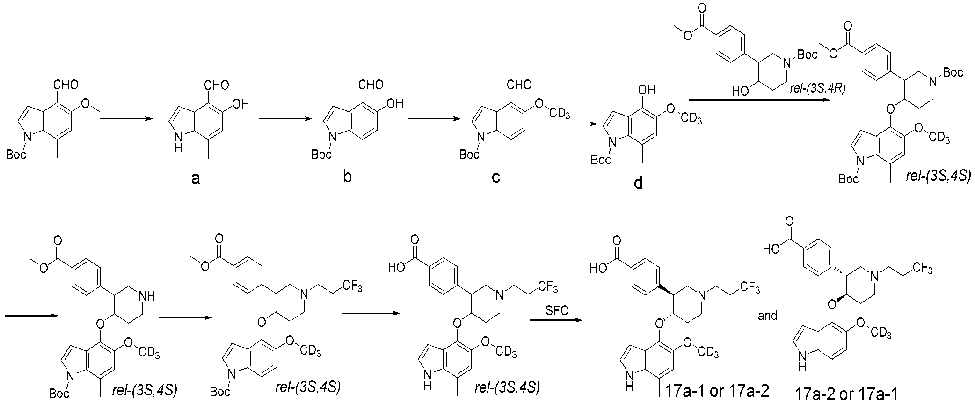

### a) Preparation of 5-hydroxy-7-methyl-1H-indole-4-carbaldehyde

Tert-butyl 4-formyl-5-methoxy-7-methyl-1H-indole-1-carboxylate (5.0 g) and dichloromethane (120 mL) were added to a two-necked flask. The mixture was purged with nitrogen for protection. Aluminum trichloride (23.04 g) was added in batches under an ice-bath, and the mixture was stirred for 10 minutes. Then it was stirred at 40 °C overnight. After the reaction was cooled to room temperature, it was slowly poured into ice-water, and a 3 N dilute aqueous solution of hydrochloric acid was added to quench the reaction. The mixture was extracted with ethyl acetate. The combined organic phases were dried over anhydrous sodium sulfate and filtered. The filtrate was concentrated under reduced pressure to yield a powdery residue, which was purified by column chromatography (mobile phase: petroleum ether/dichloromethane = 3/7 (V/V)) to obtain 1.4 g of the title compound.

### b) Preparation of tert-butyl 4-formyl-5-hydroxy-7-methyl-1H-indole-1-carboxylate

5-Hydroxy-7-methyl-1H-indole-4-carbaldehyde (1.4 g), dichloromethane (40 mL), di-tert-butyl dicarbonate (5.23 g), and 4-dimethylaminopyridine (390 mg) were added to a reaction flask. The mixture was purged with nitrogen for protection and stirred at room temperature overnight. The reaction solution was concentrated under reduced pressure to yield a powdery residue. The residue was purified by column chromatography (mobile phase: petroleum ether/ethyl acetate = 6/1 (V/V)) to obtain 2.03 g of the title compound.

### c) Preparation of tert-butyl 4-formyl-5-(methoxy-d3)-7-methyl-1H-indole-1-carboxylate

Tert-butyl 4-formyl-5-hydroxy-7-methyl-1H-indole-1-carboxylate (0.4 g), N,N-dimethylformamide (20 mL), potassium carbonate (602.4 mg), and deuterated methyl iodide (631.8 mg) were stirred at room temperature overnight. Ethyl acetate and water were added for extraction and separation. The combined organic phases were dried over anhydrous sodium sulfate and filtered. The filtrate was concentrated under reduced pressure to yield a powdery residue, which was purified by column chromatography (mobile phase: petroleum ether/ethyl acetate = 9/1 (V/V)) to obtain 375 mg of the title compound.

### d) Preparation of tert-butyl 4-hydroxy-5-(methoxy-d3)-7-methyl-1H-indole-1-carboxylate

Tert-butyl 4-formyl-5-(methoxy-*d3*)-7-methyl-1H-indole-1-carboxylate (350 mg), tetrahydrofuran (10 mL), and methanol (25 mL) were added to a reaction flask. Hydrogen peroxide (1.75 mL) was added dropwise under an ice-water bath, followed by the dropwise addition of concentrated sulfuric acid (0.2 mL). The reaction was reacted under an ice-water bath for 2.5 hours. A saturated sodium sulfite solution was slowly added under an ice-water bath to quench the reaction. The mixture was extracted with ethyl acetate. The combined organic phases were dried over anhydrous sodium sulfate and filtered. The filtrate was concentrated under reduced pressure to yield a powdery residue, which was purified by column chromatography (mobile phase: petroleum ether/ethyl acetate = 9/1 (V/V)) to obtain 310 mg of the title compound.

### Example 17a:

### 4-((3S,4S)-4-((5-(methoxy-d3)-7-methyl-1H-indol-4-yl)oxy)-1-(3,3,3-trifluoropropyl)piperidin-3-yl)benzoic acid and

### 4-((3R,4R)-4-((5-(methoxy-d3)-7-methyl-1H-indol-4-yl)oxy)-1-(3,3,3-trifluoropropyl)piperidin-3-yl)benzoic acid

It was prepared by referring to the preparation method of Example 13a, and tert-butyl 4-hydroxy-5-methoxy-7-methyl-1H-indole-1-carboxylate in step a) was replaced with tert-butyl 4-hydroxy-5-(methoxy-*d3*)-7-methyl-1H-indole-1-carboxylate, and 2,2-difluoroethyl trifluoromethanesulfonate in step c) was replaced with 3,3,3-trifluoropropyl trifluoromethanesulfonate.

¹HNMR (400 MHz, DMSO-*d6*) δ 12.80 (s, 1H), 10.83 (s, 1H), 7.91 (d, J = 8.2 Hz, 2H), 7.58 (d, J = 8.3 Hz, 2H), 7.17 (t, J = 2.7 Hz, 1H), 6.62 (s, 1H), 6.16 (d, J = 2.0 Hz, 1H), 4.45 (td, J = 9.5, 4.8 Hz, 1H), 3.11 (td, J = 10.3, 3.8 Hz, 1H), 2.89 (dd, J = 22.7, 10.0 Hz, 2H), 2.56 (dd, J = 10.1, 6.7 Hz, 2H), 2.48 - 2.38 (m, 2H), 2.36 (s, 3H), 2.34 - 2.25 (m, 1H), 2.07 (t, J = 10.4 Hz, 1H), 1.68 (dd, J = 27.4, 6.2 Hz, 2H).

LCMS m/z = 480.3 [M+1]⁺.

The enantiomers of (±)-rel-(3S,4S)-4-(4-((5-(methoxy-d3)-7-methyl-1H-indol-4-yl)oxy)-1-(3,3,3-trifluoropropyl) piperidin-3-yl)benzoic acid were resolved by chiral SFC, obtaining 17a-1, tᵣ = 0.88 min and 17a-2, tᵣ = 1.39 min (The resolution method: chromatographic column: DAICELCHIRALPAK^{®}IC 250*20 mm 10µm; mobile phase A: supercritical CO₂; mobile phase B: methanol (+0.1% 7.0 mol/L ammonia in methanol); A:B: 75:25; flow rate:100 mL/min; detection wavelength: 214 nm)

17a-1, tᵣ = 0.88 min

¹HNMR (400 MHz, DMSO-*d6*) δ 12.98 - 12.11 (m, 1H), 10.83 (s, 1H), 7.90 (d, J = 8.1 Hz, 2H), 7.59 (d, J = 8.3 Hz, 2H), 7.16 (t, J = 2.6 Hz, 1H), 6.62 (s, 1H), 6.15 (s, 1H), 4.45 (d, J = 4.9 Hz, 1H), 3.29 (s, 1H), 3.11 (s, 1H), 2.89 (dd, J = 23.0, 10.2 Hz, 2H), 2.53 (d, J = 4.7 Hz, 4H), 2.35 (d, J = 7.1 Hz, 3H), 2.12 - 2.00 (m, 1H), 1.72 (s, 2H).

17a-2, tᵣ = 1.39 min

¹HNMR (400 MHz, DMSO-*d6*) δ 12.80 (s, 1H), 10.83 (s, 1H), 7.91 (d, J = 8.0 Hz, 2H), 7.59 (d, J = 8.0 Hz, 2H), 7.17 (t, J = 2.6 Hz, 1H), 6.62 (s, 1H), 6.15 (s, 1H), 4.45 (d, J = 4.9 Hz, 1H), 3.10 (s, 1H), 2.91 (dd, J = 23.0, 10.2 Hz, 2H), 2.69 - 2.66 (m, 1H), 2.55 (d, J = 4.7 Hz, 3H), 2.36 (d, J = 7.1 Hz, 3H), 2.34 - 2.31 (m, 1H), 2.07 (m, 1H), 1.72 (s, 2H).

### Example 18 :

### (±)-rel-(3S,4S)-4-((7-methyl-5-(trifluoromethoxy)-1H-indol-4-yl)oxy)-1-(3,3,3-trifluoropropyl)piperidin-3-yl)benzoic acid

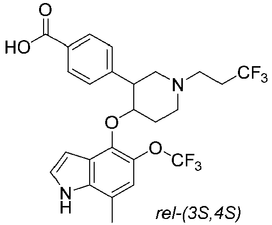

It was prepared by referring to the preparation method of Example 17, and tert-butyl 4-hydroxy-5-(methoxy-*d3*)-7-methyl-1H-indole-1-carboxylate was replaced with tert-butyl 4-hydroxy-7-methyl-5-(trifluoromethoxy)-1H-indole-1-carboxylate.

¹HNMR (400 MHz, DMSO-d6) δ 10.80 (s, 1H), 7.88 (d, J = 8.2 Hz, 2H), 7.51 (d, J = 8.2 Hz, 2H), 7.14 (s, 1H), 6.60 (s, 1H), 6.08 (s, 1H), 4.42 (td, J = 10.3, 4.1 Hz, 1H), 3.22 - 3.10 (m, 2H), 2.34 (s, 3H), 1.95 (d, J = 11.2 Hz, 1H), 1.77 (dt, J = 21.8, 13.9 Hz, 3H), 1.63 - 1.48 (m, 1H), 1.37 (t, J = 13.3 Hz, 1H), 1.18 (t, J = 7.0 Hz, 3H).

LCMS m/z = 530.1 [M+1]⁺.

### Example 19: 4-(1R,2R,5S)-(5-ethoxy-2-((5-methoxy-7-methyl-1H-indol-4-yl)thio)cyclohexyl)benzoic acid and 4-(1S,2R,SR)-(5-ethoxy-2-((5-methoxy-7-methyl-1H-indol-4-yl)thio)cyclohexyl)benzoic acid and 4-(1R,2S,SS)-(5-ethoxy-2-((5-methoxy-7-methyl-1H-indol-4-yl)thio)cyclohexyl)benzoic acid and 4-(1S,2S,5R)-(5-ethoxy-2-((5-methoxy-7-methyl-1H-indol-4-yl)thio)cyclohexyl)benzoic acid and 4-(1R,2S,SR)-(5-ethoxy-2-((5-methoxy-7-methyl-1H-indol-4-yl)thio)cyclohexyl)benzoic acid and 4-(1S,2R,SS)-(5-ethoxy-2-((5-methoxy-7-methyl-1H-indol-4-yl)thio)cyclohexyl)benzoic acid and 4-(1R,2R,5R)-(5-ethoxy-2-((5-methoxy-7-methyl-1H-indol-4-yl)thio)cyclohexyl)benzoic acid and 4-(1S,2S,SS)-(5-ethoxy-2-((5-methoxy-7-methyl-1H-indol-4-yl)thio)cyclohexyl)benzoic acid

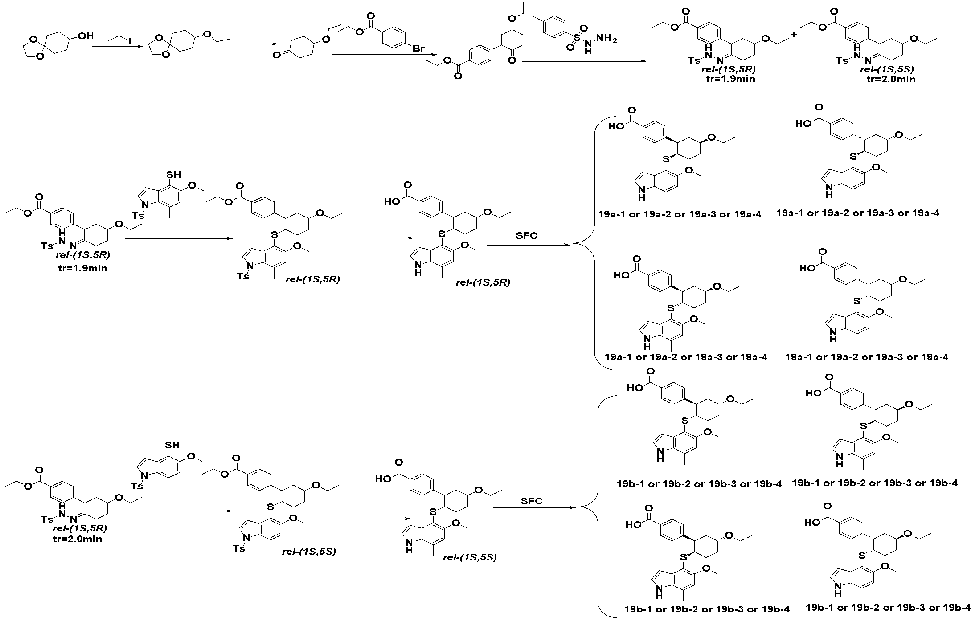

### a) Preparation of 8-ethoxy-1,4-dioxaspiro[4.5]decane

1,4-Dioxaspiro[4.5]decan-8-ol (50 g) was dissolved in tetrahydrofuran (500 mL). Under the protection of nitrogen replacement, sodium hydride (15 g) was added at 0 °C, and the mixture was stirred for 2 hours. Ethyl iodide (98.4 g) was added at 0 °C, and then the mixture was stirred at room temperature for 16 hours. The reaction was quenched with aqueous ammonium chloride solution, and then extracted with ethyl acetate (100 mL). The layers were separated, and the organic phase was dried over anhydrous sodium sulfate and filtered. The filtrate was concentrated under reduced pressure to obtain 65 g of the title compound.

### b) Preparation of 4-ethoxycyclohexan-1-one

8-Ethoxy-1,4-dioxaspiro[4.5]decane (65 g), dilute hydrochloric acid (3 M, 234 mL), and tetrahydrofuran (273 mL) were added to a reaction flask. The mixture was stirred at 90 °C for 2 hours. The reaction solution was diluted with water (50 mL) and extracted with ethyl acetate (3 × 100 mL). The layers were separated, and the combined organic layers were dried over anhydrous sodium sulfate and filtered. The filtrate was concentrated under reduced pressure to obtain 40 g of the title compound.

### c) (±)-ethyl 4-(5-ethoxy-2-oxocyclohexyl)benzoate (mixture of diastereomers)

4-Ethoxycyclohexan-1-one (25 g), ethyl 4-iodobenzoate (48.5 g), tris(dibenzylideneacetone)dipalladium(0) (16 g), 4,5-bis(diphenylphosphino)-9,9-dimethylxanthene (20 g), cesium carbonate (115 g), and anhydrous 1,4-dioxane (500 mL) were added to a reaction flask. The mixture was purged with nitrogen for protection and stirred at 100 °C for 16 hours. After the reaction solution was cooled down, it was filtered. The filtrate was diluted with water (150 mL) and extracted with ethyl acetate (3 × 150 mL). The layers were separated, and the combined organic layers were dried over anhydrous sodium sulfate, filtered and filtered. The filtrate was concentrated under reduced pressure to yield a powdery residue, which was purified by column chromatography (mobile phase: petroleum ether/ethyl acetate = 5/1 (V/V)) to obtain 14.2 g of the title compound.

### d) (±)-ethyl (5-ethoxy-2-(2-tosylhydrazineylidene)cyclohexyl)benzoate

(±)-Ethyl 4-(5-ethoxy-2-oxocyclohexyl)benzoate (6.2 g), 4-methylbenzenesulfonyl hydrazide (4.772 g), and toluene (60 mL) were added to a reaction flask. The reaction was stirred at 50 °C for 1.5 hours. The reaction solution was concentrated under reduced pressure. The residue after evaporation was diluted with water (80 mL) and extracted with ethyl acetate (100 mL). The layers were separated, and the combined organic layers were dried over anhydrous sodium sulfate, filtered and filtered. The filtrate was concentrated under reduced pressure to yield a powdery residue, which was purified by column chromatography (mobile phase: petroleum ether/ethyl acetate = 5/1 (V/V)) and then by a reverse-phase column (WelFlash C18-I, Regular C18 20-40 µm, 120 g; mobile phase A: 10 mmol NH₄HCO₃/H₂O, B: MeCN; flow rate: 80 mL/min; gradient: 60% MeCN). According to the corresponding elution order, the title compounds (±)-ethyl 4-(5-ethoxy-2-(2-tosylhydrazineylidene)cyclohexyl)benzoate (diastereomer-1, peak-1 tr = 1.9 min) and (±)-ethyl 4-(5-ethoxy-2-(2-tosylhydrazineylidene)cyclohexyl)benzoate (diastereomer-2, peak-2 tr = 2.0 min) were obtained respectively.

### Peak 1: (±)-ethyl 4-(5-ethoxy-2-(2-tosylhydrazineylidene)cyclohexyl)benzoate (diastereomer-1)

¹H NMR (400 MHz, DMSO-*d₆*) δ 10.06 (s, 1H), 7.85 (d, *J =* 8.2 Hz, 2H), 7.19 - 7.13 (m, 6H), 4.36 (q, *J =* 7.2 Hz, 2H), 3.68 - 2.61 (m, 2H), 3.46 (q, *J =* 7.0 Hz, 2H), 2.86 - 2.77 (m, 1H), 2.36 (s, 3H), 2.19 (d, *J =* 11.6 Hz, 1H), 2.10 (d, *J =* 11.2 Hz, 1H), 2.01 - 1.92 (m, 1H), 1.73 - 1.64 (m, 1H), 1.39 - 1.29 (m, 4H), 1.07 (t, *J* = 7.0 Hz, 3H).

### Peak-2: (±)-ethyl 4-(5-ethoxy-2-(2-tosylhydrazineylidene)cyclohexyl)benzoate (diastereomer-2)

¹HNMR (400 MHz, DMSO-*d₆*) δ 10.09 (s, 1H), 7.84 (d, *J =* 8.3 Hz, 2H), 7.27 (d, *J =* 8.3 Hz, 2H), 7.16 (dd, *J =* 14.9, 8.2 Hz, 4H), 4.35 (q, *J* = 7.1 Hz, 2H), 3.77 (dd, *J =* 11.1, 4.9 Hz, 1H), 3.70 (s, 1H), 3.51 - 3.43 (m, 2H), 2.48 (d, *J* = 4.7 Hz, 1H), 2.38 (s, 4H), 2.26 - 2.16 (m, 1H), 2.14 - 2.06 (m, 1H), 2.04 - 1.97 (m, 1H), 1.90 (dd, *J* = 13.6, 3.8 Hz, 1H), 1.73 - 1.61 (m, 1H), 1.36 (t, *J* = 7.1 Hz, 3H), 1.14 (t, *J =* 7.0 Hz, 3H).

### Example -19a: 4-(1R,2R,SS)-(5-ethoxy-2-((5-methoxy-7-methyl-1H-indol-4-yl)thio)cyclohexyl)benzoic acid and 4-(1S,2R,5R)-(5-ethoxy-2-((5-methoxy-7-methyl-1H-indol-4-yl)thio)cyclohexyl)benzoic acid and 4-(1R,2S,SS)-(5-ethoxy-2-((5-methoxy-7-methyl-1H-indol-4-yl)thio)cyclohexyl)benzoic acid and 4-(1S,2S,SR)-(5-ethoxy-2-((5-methoxy-7-methyl-1H-indol-4-yl)thio)cyclohexyl)benzoic acid

### a) (±)-ethyl 4-(5-ethoxy-2-((5-methoxy-7-methyl-1-tolyl-1H-indol-4-yl)thio)cyclohexyl)benzoate

5-Methoxy-7-methyl-1-tolyl-1H-indole-4-thiol (650 mg), (±)-ethyl 4-(5-ethoxy-2-(2-tosylhydrazineylidene)cyclohexyl) benzoate (diastereomer-1, peak-1 tr = 1.9 min) (1.286 g), 1,4-dioxane (30 mL), and cesium carbonate (2.134 g) were added to a reaction flask. The mixture was purged with nitrogen for protection and stirred at 110 °C for 1 hour. The reaction solution was diluted with water (50 mL) and extracted three times with ethyl acetate (150 mL). The layers were separated, and the combined organic phase was washed with saturated brine, dried over anhydrous sodium sulfate, and filtered. The filtrate was concentrated under reduced pressure to yield a powdery residue, which was purified by column chromatography (mobile phase: petroleum ether/ethyl acetate = 10/1 (V/V)) to obtain 300 mg of the title compound.

### b) Preparation of 4-(1R,2R,5S)-(5-ethoxy-2-((5-methoxy-7-methyl-1H-indol-4-yl)thio)cyclohexyl)benzoic acid and 4-(1S,2R,5R)-(5-ethoxy-2-((5-methoxy-7-methyl-1H-indol-4-yl)thio)cyclohexyl)benzoic acid and 4-(1R,2S,SS)-(5-ethoxy-2-((5-methoxy-7-methyl-1H-indol-4-yl)thio)cyclohexyl)benzoic acid and 4-(1S,2S,SR)-(5-ethoxy-2-((5-methoxy-7-methyl-1H-indol-4-yl)thio)cyclohexyl)benzoic acid

(±)-Ethyl 4-(5-ethoxy-2-((5-methoxy-7-methyl-1-tolyl-1H-indol-4-yl)thio)cyclohexyl)benzoate (300 mg) and LiOH (1.17 g) were added to a mixed solution of methanol/water (15 mL/6 mL). The reaction was stirred at 70 °C for 16 hours, then concentrated under reduced pressure. The residue after evaluation was diluted with water. The pH was adjusted to neutral with 1N dilute hydrochloric acid. Then it was extracted with ethyl acetate. The organic phase was dried over anhydrous sodium sulfate and filtered. The filtrate was concentrated under reduced pressure to yield a powdery residue, which was purified by column chromatography (mobile phase: dichloromethane/methanol = 20/1 (V/V)) to obtain 160 mg of the mixture of the title isomers.

LCMS m/z = 438.1[M-1]⁺.

The mixture of isomers was resolved by chiral SFC, obtaining 19a-1, tᵣ = 5.45 min and 19a-2, tᵣ = 6.03 min, 19a-3, tᵣ = 5.33 min and 19a-4, tᵣ = 7.81 min (The resolution method: chromatographic column: DAICELCHIRALPAK^{®}IG 250*20 mm, 10 µm; mobile phase A: supercritical CO₂; mobile phase B: methanol (+0.1% 7.0 mol/L ammonia in methanol); A:B: 50:50; flow rate:100 mL/min; detection wavelength: 214 nm)

19a-1, tᵣ = 5.45 min

¹HNMR (400 MHz, DMSO-*d6*) 10.98 (s, 1H), 7.89 (d, *J* = 8.2 Hz, 2H), 7.45 (d, *J* = 8.2 Hz, 2H), 7.28 (t, *J =* 2.8 Hz, 1H), 6.69 (s, 1H), 6.29 - 6.23 (m, 1H), 3.74 (s, 3H), 3.34 - 3.33 (m, 3H), 3.32 - 3.30 (m, 2H), 2.78 - 2.67 (m, 1H), 2.44 (s, 3H), 2.05 (d, *J* = 13.5 Hz, 1H), 1.87 (d, *J* = 11.8 Hz, 1H), 1.70 (dd, *J* = 13.6, 3.5 Hz, 1H), 1.38 (dd, *J* = 23.5, 12.3 Hz, 2H), 1.03 (t, *J* = 7.0 Hz, 3H).

LCMS m/z = 438.1[M-1]⁺.

19a-2, tᵣ = 6.03 min

¹HNMR (400 MHz, DMSO-*d6*) δ10.98 (s, 1H), 7.89 (d, *J =* 8.2 Hz, 2H), 7.45 (d, *J* = 8.2 Hz, 2H), 7.28 (t, *J* = 2.8 Hz, 1H), 6.69 (s, 1H), 6.29 - 6.23 (m, 1H), 3.74 (s, 3H), 3.34 - 3.33 (m, 3H), 3.32 - 3.30 (m, 2H), 2.79 - 2.66 (m, 1H), 2.43 (s, 3H), 2.05 (d, *J* = 10.4 Hz, 1H), 1.87 (d, *J* = 11.9 Hz, 1H), 1.70 (dd, *J* = 13.2, 3.2 Hz, 1H), 1.38 (dd, *J* = 23.5, 12.1 Hz, 2H), 1.03 (t, *J* = 7.0 Hz, 3H).

LCMS m/z = 438.1[M-1]⁺.

19a-3,tᵣ = 5.33 min

¹HNMR (400 MHz, DMSO-*d6*) δ 10.95 (s, 1H), 7.90 (d, *J =* 8.2 Hz, 2H), 7.38 (d, *J =* 8.2 Hz, 2H), 7.24 (t, *J =* 2.8 Hz, 1H), 6.65 (s, 1H), 6.11 - 6.04 (m, 1H), 3.71 - 3.68 (m, 1H), 3.66 (s, 3H), 3.55 - 3.48 (m, 3H), 3.24 - 3.16 (m, 1H), 2.41 (s, 3H), 2.08 - 1.88 (m, 3H), 1.71 - 1.47 (m, 3H), 1.13 (t, *J* = 7.0 Hz, 3H).

LCMS m/z = 438.1[M-1]⁺.

19a-4,tr = 7.81 min

¹HNMR (400 MHz, DMSO-*d6*) δ 10.96 (s, 1H), 7.82 (d, *J* = 8.1 Hz, 2H), 7.26 - 7.22 (m, 2H), 7.21 (s, 1H), 6.66 (s, 1H), 6.11 - 6.08 (m, 1H), 3.70 (s, 1H), 3.67 (s, 3H), 3.51 (tdd, *J =* 9.3, 7.0, 2.3 Hz, 3H), 3.14 (d, *J =* 12.8 Hz, 1H), 2.42 (s, 3H), 2.08 - 1.91 (m, 3H), 1.55 (dd, *J* = 42.6, 29.5 Hz, 3H), 1.13 (t, *J* = 7.0 Hz, 3H).

LCMS m/z = 438.1[M-1]⁺.

### Example -19b: 4-(1R,2S,5R)-(5-ethoxy-2-((5-methoxy-7-methyl-1H-indol-4-yl)thio)cyclohexyl)benzoic acid and 4-(1S,2R,SS)-(5-ethoxy-2-((5-methoxy-7-methyl-1H-indol-4-yl)thio)cyclohexyl)benzoic acid and 4-(1R,2R,5R)-(5-ethoxy-2-((5-methoxy-7-methyl-1H-indol-4-yl)thio)cyclohexyl)benzoic acid and 4-(1S,2S,5S)-(5-ethoxy-2-((5-methoxy-7-methyl-1H-indol-4-yl)thio)cyclohexyl)benzoic acid

It was prepared by referring to the preparation method of Example 19a, and (±)-ethyl 4-(5-ethoxy-2-(2-tosylhydrazineylidene)cyclohexyl)benzoate (diastereomer-1) in step a) was replaced with (±)-ethyl 4-(5-ethoxy-2-(2-tosylhydrazineylidene)cyclohexyl)benzoate (diastereomer-2).

The mixture of isomers was resolved by chiral SFC, obtaining 19b-1, tᵣ = 5.14 min and 19b-2, tᵣ = 5.73 min, 19b-3, tᵣ = 5.20 min and 19b-4, tᵣ = 6.30 min (The resolution method: chromatographic column: DAICELCHIRALPAK^{®}IC 250*20 mm, 10µm; mobile phase A: supercritical CO₂; mobile phase B: methanol (+0.1% 7.0 mol/L ammonia in methanol); A:B: 55:45; flow rate:70 mL/min; detection wavelength: 214 nm)

19b-1, tᵣ = 5.14 min

¹HNMR (400 MHz, DMSO-*d₆*) δ 10.97 (s, 1H), 7.88 (d, *J* = 8.2 Hz, 2H), 7.44 (d, *J* = 8.2 Hz, 2H), 7.28 (t, *J* = 2.8 Hz, 1H), 6.69 (s, 1H), 6.28 (dd, *J* = 2.8, 2.0 Hz, 1H), 3.75 (S, 3H), 3.56 (s, 1H), 3.47 - 3.40 (m, 2H), 3.19 - 3.12 (m, 1H), 2.93 (td, *J* = 12.0, 3.4 Hz, 1H), 2.43 (s, 3H), 1.90 (d, *J* = 11.4 Hz, 1H), 1.75 (d, *J* = 13.8 Hz, 1H), 1.63 (dd, *J* = 23.8, 11.8 Hz, 2H), 1.44 - 1.40 (m, 1H), 1.33 - 1.24 (m, 1H), 1.14 (t, *J* = 7.0 Hz, 3H).

19b-2, tᵣ = 5.73 min

¹HNMR (400 MHz, DMSO-*d₆*) δ 10.96 (s, 1H), 7.87 (d, *J* = 8.0 Hz, 2H), 7.39 (d, *J* = 8.2 Hz, 2H), 7.27 (t, *J* = 2.8 Hz, 1H), 6.69 (s, 1H), 6.32 - 6.24 (m, 1H), 3.73 (s, 3H), 3.46 - 3.36 (m, 4H), 2.91 (td, *J* = 11.8, 3.4 Hz, 1H), 2.42 (s, 3H), 1.89 (d, *J* = 11.4 Hz, 1H), 1.73 (d, *J* = 13.8 Hz, 1H), 1.63 (t, *J* = 12.2 Hz, 2H), 1.44 - 1.37 (m, 1H), 1.33 - 1.26 (m, 1H), 1.13 (t, *J* = 7.0 Hz, 3H).

19b-3, tᵣ = 5.20 min

¹HNMR (400 MHz, DMSO-*d₆*) δ 10.95 (s, 1H), 7.90 (d, *J* = 8.4 Hz, 2H), 7.45 (d, *J* = 8.4 Hz, 2H), 7.24 (t, *J* = 2.8 Hz, 1H), 6.65 (s, 1H), 6.08 (dd, *J* = 2.8, 2.0 Hz, 1H), 3.80 - 3.76 (m, 2H), 3.66 (s, 3H), 3.51 - 3.47 (m, 1H), 3.44 - 3.28 (m, 2H), 2.42 (s, 3H), 2.25 - 2.10 (m, 2H), 1.97 - 1.82 (m, 2H), 1.56 (d, *J* = 13.4 Hz, 1H), 1.33 - 1.25 (m, 1H), 1.09 (t, *J* = 7.0 Hz, 3H).

19b-4, tᵣ = 6.30 min

¹HNMR (400 MHz, DMSO-*d₆*) δ 10.96 (s, 1H), 7.90 (d, *J* = 8.2 Hz, 2H), 7.43 (d, *J* = 8.2 Hz, 2H), 7.24 (t, *J* = 2.7 Hz, 1H), 6.65 (s, 1H), 6.15 - 6.03 (m, 1H), 3.79 (s, 2H), 3.66 (s, 3H), 3.47 (s, 1H), 3.40 (d, *J* = 7.0 Hz, 2H), 2.41 (s, 3H), 2.28 - 2.09 (m, 2H), 1.97 - 1.81 (m, 2H), 1.55 (d, *J* = 13.4 Hz, 1H), 1.33 - 1.25 (m, 1H), 1.09 (t, *J* = 7.0 Hz, 3H).

### Example 20: 4-((1R,2S,SR)-5-ethoxy-2-((5-methoxy-7-methyl-1H-indol-4-yl)oxy)cyclohexyl)benzoic acid and 4-((1S,2S,5S)-5-ethoxy-2-((5-methoxy-7-methyl-1H-indol-4-yl)oxy)cyclohexyl)benzoic acid and 4-((1R,2R,5R)-5-ethoxy-2-((5-methoxy-7-methyl-1H-indol-4-yl)oxy)cyclohexyl)benzoic acid and 4-((1S,2R,SS)-5-ethoxy-2-((5-methoxy-7-methyl-1H-indol-4-yl)oxy)cyclohexyl)benzoic acid and 4-((1R,2R,SS)-5-ethoxy-2-((5-methoxy-7-methyl-1H-indol-4-yl)oxy)cyclohexyl)benzoic acid and 4-((1S,2R,SR)-5-ethoxy-2-((5-methoxy-7-methyl-1H-indol-4-yl)oxy)cyclohexyl)benzoic acid and 4-((1R,2S,SS)-5-ethoxy-2-((5-methoxy-7-methyl-1H-indol-4-yl)oxy)cyclohexyl)benzoic acid and 4-((1S,2S,5R)-5-ethoxy-2-((5-methoxy-7-methyl-1H-indol-4-yl)oxy)cyclohexyl)benzoic acid

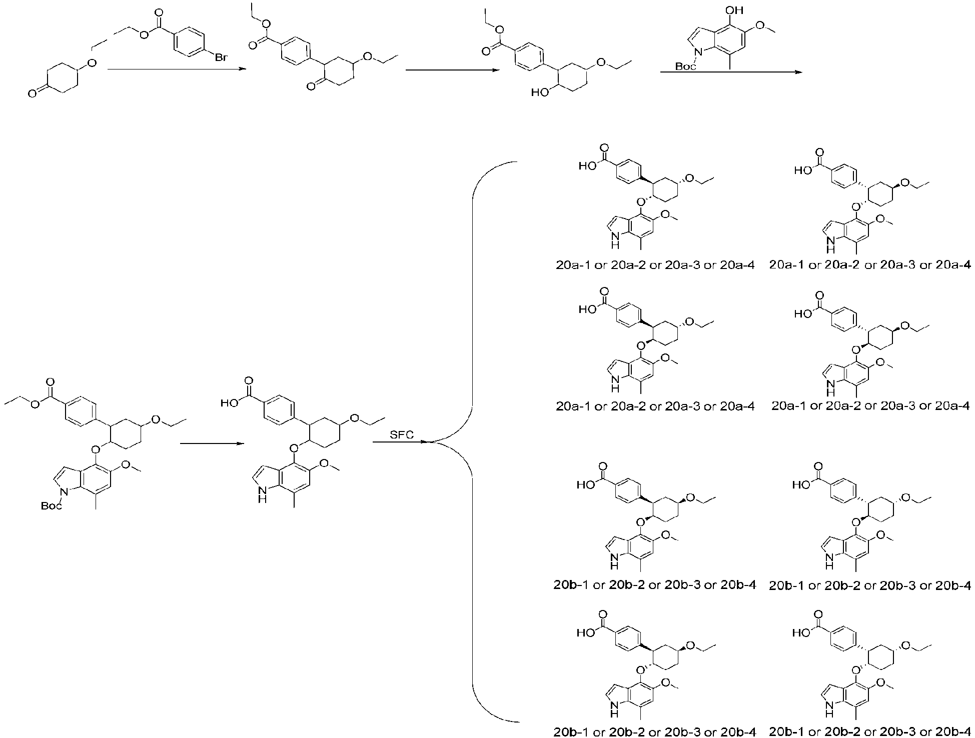

### a) (±)-ethyl 4-(5-ethoxy-2-oxocyclohexyl)benzoate (mixture of diastereomers)

Following the preparation method in step c of Example 19, the title compound (±)-ethyl 4-(5-ethoxy-2-oxocyclohexyl)benzoate was obtained. The product was purified by column chromatography (mobile phase: petroleum ether/ethyl acetate = 5/1 (V/V)). According to the corresponding elution order, (±)-ethyl 4-(5-ethoxy-2-oxocyclohexyl)benzoate (diastereomer-1, peak-1 tr = 3.8 min) and (±)-ethyl 4-(5-ethoxy-2-oxocyclohexyl) benzoate (diastereomer-2, peak-2 tr = 3.5 min) were obtained.

(±)-ethyl 4-(5-ethoxy-2-oxocyclohexyl)benzoate (diastereomer-1, peak-1 tr = 3.8 min)

¹HNMR (400 MHz, DMSO-*d6*) δ 7.88 (d, *J =* 8.1 Hz, 2H), 7.30 (t, *J =* 8.2 Hz, 2H), 4.34 - 4.27 (m, 2H), 4.05 (dd, *J* = 12.8, 5.7 Hz, 1H), 3.83 (s, 1H), 3.54 (dd, *J =* 12.9, 5.9 Hz, 2H), 2.77 - 2.65 (m, 1H), 2.32 - 2.23 (m, 2H), 2.18 (dd, *J =* 13.9, 4.6 Hz, 2H), 1.94 (td, *J* = 13.3, 6.6 Hz, 1H), 1.32 (dd, *J* = 12.2, 5.1 Hz, 3H), 1.19 (dt, *J* = 7.1, 5.6 Hz, 3H).

(±)-ethyl 4-(5-ethoxy-2-oxocyclohexyl)benzoate (diastereomer-2, peak-2 tr = 3.5 min)

¹HNMR (400 MHz, DMSO-*d6*) δ 7.88 (d, *J =* 8.1 Hz, 2H), 7.30 (t, *J =* 8.2 Hz, 2H), 4.34 - 4.27 (m, 2H), 4.05 (dd, *J* = 12.8, 5.7 Hz, 2H), 3.83 (s, 2H), 3.54 (dd, *J =* 12.9, 5.9 Hz, 1H), 2.77 - 2.65 (m, 1H), 2.32 - 2.23 (m, 2H), 2.18 (dd, *J =* 13.9, 4.6 Hz, 1H), 1.94 (td, *J* = 13.3, 6.6 Hz, 1H), 1.32 (dd, *J* = 12.2, 5.1 Hz, 3H), 1.19 (dt, *J* = 7.1, 5.6 Hz, 3H).

Example 20a: 4-((1R,2S,SR)-5-ethoxy-2-((5-methoxy-7-methyl-1H-indol-4-yl)oxy)cyclohexyl)benzoic acid and 4-((1S,2S,5S)-5-ethoxy-2-((5-methoxy-7-methyl-1H-indol-4-yl)oxy)cyclohexyl)benzoic acid and 4-((1R,2R,5R)-5-ethoxy-2-((5-methoxy-7-methyl-1H-indol-4-yl)oxy)cyclohexyl)benzoic acid and 4-((1S,2R,5S)-5-ethoxy-2-((5-methoxy-7-methyl-1H-indol-4-yl)oxy)cyclohexyl)benzoic acid

### a) Preparation of (±)-ethyl 4-(5-ethoxy-2-oxocyclohexyl)benzoate

(±)-Ethyl 4-(5-ethoxy-2-oxocyclohexyl)benzoate (diastereomer-1, peak-1 tᵣ = 3.8 min) (15 g) was added to a three-necked flask. Under nitrogen protection, tetrahydrofuran (300 mL) was added. The reaction system was cooled to -78 °C, and 1 M lithium tri-sec-butylborohydride (103 mL) was slowly added dropwise. The mixture was reacted at -78 °C for 1 hour. The reaction solution was poured into an ice-cold saturated ammonium chloride aqueous solution and extracted with ethyl acetate (3*200 mL). The organic phases were combined after separation, washed with saturated brine solution (3* 200 mL), and dried over anhydrous sodium sulfate and filtrated. The filtrate was concentrated under reduced pressure to yield a powdery residue, which was purified by column chromatography (mobile phase: petroleum ether/ethyl acetate = 8/1 (V/V)) to obtain 14 g of the title compound.

### b) Preparation of (±)-tert-butyl 4-((4-ethoxy-2-(4-(ethoxycarbonyl)phenyl)cyclohexyl)oxy)-5-methoxy-7-methyl-1H-indole-1-carboxylate

(±)-Ethyl 4-(5-ethoxy-2-oxocyclohexyl)benzoate (7.88 g), tert-butyl 4-hydroxy-5-methoxy-7-methyl-1H-indole-1-carboxylate (7.42 g), triphenylphosphine (14.14 g), and anhydrous tetrahydrofuran (400 mL) were added to a three-necked flask. The mixture was purged with nitrogen for protection. Diisopropyl azodicarboxylate (10.9 g) was added dropwise at 0 °C. The reaction system was warmed to room temperature and reacted for 16 hours, then diluted with water (500 mL) and extracted with ethyl acetate (3 × 200 mL). The layers were separated, and the combined organic phase was dried over anhydrous sodium sulfate and filtered. The filtrate was concentrated under reduced pressure to yield a powdery residue, which was purified by column chromatography (mobile phase: petroleum ether/ethyl acetate = 20/1 (V/V)) to obtain 4.2 g of the title compound.

### c) Preparation of 4-((1R,2S,5R)-5-ethoxy-2-((5-methoxy-7-methyl-1H-indol-4-yl)oxy)cyclohexyl)benzoic acid and 4-((1S,2S,5S)-5-ethoxy-2-((5-methoxy-7-methyl-1H-indol-4-yl)oxy)cyclohexyl)benzoic acid and 4-((1R,2R,5R)-5-ethoxy-2-((5-methoxy-7-methyl-1H-indol-4-yl)oxy)cyclohexyl)benzoic acid and 4-((1S,2R,5S)-5-ethoxy-2-((5-methoxy-7-methyl-1H-indol-4-yl)oxy)cyclohexyl)benzoic acid

The mixture of isomers was obtained by hydrolysis following the same hydrolysis procedure in Example 17.

LCMS m/z = 424.2[M+1]⁺.

The mixture of isomers was resolved by chiral SFC, obtaining 20a-1, tᵣ = 4.70 min and 20a-2, tᵣ = 4.80 min, 20a-3, tᵣ = 3.43 min and 20a-4, tᵣ = 3.59 min (The resolution method: chromatographic column: DAICELCHIRALCEL^{®}AD 250*20 mm, 10 µm; mobile phase A: supercritical CO₂; mobile phase B: methanol (+0.1% 7.0 mol/L ammonia in methanol); A:B: 70:30;flow rate:100 mL/min; detection wavelength: 214 nm)

20a-1, tᵣ = 4.70 min

¹HNMR (400 MHz, DMSO-*d6*) δ 10.79 (s, 1H), 7.90 (d, *J =* 8.2 Hz, 2H), 7.57 (d, *J =* 8.2 Hz, 2H), 7.05 (t, *J =* 2.6 Hz, 1H), 6.60 (s, 1H), 5.60 (s, 1H), 4.64 (s, 1H), 3.84 (s, 1H), 3.55 (s, 3H), 3.45 (q, *J =* 6.9 Hz, 2H), 3.34 (s, 1H), 2.46 (d, *J =* 13.7 Hz, 1H), 2.33 (s, 3H), 2.13 (t, *J =* 13.0 Hz, 1H), 1.88 (d, *J =* 12.4 Hz, 1H), 1.66 (dd, *J* = 25.0, 13.2 Hz, 2H), 1.54 (d, *J* = 12.7 Hz, 1H), 1.14 (t, *J =* 7.0 Hz, 3H).

LCMS m/z = 424.2[M+1]⁺.

20a-2, tᵣ = 4.80 min

¹HNMR (400 MHz, DMSO-*d6*) δ 10.79 (s, 1H), 7.90 (d, *J =* 8.1 Hz, 2H), 7.56 (d, *J =* 8.1 Hz, 2H), 7.05 (t, *J =* 2.6 Hz, 1H), 6.60 (s, 1H), 5.60 (s, 1H), 4.64 (s, 1H), 3.84 (s, 1H), 3.55 (s, 3H), 3.45 (q, *J =* 7.0 Hz, 2H), 3.35 (s, 1H), 2.46 (d, *J =* 13.7 Hz, 1H), 2.33 (s, 3H), 2.13 (t, *J =* 13.6 Hz, 1H), 1.88 (d, *J =* 12.7 Hz, 1H), 1.65 (dd, *J* = 24.9, 13.8 Hz, 2H), 1.53 (d, *J* = 11.8 Hz, 1H), 1.14 (t, *J =* 7.0 Hz, 3H).

LCMS m/z = 424.2[M+1]⁺.

20a-3, tᵣ = 3.43 min

¹HNMR (400 MHz, DMSO-d6) δ 12.64 (s, 1H), 10.79 (s, 1H), 7.89 (d, *J =* 8.1 Hz, 2H), 7.54 (d, *J =* 8.2 Hz, 2H), 7.14 (t, *J* = 2.6 Hz, 1H), 6.60 (s, 1H), 6.08 (d, *J =* 2.2 Hz, 1H), 4.43 (td, *J =* 10.3, 4.0 Hz, 1H), 3.57 (s, 1H), 3.56 (s, 3H), 3.44 (q, *J* = 7.0 Hz, 2H), 3.22 - 3.14 (m, 1H), 2.34 (s, 3H), 1.95 (d, *J =* 11.1 Hz, 1H), 1.77 (dt, *J* = 21.9, 14.3 Hz, 3H), 1.60 - 1.50 (m, 1H), 1.37 (t, *J* = 13.3 Hz, 1H), 1.18 (t, *J* = 7.0 Hz, 3H).

LCMS m/z = 424.2[M+1]⁺.

20a-4, tᵣ = 3.59 min

¹HNMR (400 MHz, DMSO-*d6*) δ 13.29 - 11.95 (m, 1H), 10.80 (s, 1H), 7.90 (d, *J =* 8.2 Hz, 2H), 7.55 (d, *J =* 8.2 Hz, 2H), 7.14 (t, *J =* 2.7 Hz, 1H), 6.60 (s, 1H), 6.12 - 6.04 (m, 1H), 4.43 (td, *J =* 10.4, 4.1 Hz, 1H), 3.57 (s, 1H), 3.57 (s, 3H), 3.45 (q, *J* = 7.0 Hz, 2H), 3.22 - 3.13 (m, 1H), 2.35 (s, 3H), 1.96 (d, *J =* 13.6 Hz, 1H), 1.77 (dt, *J =* 21.9, 14.0 Hz, 3H), 1.63 - 1.51 (m, 1H), 1.43 - 1.31 (m, 1H), 1.18 (t, *J =* 7.0 Hz, 3H).

LCMS m/z = 424.2[M+1]⁺.

### Example 20b: 4-((1R,2R,5S)-5-ethoxy-2-((5-methoxy-7-methyl-1H-indol-4-yl)oxy)cyclohexyl)benzoic acid and 4-((1S,2R,5R)-5-ethoxy-2-((5-methoxy-7-methyl-1H-indol-4-yl)oxy)cyclohexyl)benzoic acid and 4-((1R,2S,5S)-5-ethoxy-2-((5-methoxy-7-methyl-1H-indol-4-yl)oxy)cyclohexyl)benzoic acid and 4-((1S,2S,5R)-5-ethoxy-2-((5-methoxy-7-methyl-1H-indol-4-yl)oxy)cyclohexyl)benzoic acid

The mixture of isomers was obtained following the method in Example 20a.

The mixture of isomers was resolved by chiral SFC, obtaining 20b-1, tr = 0.89 min and 20b-2, tr = 1.22 min, 20b-3, tr = 0.92 min and peak 20b-4, tr = 1.46 min (The resolution method: chromatographic column: DAICELCHIRALCEL^{®}AD 250*20 mm, 10 µm; mobile phase A: supercritical CO₂; mobile phase B: methanol (+0.1% 7.0 mol/L ammonia in methanol); A:B: 75: 25; flow rate: 120 mL/min; detection wavelength: 214 nm)

20b-1, tᵣ = 0.89 min

^{I}HNMR (400 MHz, DMSO-*d*₆) δ 10.80 (s, 1H), 7.89 (d, *J =* 8.2 Hz, 2H), 7.54 (d, *J =* 8.2 Hz, 2H), 7.05 (t, *J =* 2.8 Hz, 1H), 6.60 (s, 1H), 5.59 (d, *J =* 2.1 Hz, 1H), 4.64 (s, 1H), 3.84 (s, 1H), 3.55 (s, 3H), 3.45 (dd, *J =* 14.0, 7.0 Hz, 3H), 2.33 (s, 3H), 2.14 (s, 1H), 1.88 (d, *J* = 13.1 Hz, 1H), 1.65 (d, *J =* 10.6 Hz, 2H), 1.55 (s, 1H), 1.24 (s, 1H), 1.14 (t, *J =* 7.0 Hz, 3H).

LCMS m/z = 424.1[M+1]⁺.

20b-2, tᵣ = 1.22 min

^{I}HNMR (400 MHz, DMSO-*d*₆) δ 10.80 (s, 1H), 7.91 (d, *J =* 8.2 Hz, 2H), 7.58 (d, *J =* 8.3 Hz, 2H), 7.06 (t, *J =* 2.8 Hz, 1H), 6.61 (s, 1H), 5.61 - 5.59 (m, 1H), 4.65 (s, 1H), 3.85 (s, 1H), 3.55 (s, 3H), 3.46 (dd, *J =* 14.0, 7.0 Hz, 3H), 2.34 (s, 3H), 2.15 (s, 1H), 1.89 (d, *J =* 11.7 Hz, 1H), 1.66 (d, *J =* 11.3 Hz, 2H), 1.56 (s, 1H), 1.24 (s, 1H), 1.15 (t, *J =* 7.0 Hz, 3H).

20b-3, tᵣ = 0.92 min

^{I}HNMR (400 MHz, DMSO-*d*₆) δ 12.69 (s, 1H), 10.81 (s, 1H), 7.90 (d, *J =* 8.3 Hz, 2H), 7.56 (d, *J =* 8.3 Hz, 2H), 7.07 (t, *J* = 2.7 Hz, 1H), 6.60 (s, 1H), 5.62 (dd, *J* = 2.9, 2.0 Hz, 1H), 4.58 (s, 1H), 3.56 (dd, *J* = 7.0, 2.3 Hz, 1H), 3.53 (s, 3H), 3.50 (dd, *J =* 7.0, 2.3 Hz, 1H), 3.47 - 3.41 (m, 1H), 3.06 (d, *J =* 13.2 Hz, 1H), 2.33 (s, 3H), 2.21 (dd, *J =* 24.3, 11.6 Hz, 1H), 2.01 (d, *J=* 11.6 Hz, 1H), 1.94 - 1.83 (m, 1H), 1.77 (d, *J =* 13.9 Hz, 2H), 1.43 (t, *J =* 13.2 Hz, 1H), 1.14 (t, *J =* 7.0 Hz, 3H).

LCMS m/z = 424.1[M+1]⁺.

20b-4, tr = 1.46 min

^{I}HNMR (400 MHz, DMSO-*d*₆) δ 10.77 (s, 1H), 7.87 (d, *J =* 8.3 Hz, 2H), 7.52 (d, *J =* 8.3 Hz, 2H), 7.03 (t, *J =* 2.7 Hz, 1H), 6.56 (s, 1H), 5.59 (dd, *J =* 2.8, 2.0 Hz, 1H), 4.55 (s, 1H), 3.53 (dd, *J =* 7.0, 2.3 Hz, 1H), 3.50 (s, 3H), 3.47 (dd, *J =* 7.0, 2.3 Hz, 1H), 3.44 - 3.37 (m, 1H), 3.03 (d, *J =* 13.0 Hz, 1H), 2.30 (s, 3H), 2.18 (dd, *J =* 24.2, 11.8 Hz, 1H), 1.98 (d, *J =* 11.6 Hz, 1H), 1.84 (dd, *J =* 19.4, 8.1 Hz, 1H), 1.72 (t, *J =* 13.6 Hz, 2H), 1.39 (t, *J =* 13.0 Hz, 1H), 1.10 (t, *J =* 7.0 Hz, 3H).

LCMS m/z = 424.1[M+1]⁺.

### Example 21: 4-((2S,4S)-4-ethoxy-1-((5-methoxy-7-methyl-1H-indol-4-yl)sulfonyl)piperidin-2-yl)benzoic acid and 4-((2R,4R)-4-ethoxy-1-((5-methoxy-7-methyl-1H-indol-4-yl)sulfonyl)piperidin-2-yl)benzoic acid

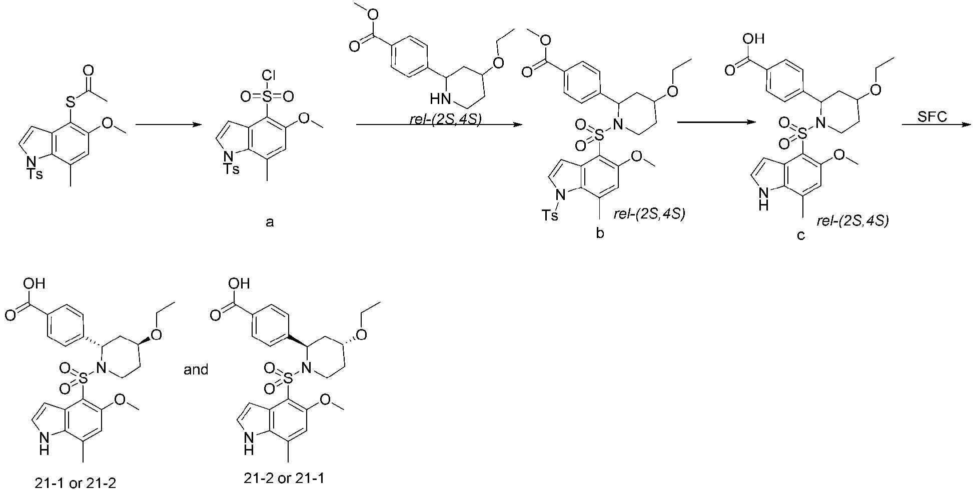

### a) Preparation of 5-methoxy-7-methyl-1-tolyl-1H-indole-4-sulfonyl chloride

S-(5-Methoxy-7-methyl-1-tolyl-1H-indol-4-yl) ethanethioate (100 mg) and acetonitrile/water (2.5 mL/0.5 mL) were added to a reaction flask. Under an ice-water bath, N-chlorosuccinimide (103.0 mg) was added, and 2 M dilute hydrochloric acid (5 drops) was slowly added, and stirred for 3.5 hours. The reaction was diluted with water (20 mL) and extracted with ethyl acetate (100 mL). The organic phase was dried over anhydrous sodium sulfate and filtered. The filtrate was concentrated under reduced pressure to yield a powdery residue, which was purified by column chromatography (mobile phase: petroleum ether/ethyl acetate = 4/1 (V/V)) to obtain 88 mg of the title compound.

### b) Preparation of (±)-rel-(2S,4S)-methyl 4-(4-ethoxy-1-((5-methoxy-7-methyl-1-tolyl-1H-indol-4-yl)sulfonyl)piperidin -2-yl)benzoate

(±)-Rel-(2S,4S)-methyl 4-(4-ethoxypiperidin-2-yl)benzoate (69.5 mg), triethylamine (80.1 mg), dichloromethane (5 mL), and 5-methoxy-7-methyl-1-tolyl-1H-indole-4-sulfonyl chloride (164 mg) were added to a reaction flask and stirred for 16 hours. The reaction was diluted with water (40 mL) and extracted with dichloromethane (100 mL). The organic phase was dried over anhydrous sodium sulfate and filtered. The filtrate was concentrated under reduced pressure to yield a powdery residue, which was purified by column chromatography (mobile phase: petroleum ether/ethyl acetate = 2/1 (V/V)) to obtain 179 mg of the title compound.

### c) Preparation of 4-((2S,4S)-4-ethoxy-1-((5-methoxy-7-methyl-1H-indol-4-yl)sulfonyl)piperidin-2-yl)benzoic acid and 4-((2R,4R)-4-ethoxy-1-((5-methoxy-7-methyl-1H-indol-4-yl)sulfonyl)piperidin-2-yl)benzoic acid

The enantiomeric isomers were obtained by hydrolysis following the hydrolysis method in Example 17.

LCMS m/z = 473.1[M+1]⁺.

The enantiomeric isomers were resolved by chiral SFC, obtaining 21-1, tᵣ = 1.34 min and 21-2, tᵣ = 2.68 min (The resolution method: chromatographic column: DAICELCHIRALCELOAD 250*20 mm, 10 µm; mobile phase A: supercritical CO₂; mobile phase B: methanol (+0.1% 7.0 mol/L ammonia in methanol); A:B: 55:45; flow rate:70 mL/min; detection wavelength: 214 nm)

21-1, tr = 1.34 min

^{I}HNMR (400 MHz, DMSO-*d*₆) δ 11.39 (s, 1H), 7.92 (d, J = 8.4 Hz, 2H), 7.55 - 7.42 (m, 3H), 6.94 (s, 1H), 6.91 (dd, J = 3.0, 2.0 Hz, 1H), 5.39 (s, 1H), 3.90 (s, 3H), 3.84- 3.81 (m, 1H), 3.30 (s, 2H), 3.06 (s, 1H), 2.95- 2.89 (m, 1H), 2.55 (s, 3H), 2.05 - 1.93 (m, 1H), 1.68 (d, J = 9.8 Hz, 1H), 1.47- 1.42 (m, 2H), 0.99-0.96 (m, 3H).

21-2, tr = 2.68 min

^{I}HNMR (400 MHz, DMSO-*d*₆) δ 11.39 (s, 1H), 7.92 (d, J = 8.4 Hz, 2H), 7.54 - 7.42 (m, 3H), 6.94 (s, 1H), 6.93 - 6.88 (m, 1H), 5.39 (s, 1H), 3.90 (s, 3H), 3.83 (d, J = 12.4 Hz, 1H), 3.30 (s, 2H), 3.06 (s, 1H), 2.95- 2.89 (m, 1H), 2.55 (s, 3H), 2.02 - 1.97 (m, 1H), 1.68 (d, J = 11.0 Hz, 1H), 1.51 - 1.38 (m, 2H), 0.99- 0.96 (m, 3H).

### Example 22:

### (±)(Z)-1¹-((5-methoxy-7-methyl-1H-indol-4-yl)methyl)-3-oxa-1(2,4)-piperazina-2(1,2)-benzenacyclodecaphan-8-ene-2 ⁴-carboxylic acid

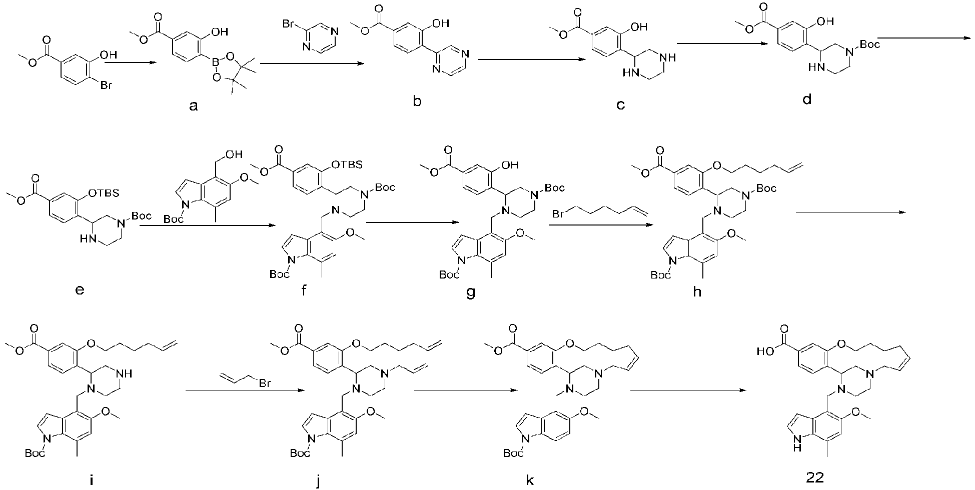

### a) Preparation of methyl 3-hydroxy-4-(4,4,5,5-tetramethyl-1,3,2-dioxaborolan-2-yl)benzoate

Methyl 4-bromo-3-hydroxybenzoate (10 g), bis(pinacolato)diboron (12.09 g), and potassium acetate (10.62 g) were added to a two-necked flask (500 mL), then 1,4-dioxane (150 mL) was added. The mixture was purged with nitrogen for protection. 1,1'-Bis(diphenylphosphino)ferrocene palladium(II) dichloride dichloromethane complex (3.53 g) was added, and the mixture was stirred at 90 °C for 4 hours. The reaction solution was cooled to room temperature, quenched by adding water. The mixture was extracted three times with ethyl acetate (100 mL*3). The organic phases were combined, washed three times with saturated brine, dried over anhydrous sodium sulfate, and filtered. The filtrate was concentrated under reduced pressure to yield a powdery residue, which was purified by column chromatography (mobile phase: petroleum ether/ethyl acetate = 10/1 (V/V)) to obtain 7.7 g of the title compound.

LCMS m/z = 279.1 [M+H]+

### b) Preparation of methyl 3-hydroxy-4-(pyrazin-2-yl)benzoate

Methyl 3-hydroxy-4-(4,4,5,5-tetramethyl-1,3,2-dioxaborolan-2-yl)benzoate (7.5 g), 2-bromopyrazine (6.43 g), and sodium carbonate (8.57 g) were added to a two-necked flask (500mL). A mixed solvent of 1,4-dioxane/water (150 mL/30 mL) was added. The mixture was purged with nitrogen for protection. Tetrakis(triphenylphosphine)palladium (2.49 g) was added, and the mixture was stirred at 100 °C for 4 hours. The reaction was quenched by adding water and extracted three times with ethyl acetate (100 mL*3). The organic phases were combined, washed three times with saturated brine, dried over anhydrous sodium sulfate, and filtered. The filtrate was concentrated under reduced pressure to yield a powdery residue, which was purified by column chromatography (mobile phase: petroleum ether/ethyl acetate = 10/1 (V/V)) to obtain 3.7 g of the title compound.

LCMS m/z = 231.2[M+H]+.

### c) Preparation of (±)methyl 3-hydroxy-4-(piperazin-2-yl)benzoate

Methyl 3-hydroxy-4-(pyrazin-2-yl)benzoate (3.7 g) was dissolved in methanol (200 mL). Palladium acetate (361 mg), acetic acid (19.3 g), and 10% palladium on carbon (1.3 g) were added. The system was purged with hydrogen, then stirred at room temperature overnight. After filtration, the filtrate was concentrated under reduced pressure to obtain 3 g of the title compound.

LCMS m/z = 237.1[M+H]+.

### d) Preparation of (±)tert-butyl 3-(2-hydroxy-4-(methoxycarbonyl)phenyl)piperazine-1-carboxylate

(±)Methyl 3-hydroxy-4-(piperazin-2-yl)benzoate (2.8 g) was dissolved in dichloromethane (100 mL). Triethylamine (1.8 g) was added. The mixture was purged with nitrogen for protection. A solution of di-tert-butyl dicarbonate (1.55 g) in dichloromethane (10 mL) was added dropwise. The mixture was stirred at room temperature for 1 hour. The reaction solution was concentrated under reduced pressure to yield a powdery residue, which was purified by column chromatography (mobile phase: dichloromethane/methanol = 20/1 (V/V)) to obtain 2.5 g of the title compound.

LCMS m/z = 337.4[M+H]+.

### e) (±)tert-butyl 3-(2-((tert-butyldimethylsilyl)oxy)-4-(methoxycarbonyl)phenyl)piperazine-1-carboxylate

(±)-Tert-butyl 3-(2-hydroxy-4-(methoxycarbonyl)phenyl)piperazine-1-carboxylate (2.3 g) was dissolved in N,N-dimethylformamide (50 mL). Imidazole (931 mg) was added. The mixture was purged with nitrogen for protection. The mixture was cooled to 0 °C in an ice-water bath, then tert-butyldimethylsilyl chloride (2.06 g) and 4-dimethylaminopyridine (84 mg) were added. The mixture was stirred at room temperature overnight. The reaction was quenched by adding water and extracted three times with ethyl acetate (50 mL*3). The organic phases were combined, washed three times with saturated brine, dried over anhydrous sodium sulfate, and filtered. The filtrate was concentrated under reduced pressure to yield a powdery residue, which was purified by column chromatography (mobile phase: petroleum ether/ethyl acetate = 20/1 (V/V)) to obtain 2.2 g of the title compound.

LCMS m/z = 451.7[M+H]+.

### f) Preparation of (±)tert-butyl 4-((4-(tert-butoxycarbonyl)-2-(2-((tert-butyldimethylsilyl)oxy)-4-(methoxycarbonyl) phenyl)piperazin-1-yl)methyl)-5-methoxy-7-methyl-1H-indole-1-carboxylate

Tert-butyl 4-(hydroxymethyl)-5-methoxy-7-methyl-1H-indole-1-carboxylate (1.32 g) was dissolved in dichloromethane (30 mL). The mixture was purged with nitrogen for protection. The mixture was cooled to 0 °C in an ice-water bath. Triphenylphosphine dibromide (2.07 g) was added in batches, and the mixture was stirred at 0 °C for 1.5 hours. Then (±)tert-butyl 3-(2-((tert-butyldimethylsilyl)oxy)-4-(methoxycarbonyl)phenyl)piperazine-1-carboxylate (1.7 g) and N,N-diisopropylethylamine (1.46 g) were added. The mixture was stirred at 0 °C for 1.5 hours. The reaction was quenched by adding water and extracted three times with dichloromethane (30 mL*3). The organic phases were combined, washed three times with saturated brine, dried over anhydrous sodium sulfate, and filtered. The filtrate was concentrated under reduced pressure to yield a powdery residue, which was purified by column chromatography (mobile phase: petroleum ether/ethyl acetate = 10/1 (V/V)) to obtain 2.5 g of the title compound.

LCMS m/z = 725.0[M+H]+.

### g) Preparation of (±)tert-butyl 4-((4-(tert-butoxycarbonyl)-2-(2-hydroxy-4-(methoxycarbonyl)phenyl)piperazin-1-yl) methyl)-5-methoxy-7-methyl-1H-indole-1-carboxylate

(±)-Tert-butyl 4-((4-(tert-butoxycarbonyl)-2-(2-((tert-butyldimethylsilyl)oxy)-4-(methoxycarbonyl)phenyl)piperazin-1-yl)methyl)-5-methoxy-7-methyl-1H-indole-1-carboxylate (2.4 g) was dissolved in tetrahydrofuran (50 mL). The mixture was purged with nitrogen for protection. Then a solution of tetrabutylammonium fluoride in tetrahydrofuran (5.6 mL, 1 mol/L) was added. The mixture was stirred at room temperature for 1 hour. The reaction was quenched by adding water and extracted three times with ethyl acetate (30 mL*3). The organic phases were combined, washed three times with saturated brine, dried over anhydrous sodium sulfate, and filtered. The filtrate was concentrated under reduced pressure to yield a powdery residue, which was purified by column chromatography (mobile phase: petroleum ether/ethyl acetate = 10/1 (V/V)) to obtain 2 g of the title compound.

LCMS m/z = 610.7[M+H]+.

### h) Preparation of (±)tert-butyl 4-((4-(tert-butoxycarbonyl)-2-(2-(hex-5-en-1-yloxy)-4-(methoxycarbonyl)phenyl) piperazin-1-yl)methyl)-5-methoxy-7-methyl-1H-indole-1-carboxylate

(±)-Tert-butyl 4-((4-(tert-butoxycarbonyl)-2-(2-hydroxy-4-(methoxycarbonyl)phenyl)piperazin-1-yl)methyl)-5-methoxy-7-methyl-1H-indole-1-carboxylate (2 g) was dissolved in N,N-dimethylformamide (30 mL). Potassium carbonate (1.36 g) was added. The mixture was purged with nitrogen for protection. Then 6-bromohex-1-ene (1.07 g) was added. The mixture was stirred at room temperature overnight. The reaction was quenched by adding water and extracted three times with ethyl acetate (30 mL*3). The organic phases were combined, washed three times with saturated brine, dried over anhydrous sodium sulfate, and filtered. The filtrate was concentrated under reduced pressure to yield a powdery residue, which was purified by column chromatography (mobile phase: petroleum ether/ethyl acetate = 10/1 (V/V)) to obtain 2 g of the title compound.

LCMS m/z = 692.9[M+H]+.

### i) Preparation of (±)tert-butyl 4-((2-(2-(hex-5-en-1-yloxy)-4-(methoxycarbonyl)phenyl)piperazin-1-yl)methyl)-5-methoxy-7-methyl-1H-indole-1-carboxylate

(±)-Tert-butyl 4-((4-(tert-butoxycarbonyl)-2-(2-(hex-5-en-1-yloxy)-4-(methoxycarbonyl)phenyl)piperazin-1-yl)methyl) -5-methoxy-7-methyl-1H-indole-1-carboxylate (2 g) was dissolved in methanol (50 mL). A hydrochloric acid solution in 1,4-dioxane (11 mL, 4 M) was added. The mixture was stirred at room temperature for 2 hours. The reaction solution was concentrated under reduced pressure to obtain 1.8 g of the title compound.

LCMS m/z = 592.7[M+H]+.

### j) Preparation of (±)tert-butyl 4-((4-allyl-2-(2-(hex-5-en-1-yloxy)-4-(methoxycarbonyl)phenyl)piperazin-1-yl)methyl)-5-methoxy-7-methyl-1H-indole-1-carboxylate

(±)-Tert-butyl 4-((2-(2-(hex-5-en-1-yloxy)-4-(methoxycarbonyl)phenyl)piperazin-1-yl)methyl)-5-methoxy-7-methyl-1H-indole-1-carboxylate (1.6 g, 2.7 mmol) and 3-bromoprop-1-ene (0.98 g) were dissolved in acetonitrile (40 mL). Triethylamine (1.64 g) was added. The mixture was purged with nitrogen for protection. The mixture was stirred at room temperature overnight. The reaction was quenched by adding water and extracted three times with ethyl acetate (30 mL × 3). The organic phases were combined, washed three times with saturated brine, dried over anhydrous sodium sulfate, and filtered. The filtrate was concentrated under reduced pressure to yield a powdery residue, which was purified by column chromatography (mobile phase: dichloromethane/methanol = 50/1 (V/V)) to obtain 1.2 g of the title compound.

LCMS m/z = 632.8[M+H]+.

### k) Preparation of (±)methyl (Z)-1¹-((1-(tert-butoxycarbonyl)-5-methoxy-7-methyl-1H-indol-4-yl)methyl)-3-oxa-1(2,4)-piperazina-2(1,2)-benzenacyclodecaphan-8-ene-2⁴-carboxylate

(±)-Tert-butyl 4-((4-allyl-2-(2-(hex-5-en-1-yloxy)-4-(methoxycarbonyl)phenyl)piperazin-1-yl)methyl)-5-methoxy-7-methyl-1H-indole-1-carboxylate (200 mg) was dissolved in toluene (200 mL). Grubbs 1st (130 mg) was added. The mixture was purged with nitrogen for protection. The mixture was stirred at 80 °C for 18 hours. The reaction solution was concentrated under reduced pressure to yield a powdery residue, which was purified by column chromatography (mobile phase: dichloromethane/methanol = 10/1 (V/V)) to obtain 175 mg of the title compound.

### l) Preparation of (±)(Z)-1¹-((5-methoxy-7-methyl-1H-indol-4-yl)methyl)-3-oxa-1(2,4)-piperazina-2(1,2)-benzenacyclodecaphan-8-ene-2⁴-carboxylic acid

(±)methyl (Z)-1¹-((1-(tert-butoxycarbonyl)-5-methoxy-7-methyl-1H-indol-4-yl)methyl)-3-oxa-1(2,4)-piperazina-2(1,2) -benzenacyclodecaphan-8-ene-2⁴-carboxylate (175 mg) was dissolved in a mixture of methanol/tetrahydrofuran (10 mL/10 mL). An aqueous solution of lithium hydroxide (10 mL, 2 N) was added. The mixture was stirred at 70 °C for 2 hours. The reaction solution was concentrated to dryness under reduced pressure, diluted with water, and the pH was adjusted to 7 with dilute hydrochloric acid. The mixture was extracted three times with ethyl acetate. The organic phases were combined, dried over anhydrous sodium sulfate, and filtered. The filtrate was concentrated under reduced pressure to yield a powdery residue, which was purified by column chromatography (mobile phase: dichloromethane/methanol = 20/1 (V/V)) to obtain 5 mg of the title compound.

¹H NMR (400 MHz, DMSO-d6) δ 10.84 (s, 1H), 7.99 (d, J = 7.9 Hz, 1H), 7.62 (d, J = 8.0 Hz, 1H), 7.44 (s, 1H), 7.28 (s, 1H), 6.76 - 6.64 (m, 1H), 6.62 (s, 1H), 5.45 (t, J = 10.5 Hz, 1H), 5.29 (s, 1H), 4.25 (s, 1H), 4.16 (d, J = 9.3 Hz, 1H), 3.95 (t, J = 10.9 Hz, 2H), 3.77 (d, J = 11.9 Hz, 1H), 3.74 (s, 3H), 2.90 (d, J = 11.5 Hz, 2H), 2.75 (d, J = 11.3 Hz, 1H), 2.68 (d, J = 17.4 Hz, 2H), 2.43 (s, 3H), 2.33 (d, J = 11.7 Hz, 2H), 1.91 (s, 2H), 1.66 (s, 1H), 1.30 - 1.08 (m, 4H).

LCMS m/z = 490.6 [M+1]+.

### Example 23: (1³R,1⁴R,Z)-1⁴-((5-methoxy-7-methyl-1H-indol-4-yl)oxy)-3-oxa-1(3,1)-piperidina-2(1,2)-benzenacyclodecaphan-8-ene-2⁴-carboxylic acid

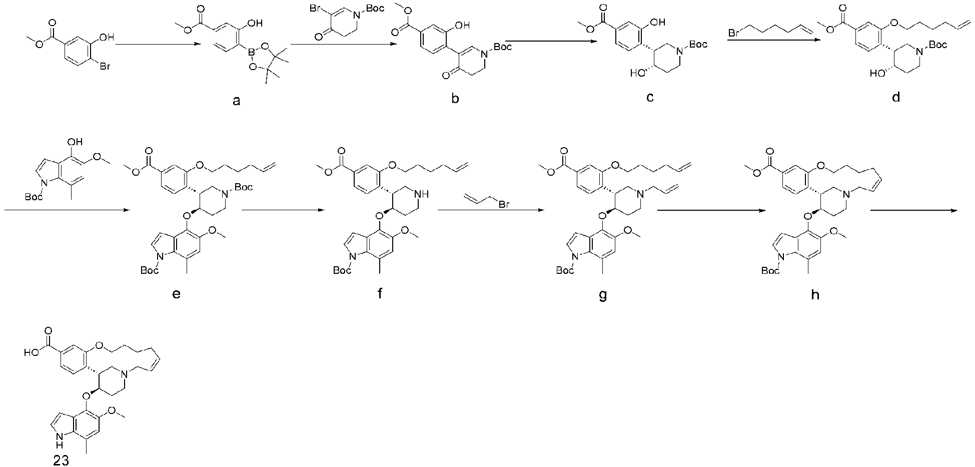

### a) Preparation of methyl 3-hydroxy-4-(4,4,5,5-tetramethyl-1,3,2-dioxaborolan-2-yl)benzoate

Methyl 4-bromo-3-hydroxybenzoate (25 g), bis(pinacolato)diboron (30.23 g), and potassium acetate (26.55 g) were added to a 500 mL three-necked flask. Then 1,4-dioxane (350 mL) was added. The mixture was purged with nitrogen for protection. [1,1'-Bis(diphenylphosphino)ferrocene]palladium(II) dichloride dichloromethane complex (4.42 g) was added. The mixture was heated to 90 °C and stirred for 4 hours. The reaction was quenched by adding water (300 mL) and extracted three times with ethyl acetate (200 mL*3). The organic phases were combined, washed three times with saturated brine, dried over anhydrous sodium sulfate, and filtered. The filtrate was concentrated under reduced pressure to yield a powdery residue, which was purified by column chromatography (mobile phase: petroleum ether/ethyl acetate = 10/1 (V/V)) to obtain 30 g of the title compound.

LCMS m/z = 279.1 [M+H]+.

### b) Preparation of tert-butyl 5-(2-hydroxy-4-(methoxycarbonyl)phenyl)-4-oxo-3,4-dihydropyridine-1(2H)-carboxylate

Methyl 3-hydroxy-4-(4,4,5,5-tetramethyl-1,3,2-dioxaborolan-2-yl)benzoate (19 g), tert-butyl 5-bromo-4-oxo-3,4-dihydropyridine-1(2H)-carboxylate (12.58 g), and potassium carbonate (18.88 g) were added to a three-necked flask. Then 1,4-dioxane/water (300 mL/100 mL) was added. The mixture was purged with nitrogen for protection. Tetrakis(triphenylphosphine)palladium (4.42 g) was added. The mixture was heated to 60 °C and stirred for 4 hours. The reaction was quenched by adding water (300 mL) and extracted three times with ethyl acetate (200 mL*3). The organic phases were combined, washed three times with saturated brine, dried over anhydrous sodium sulfate, and filtered. The filtrate was concentrated under reduced pressure to yield a powdery residue, which was purified by column chromatography (mobile phase: petroleum ether/ethyl acetate = 10/1 (V/V)) to obtain 7.5 g of the title compound.

LCMS m/z = 348.4 [M+H]+.

### c) Preparation of tert-butyl (3R,4S)-4-hydroxy-3-(2-hydroxy-4-(methoxycarbonyl)phenyl)piperidine-1-carboxylate

At 0 °C, anhydrous formic acid (30 mL) was added dropwise to triethylamine (43 mL) to prepare a solution (temperature controlled at 5-10 °C). Tert-butyl 5-(2-hydroxy-4-(methoxycarbonyl)phenyl)-4-oxo-3,4-dihydropyridine-1(2H) -carboxylate (7.5 g) and RuCl(p-cymene)[(S,S)-Ts-DPEN] (1.36 g) were added to a reaction flask. Then the prepared solution of anhydrous formic acid and triethylamine was added to the reaction flask. The system was purged with nitrogen. The temperature was slowly raised to room temperature, and the mixture was stirred overnight. The reaction system was cooled to 0-5 °C in an ice-water bath. Triethylamine (5 mL) was slowly added to adjust the pH to weakly basic, with the temperature controlled at around 10 °C. The reaction was quenched by adding water (200 mL). The mixture was extracted with ethyl acetate (150 mL × 3), washed with saturated brine (200 mL × 1). The organic phase was dried over anhydrous sodium sulfate, and filtered. The filtrate was concentrated under reduced pressure to yield a powdery residue, which was purified by column chromatography (mobile phase: petroleum ether/ethyl acetate = 20/3 (V/V)) to obtain 2 g of the title compound.

LCMS m/z = 352.4 [M+H]+.

### d) Preparation of tert-butyl (3R,4S)-3-(2-(hex-5-en-1-yloxy)-4-(methoxycarbonyl)phenyl)-4-hydroxypiperidine-1-carboxylate

Tert-butyl (3R,4S)-4-hydroxy-3-(2-hydroxy-4-(methoxycarbonyl)phenyl)piperidine-1-carboxylate (1.9 g), 6-bromohex -1-ene (1.59 g), and potassium carbonate (2.24 g) were added to a three-necked flask. Then N,N-dimethylformamide (40 mL) was added. The mixture was purged with nitrogen for protection. The mixture was stirred at room temperature overnight. The reaction was quenched by adding water (100 mL). The mixture was extracted with ethyl acetate (100 mL*3). The organic phases were combined, washed with saturated brine (100 mL × 3), dried over anhydrous sodium sulfate, and filtered. The filtrate was concentrated under reduced pressure to yield a powdery residue, which was purified by column chromatography (mobile phase: petroleum ether/ethyl acetate = 20/3 (V/V)) to obtain 1.55 g of the title compound.

LCMS m/z = 434.5[M+H]+.

### e) Preparation of tert-butyl 4-(((3R,4R)-1-(tert-butoxycarbonyl)-3-(2-(hex-5-en-1-yloxy)-4-(methoxycarbonyl)phenyl) piperidin-4-yl)oxy)-5-methoxy-7-methyl-1H-indole-1-carboxylate

Tert-butyl (3R,4S)-3-(2-(hex-5-en-1-yloxy)-4-(methoxycarbonyl)phenyl)-4-hydroxypiperidine-1-carboxylate (1.55 g, 3.58 mmol), tert-butyl 4-hydroxy-5-methoxy-7-methyl-1H-indole-1-carboxylate (1.5 g), and triphenylphosphine (1.13 g) were added to a three-necked flask. The mixture was purged with nitrogen for protection. Anhydrous tetrahydrofuran (40 mL) was added. Diisopropyl azodicarboxylate (868 mg) was added dropwise at 0 °C. The reaction system was slowly warmed to room temperature and stirred overnight. The reaction was quenched by adding water (100 mL). The mixture was extracted with ethyl acetate (100 mL*3). The organic phases were combined, washed with saturated brine (100 mL × 1), dried over anhydrous sodium sulfate, and filtered. The filtrate was concentrated under reduced pressure to yield a powdery residue, which was purified by column chromatography (mobile phase: petroleum ether/ethyl acetate = 20/1 (V/V)) to obtain 550 mg of the title compound.

LCMS m/z = 693.9[M+H]+.

### f) Preparation of tert-butyl 4-(((3R,4R)-3-(2-(hex-5-en-1-yloxy)-4-(methoxycarbonyl)phenyl)piperidin-4-yl)oxy)-5-methoxy-7-methyl-1H-indole-1-carboxylate

Tert-butyl 4-(((3R,4R)-1-(tert-butoxycarbonyl)-3-(2-(hex-5-en-1-yloxy)-4-(methoxycarbonyl)phenyl)piperidin-4-yl) oxy)-5-methoxy-7-methyl-1H-indole-1-carboxylate (530 mg) was added to 1,4-dioxane (3 mL). A hydrochloric acid solution in 1,4-dioxane (3 mL, 4 M) was added. Under nitrogen protection, the mixture was stirred to react at room temperature for 2 hours. Saturated sodium bicarbonate solution (30 mL) was added for neutralization. The mixture was extracted with ethyl acetate (50 mL*3). The organic phases were combined, washed with saturated brine (100 mL), dried over anhydrous sodium sulfate, and filtered. The filtrate was concentrated under reduced pressure to dryness to obtain 440 mg of the title compound.

LCMS m/z = 593.7[M+H]+

### g) Preparation of tert-butyl 4-(((3R,4R)-1-allyl-3-(2-(hex-5-en-1-yloxy)-4-(methoxycarbonyl)phenyl)piperidin-4-yl) oxy)-5-methoxy-7-methyl-1H-indole-1-carboxylate

Tert-butyl 4-(((3R,4R)-3-(2-(hex-5-en-1-yloxy)-4-(methoxycarbonyl)phenyl)piperidin-4-yl)oxy)-5-methoxy-7-methyl-1H-indole-1-carboxylate (430 mg) was dissolved in acetonitrile (10 mL). Then triethylamine (440 mg) and 3-bromoprop-1-ene (264 mg) were added. The mixture was stirred to react at 50 °C overnight. The reaction was quenched by adding water (50 mL). The mixture was extracted with ethyl acetate (50 mL*3). The organic phases were combined, washed with saturated brine (50 mL*1), dried over anhydrous sodium sulfate, and filtered. The filtrate was concentrated under reduced pressure to yield a powdery residue, which was purified by column chromatography (mobile phase: dichloromethane/methanol = 20/1 (V/V)) to obtain 350 mg of the title compound.

LCMS m/z = 633.8[M+H]+.

### h) Preparation of methyl (1³R,1⁴R,Z)-1⁴-((1-(tert-butoxycarbonyl)-5-methoxy-7-methyl-1H-indol-4-yl)oxy)-3-oxa-1(3,1)-piperidina-2(1,2)-benzenacyclodecaphan-8-ene-2⁴-carboxylate

Tert-butyl 4-(((3R,4R)-1-allyl-3-(2-(hex-5-en-1-yloxy)-4-(methoxycarbonyl)phenyl)piperidin-4-yl)oxy)-5-methoxy-7-methyl-1H-indole-1-carboxylate (350 mg) was added to dichloromethane (350 mL). Grubbs 1st (228 mg) was added. The mixture was purged with nitrogen for protection and stirred at 40 °C for 18 hours. The reaction solution was concentrated under reduced pressure to yield a powdery residue, which was purified by column chromatography (mobile phase: dichloromethane/methanol = 20/1 (V/V)) to obtain 130 mg of the title compound.

LCMS m/z = 605.7[M+H]+.

### i) Preparation of (1³R,1⁴R,Z)-1⁴-((5-methoxy-7-methyl-1H-indol-4-yl)oxy)-3-oxa-1(3,1)-piperidina-2(1,2)-benzenacyclodecaphan-8-ene-2⁴-carboxylic acid

Methyl (1³R,1⁴R,Z)-1⁴-((1-(tert-butoxycarbonyl)-5-methoxy-7-methyl-1H-indol-4-yl)oxy)-3-oxa-1(3,1)-piperidina-2(1,2)-benzenacyclodecaphan-8-ene-2⁴-carboxylate (35 mg) was dissolved in a mixed solution of tetrahydrofuran/methanol (3 mL/3 mL). An aqueous solution of lithium hydroxide (3 mL, 2 N) was added. The mixture was stirred at 70 °C for 2 hours, then concentrated to dryness under reduced pressure. Dilute hydrochloric acid aqueous solution was added to adjust the pH to neutral. The mixture was extracted with ethyl acetate (10 mL*3). The organic phases were combined, washed with saturated brine (20 mL*1), dried over anhydrous sodium sulfate, and filtered. The filtrate was concentrated under reduced pressure to yield a powdery residue, which was purified by column chromatography (mobile phase: dichloromethane/methanol = 20/1 (V/V)) to obtain 6.1 mg of the title compound. ¹H NMR (400 MHz, DMSO-d6) δ 10.87 (d, *J* = 9.4 Hz, 1H), 7.90 (dd, *J =* 30.1, 7.7 Hz, 1H), 7.57 (t, *J =* 7.3 Hz, 1H), 7.40 (dd, *J =* 20.6, 12.0 Hz, 2H), 7.24 - 7.17 (m, 1H), 6.71 (d, *J =* 16.0 Hz, 1H), 5.43 (s, 1H), 4.93 (d, *J =* 34.3 Hz, 1H), 4.09 (s, 1H), 3.79 (t, *J =* 15.9 Hz, 3H), 2.39 (d, *J* = 5.7 Hz, 3H), 2.23 - 2.15 (m, 1H), 2.03 - 1.96 (m, 2H), 1.30 (s, 3H), 1.26 (s, 5H), 1.23 (s, 6H). LCMS m/z = 491.6[M+H]+.

### Example 24:

### (1³R,1⁴R)-1⁴-((5-methoxy-7-methyl-1H-indol-4-yl)oxy)-3-oxa-1(3,1)-piperidina-2(1,2)-benzenacyclodecaphane-2⁴-car boxylic acid

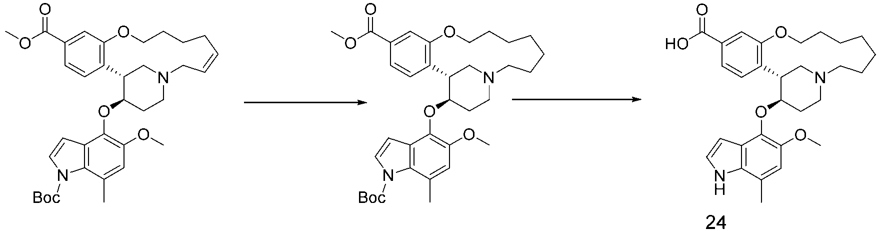

### a) Preparation of methyl (1³R,1⁴R)-1⁴-((1-(tert-butoxycarbonyl)-5-methoxy-7-methyl-1H-indol-4-yl)oxy)-3-oxa-1(3,1)-piperidina-2(1,2)-benzenacyclodecaphane-2⁴-carboxylate

Methyl (1³R,1⁴R,Z)-1⁴-((1-(tert-butoxycarbonyl)-5-methoxy-7-methyl-1H-indol-4-yl)oxy)-3-oxa-1(3,1)-piperidina-2(1,2)-benzenacyclodecaphan-8-ene-2⁴-carboxylate (60 mg) was dissolved in methanol (5 mL) and added to a three-necked flask. Palladium on carbon (35 mg, 10%) was added. The mixture was purged with hydrogen, then stirred overnight at room temperature. The mixture was filtered through diatomaceous earth, and the filtrate was concentrated to dryness by distillation under reduced pressure to obtain 45 mg of the title compound.

LCMS m/z = 607.4 [M+H]+.

### b) Preparation of (1³R,1⁴R)-1⁴-((5-methoxy-7-methyl-1H-indol-4-yl)oxy)-3-oxa-1(3,1)-piperidina-2(1,2)-benzenacyclodecaphane-2⁴-carboxylic acid

Methyl (1³R,1⁴R)-1⁴-((1-(tert-butoxycarbonyl)-5-methoxy-7-methyl-1H-indol-4-yl)oxy)-3-oxa-1(3,1)-piperidina-2(1,2) -benzenacyclodecaphane-2⁴-carboxylate (50 mg) was dissolved in a mixed solution of tetrahydrofuran/methanol (3 mL/3 mL). An aqueous solution of lithium hydroxide (3 mL, 2 N) was added. The mixture was stirred at 70 °C for 2 hours and then concentrated to dryness under reduced pressure. Dilute hydrochloric acid aqueous solution was added to adjust the pH to neutral. The mixture was extracted with ethyl acetate (10 mL*3). The organic phases were combined, washed with saturated brine (20 mL*1), dried over anhydrous sodium sulfate, and filtered. The filtrate was concentrated under reduced pressure to yield a powdery residue, which was purified by column chromatography (mobile phase: dichloromethane/methanol = 20/1 (V/V)) to obtain 5.1 mg of the title compound.

¹H NMR (400 MHz, DMSO-d6) δ 10.86 (s, 1H), 7.85 (d, J = 7.5 Hz, 1H), 7.54 (d, J = 7.9 Hz, 1H), 7.45 (s, 1H), 7.20 (s, 1H), 6.74 (s, 1H), 6.38 (s, 1H), 4.84 (d, J = 7.9 Hz, 1H), 4.21 (s, 1H), 3.83 (s, 3H), 3.25 (d, J = 10.5 Hz, 3H), 2.71 (d, J = 9.7 Hz, 2H), 2.40 (s, 3H), 2.10 (d, J = 11.2 Hz, 1H), 1.98 (s, 2H), 1.83 (s, 1H), 1.70 (s, 3H), 1.55 (s, 2H), 1.40 (s, 2H), 1.30 (dd, J = 26.9, 17.4 Hz, 4H).

LCMS m/z = 493.3[M+H]+.

### Example 25:

### 4-((3S,4S)-4-((5-(difluoromethoxy)-7-methyl-1H-indol-4-yl)oxy)-1-(3,3,3-trifluoropropyl)piperidin-3-yl)benzoic acid and 4-((3R,4R)-4-((5-(difluoromethoxy)-7-methyl-1H-indol-4-yl)oxy)-1-(3,3,3-trifluoropropyl)piperidin-3-yl)benzoic acid

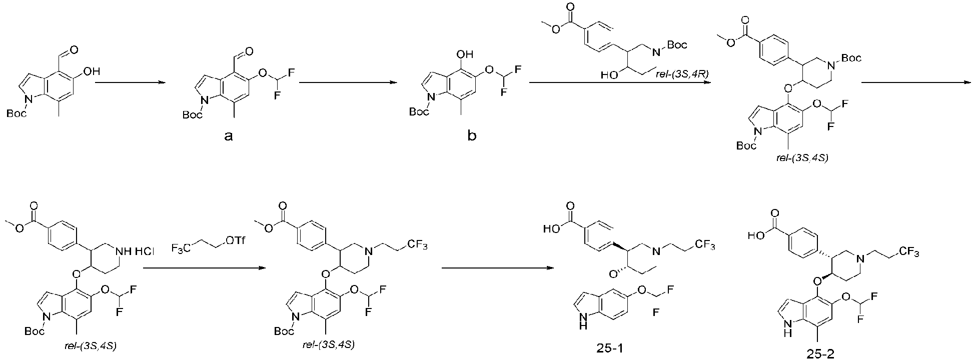

### a) Preparation of tert-butyl 5-(difluoromethoxy)-4-formyl-7-methyl-1H-indole-1-carboxylate

Tert-butyl 4-formyl-5-hydroxy-7-methyl-1H-indole-1-carboxylate (1.5 g) was dissolved in dichloromethane (25 mL) and added to a two-necked flask. The mixture was purged with nitrogen for protection and cooled to 0 °C in an ice-water bath. An aqueous solution of potassium hydroxide (12.23 g) was added dropwise. After stirring for 5 minutes, a solution of (bromodifluoromethyl)trimethylsilane (3.32 g) in dichloromethane (5 mL) was added dropwise. The mixture was reacted at 0 °C for 2 hours. The reaction was quenched by adding water (50 mL). The mixture was extracted with dichloromethane (50 mL*3). The organic phases were combined, washed with saturated brine (50 mL*1), dried over anhydrous sodium sulfate, and filtered. The filtrate was concentrated under reduced pressure to yield a powdery residue, which was purified by column chromatography (mobile phase: petroleum ether/ethyl acetate = 10/1 (V/V)) to obtain 670 mg of the title compound.

LCMS m/z = 326[M+H]+.

### b) Preparation of tert-butyl 5-(difluoromethoxy)-4-hydroxy-7-methyl-1H-indole-1-carboxylate

Tert-butyl 5-(difluoromethoxy)-4-formyl-7-methyl-1H-indole-1-carboxylate (670 mg) was dissolved in dichloromethane (20 mL) and added to a 100-mL two-necked flask. The mixture was purged with nitrogen for protection. m-chloroperoxybenzoic acid (3.55 g) was added at 0 °C. The mixture was reacted at room temperature for 3 hours. The reaction was quenched by adding an aqueous solution of sodium bisulfite. The mixture was extracted with ethyl acetate (50 mL × 3). The organic phases were combined, washed with saturated brine (50 mL × 1), dried over anhydrous sodium sulfate, and filtered. The filtrate was concentrated under reduced pressure to yield a powdery residue, which was purified by column chromatography (mobile phase: petroleum ether/ethyl acetate = 4/1 (V/V)) to obtain 170 mg of the title compound.

LCMS m/z = 314.2[M+H]+.

### c) Preparation of (±)-rel-(3S,4S)-tert-butyl 4-((1-(tert-butoxycarbonyl)-3-(4-(methoxycarbonyl)phenyl)piperidin-4-yl) oxy)-5-(difluoromethoxy)-7-methyl-1H-indole-1-carboxylate

Tert-butyl 5-(difluoromethoxy)-4-hydroxy-7-methyl-1H-indole-1-carboxylate (120 mg), (±)-rel-(3S,4R)-tert-butyl 4-hydroxy-3-(4-(methoxycarbonyl)phenyl)piperidine-1-carboxylate (193 mg), and triphenylphosphine (201 mg) were added to a 25mL two-necked flask. The mixture was purged with nitrogen for protection. Anhydrous tetrahydrofuran (10 mL) was added. Diisopropyl azodicarboxylate (155 mg) was added dropwise at 0 °C. The temperature was slowly raised to room temperature, and the mixture was stirred overnight. The reaction was quenched by adding water (20 mL). The mixture was extracted with dichloromethane (20 mL*3). The organic phases were combined, washed with saturated brine (20 mL*1), dried over anhydrous sodium sulfate, and filtered. The filtrate was concentrated under reduced pressure to yield a powdery residue, which was purified by column chromatography (mobile phase: petroleum ether/ethyl acetate = 4/1 (V/V)) to obtain 137 mg of the title compound.

LCMS m/z = 631.3[M+H]+.

### d) Preparation of (±)-rel-(3S,4S)-tert-butyl 5-(difluoromethoxy)-4-((3-(4-(methoxycarbonyl)phenyl)piperidin-4-yl)oxy) -7-methyl-1H-indole-1-carboxylate

(±)-Rel-(3S,4S)-tert-butyl 4-((1-(tert-butoxycarbonyl)-3-(4-(methoxycarbonyl)phenyl)piperidin-4-yl)oxy)-5-(difluoromethoxy)-7-methyl-1H-indole-1-carboxylate (137 mg) was dissolved in methanol (15 mL). A solution of hydrogen chloride in 1,4-dioxane (10 mL, 4 M) was added. The mixture was purged with nitrogen for protection. The mixture was stirred at room temperature for 2 hours. The reaction solution was concentrated to dryness under reduced pressure to obtain 150 mg of the title compound.

LCMS m/z = 531[M+H]+.

### e) Preparation of (±)-rel-(3S,4S)-tert-butyl 5-(difluoromethoxy)-4-((3-(4-(methoxycarbonyl)phenyl)-1-(3,3,3-trifluoropropyl)piperidin-4-yl)oxy)-7-methyl-1H-indole-1-carboxylate

(±)-Rel-(3S,4S)-tert-butyl

5-(difluoromethoxy)-4-((3-(4-(methoxycarbonyl)phenyl)piperidin-4-yl)oxy)-7-methyl-1H-indole-1-carboxylate (150 mg) was dissolved in tetrahydrofuran (10 mL). 3,3,3-Trifluoropropyl trifluoromethanesulfonate (195.6 mg) was added, followed by the addition of N,N-diisopropylethylamine (137 mg). The mixture was stirred at 70 °C overnight. The reaction was quenched by adding water (20 mL). The mixture was extracted with dichloromethane (20 mL*3). The organic phases were combined, washed with saturated brine (20 mL*1), dried over anhydrous sodium sulfate, and filtered. The filtrate was concentrated under reduced pressure to yield a powdery residue, which was purified by column chromatography (mobile phase: petroleum ether/ethyl acetate = 4/1 (V/V)) to obtain 140 mg of the title compound.

LCMS m/z = 627[M+H]+.

### f) Preparation of 4-((3S,4S)-4-((5-(difluoromethoxy)-7-methyl-1H-indol-4-yl)oxy)-1-(3,3,3-trifluoropropyl) piperidin-3-yl)benzoic acid and 4-((3R,4R)-4-((5-(difluoromethoxy)-7-methyl-1H-indol-4-yl)oxy)-1-(3,3,3-trifluoropropyl) piperidin-3-yl)benzoic acid

(±)-Rel-(3S,4S)-tert-butyl 5-(difluoromethoxy)-4-((3-(4-(methoxycarbonyl)phenyl)-1-(3,3,3-trifluoropropyl)piperidin -4-yl)oxy)-7-methyl-1H-indole-1-carboxylate (140 mg) was dissolved in tetrahydrofuran/methanol (10 mL/10 mL). An aqueous solution of lithium hydroxide (5 mL, 2 N) was added. The mixture was stirred at 70 °C for 2 hours and then concentrated to dryness under reduced pressure. Dilute hydrochloric acid aqueous solution was added to adjust the pH to neutral. The mixture was extracted with ethyl acetate (10 mL*3). The organic phases were combined, washed with saturated brine (20 mL*1), dried over anhydrous sodium sulfate, and filtered. The filtrate was concentrated under reduced pressure to yield a powdery residue, which was purified by column chromatography (mobile phase: dichloromethane/methanol = 20/1 (V/V)) to obtain 130 mg of the enantiomer of the title compound.

¹H NMR (400 MHz, DMSO-d6) δ 11.16 (s, 1H), 7.89 (d, *J =* 8.2 Hz, 2H), 7.57 (d, *J =* 8.2 Hz, 2H), 7.28 (t, *J =* 2.7 Hz, 1H), 6.94 - 6.54 (m, 2H), 6.21 (s, 1H), 4.59 - 4.47 (m, 1H), 3.56 (t, *J =* 6.1 Hz, 1H), 3.19 - 3.07 (m, 1H), 2.91 (dd, *J =* 21.7, 10.3 Hz, 2H), 2.56 (d, *J =* 6.9 Hz, 2H), 2.36 (s, 3H), 2.12 (t, *J =* 10.6 Hz, 1H), 1.77 (d, *J =* 8.8 Hz, 1H), 1.69 (d, *J =* 9.1 Hz, 1H), 1.43 (dd, *J =* 7.8, 5.5 Hz, 2H).

LCMS m/z = 513.2[M+1]+.

The enantiomers of (±)-rel-(3S,4S)-4-(1-(2,2-difluoroethyl)-4-((5-methoxy-7-methyl-1H-indol-4-yl)oxy)piperidin-3-yl) benzoic acid were resolved by chiral SFC, obtaining 25-1, tr = 1.69 min and 25-2, tr = 2.40 min. (The resolution method: chromatographic column: DAICELCHIRALPAK^{®}IC 250*20 mm,10 µm; mobile phase A: supercritical CO₂; mobile phase B: methanol (+0.1% 7.0 mol/L ammonia in methanol); A:B: 75:25; flow rate: 100 mL/min; detection wavelength: 214 nm)

25-1, tr = 1.69 min

¹H NMR (400 MHz, DMSO-d6) δ 11.18 (s, 1H), 7.89 (d, *J =* 8.2 Hz, 2H), 7.57 (d, *J =* 8.2 Hz, 2H), 7.28 (t, *J =* 2.7 Hz, 1H), 6.72 (dd, *J =* 89.3, 62.3 Hz, 2H), 6.27 - 6.14 (m, 1H), 4.53 (td, *J =* 9.6, 4.3 Hz, 1H), 3.55 (dd, *J =* 17.3, 11.2 Hz, 1H), 3.12 (td, *J =* 10.0, 3.7 Hz, 1H), 2.91 (dd, *J =* 23.0, 11.6 Hz, 2H), 2.56 (dd, *J =* 15.0, 8.3 Hz, 2H), 2.42 - 2.31 (m, 3H), 2.11 (dd, *J =* 20.1, 8.8 Hz, 1H), 1.73 (q, *J =* 10.7 Hz, 2H), 1.51 (d, *J =* 2.7 Hz, 2H).

25-2, tr = 2.40 min.

¹H NMR (400 MHz, DMSO) δ 11.19 (s, 1H), 7.89 (d, *J* = 8.3 Hz, 2H), 7.57 (d, *J* = 8.3 Hz, 2H), 7.28 (t, *J =* 2.8 Hz, 1H), 6.72 (dd, *J =* 89.4, 62.1 Hz, 2H), 6.21 (dd, *J =* 3.0, 2.0 Hz, 1H), 4.53 (td, *J =* 9.8, 4.5 Hz, 1H), 3.56 (t, *J =* 6.1 Hz, 1H), 3.12 (td, *J =* 10.4, 3.9 Hz, 1H), 2.91 (dd, *J =* 23.0, 11.4 Hz, 2H), 2.64 - 2.53 (m, 2H), 2.41 - 2.33 (m, 3H), 2.11 (dd, *J =* 19.4, 9.1 Hz, 1H), 1.82 - 1.63 (m, 2H), 1.49 - 1.36 (m, 2H).

### Example 26: 4-((3R,4R)-1-(2,2-difluoroethyl)-4-((5-(methoxy-d3)-7-methyl-1H-indol-4-yl)oxy)piperidin-3-yl) benzoic acid

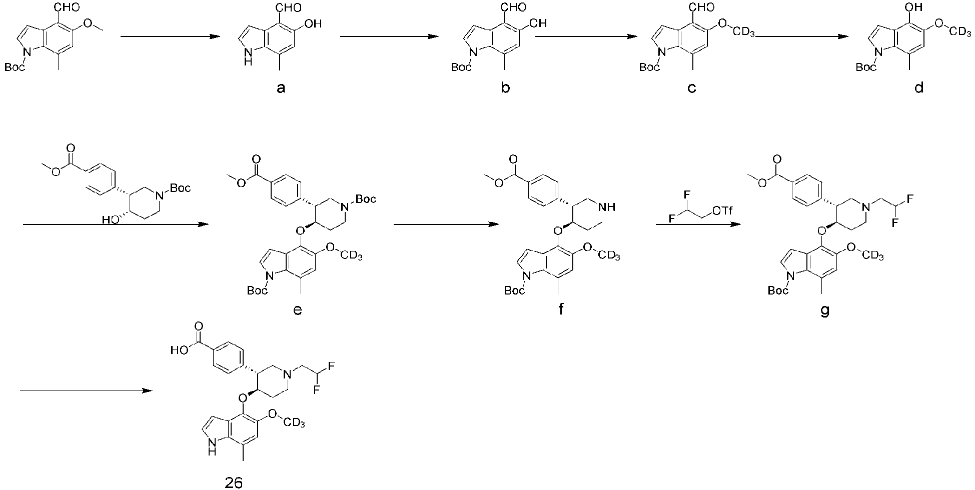

### a) Preparation of 5-hydroxy-7-methyl-1H-indole-4-carbaldehyde

Under an ice-water bath, tert-butyl 4-formyl-5-methoxy-7-methyl-1H-indole-1-carboxylate (5.0 g) was added to a 250 mL two-necked flask. Then dichloromethane (130 mL) was added. The mixture was purged with nitrogen for protection. Boron tribromide (21.66 g) was added dropwise. The mixture was slowly raised to room temperature, and reacted overnight. The reaction solution was poured into ice-water and then filtered by suction. The filter cake was washed with dichloromethane. The filtrate was washed with saturated sodium bicarbonate solution and saturated brine, dried over anhydrous sodium sulfate, and filtered. The filtrate was concentrated under reduced pressure to yield a powdery residue, which was purified by column chromatography (mobile phase: petroleum ether/dichloromethane = 3/2 (V/V)) to obtain 1.2 g of the title compound.

LCMS m/z = 176.1 [M+H]+.

### b) Preparation of tert-butyl 5-(difluoromethoxy)-4-hydroxy-7-methyl-1H-indole-1-carboxylate

5-Hydroxy-7-methyl-1H-indole-4-carbaldehyde (1.2 g) was added to dichloromethane (40 mL). Then di-tert-butyl dicarbonate (4.48 g) and 4-dimethylaminopyridine (334 mg) were added. The mixture was purged with nitrogen for protection and stirred at room temperature overnight. The reaction solution was concentrated to dryness under reduced pressure. Then methanol (30 mL) and potassium carbonate (10 g) were added, and the mixture was stirred at room temperature for 1 hour. The pH was adjusted to weakly acidic with dilute hydrochloric acid, and then water and ethyl acetate were added for extraction and separation. The organic phase was dried over anhydrous sodium sulfate and filtered. The filtrate was concentrated under reduced pressure to yield a powdery residue, which was purified by column chromatography (mobile phase: petroleum ether/ethyl acetate = 85/15 (V/V)) to obtain 1.56 g of the title compound.

LCMS m/z = 276.1 [M+H]+.

### c) Preparation of tert-butyl 4-formyl-5-(methoxy-d3)-7-methyl-1H-indole-1-carboxylate

Tert-butyl 5-(difluoromethoxy)-4-hydroxy-7-methyl-1H-indole-1-carboxylate (1.4 g), N,N-dimethylformamide (20 mL), deuterated methyl iodide (2.21 g), and potassium carbonate (2.11 g) were added to a reaction flask. The mixture was reacted at room temperature overnight. The reaction was quenched by adding water (30 mL). The mixture was extracted with ethyl acetate (30 mL*3). The organic phases were combined, washed with saturated brine (30 mL*1), dried over anhydrous sodium sulfate, and filtered. The filtrate was concentrated under reduced pressure to yield a powdery residue, which was purified by column chromatography (mobile phase: petroleum ether/ethyl acetate = 85/15 (V/V)) to obtain 873 mg of the title compound.

LCMS m/z = 293.2 [M+H]+.

### d) Preparation of tert-butyl 4-hydroxy-5-(methoxy-d3)-7-methyl-1H-indole-1-carboxylate

Tert-butyl 4-formyl-5-(methoxy-d3)-7-methyl-1H-indole-1-carboxylate (800 mg), tetrahydrofuran (5 mL), and methanol (15 mL) were added to a reaction flask. The mixture was cooled in an ice-water bath. Hydrogen peroxide (5 mL) and sulfuric acid (0.5 mL) were added dropwise in sequence. The mixture was further stirred under the ice-water bath for 2.5 hours, then a saturated sodium sulfite solution was slowly added to quench the reaction. The mixture was extracted with ethyl acetate (30 mL*3). The organic phases were combined, washed with saturated brine (30 mL*1), dried over anhydrous sodium sulfate, and filtered. The filtrate was concentrated under reduced pressure to yield a powdery residue, which was purified by column chromatography (mobile phase: petroleum ether/ethyl acetate = 90/10 (V/V)) to obtain 723 mg of the enantiomer of the title compound.

### e) Preparation of tert-butyl 4-(((3R,4R)-1-(tert-butoxycarbonyl)-3-(4-(methoxycarbonyl)phenyl)piperidin-4-yl)oxy)-5-(methoxy-d3)-7-methyl-1H-indole-1-carboxylate

The enantiomers of (±)-rel-(3S,4R)-tert-butyl 4-hydroxy-3-(4-(methoxycarbonyl)phenyl)piperidine-1-carboxylate were resolved by a chiral chromatography column, obtaining (3S,4R)-tert-butyl 4-hydroxy-3-(4-(methoxycarbonyl)phenyl) piperidine-1-carboxylate (tr = 20.33 min) and (3R,4S)-tert-butyl 4-hydroxy-3-(4-(methoxycarbonyl)phenyl)piperidine-1-carboxylate (tr = 16.51 min). The absolute stereochemical configuration of (3R,4S)-tert-butyl 4-hydroxy-3-(4-(methoxycarbonyl)phenyl) piperidine-1-carboxylate was confirmed by X-ray single crystal diffraction in Experimental Example 8. (The separation method: chromatographic column: DAICEL CHIRALPAK^{®} IC 250*4.6 mm, 5 µm; mobile phase A: n-hexane; mobile phase B: ethanol; A:B = 90:10; flow rate: 1 mL/min; detection wavelength: 254 nm).

(3S,4R)-tert-butyl 4-hydroxy-3-(4-(methoxycarbonyl)phenyl)piperidine-1-carboxylate

¹H NMR (400 MHz, DMSO-d6) δ 7.88 (d, J = 8.3 Hz, 2H), 7.42 (d, J = 8.3 Hz, 2H), 4.75 (d, J = 4.2 Hz, 1H), 4.00 (d, J = 14.8 Hz, 1H), 3.84 (s, 3H), 3.77 (d, J = 11.7 Hz, 2H), 3.34 (s, 1H), 3.16 (s, 1H), 2.82 (d, J = 11.1 Hz, 1H), 1.69 (dd, J = 19.9, 3.0 Hz, 2H), 1.39 (s, 9H).

(3R,4S)- tert-butyl 4-hydroxy-3-(4-(methoxycarbonyl)phenyl)piperidine-1-carboxylate

¹H NMR (400 MHz, DMSO-d6) δ 7.88 (d, J = 8.3 Hz, 2H), 7.42 (d, J = 8.3 Hz, 2H), 4.75 (d, J = 4.1 Hz, 1H), 4.00 (d, J = 14.8 Hz, 1H), 3.82 (d, J = 15.6 Hz, 3H), 3.76 (d, J = 11.9 Hz, 2H), 3.37 (s, 1H), 3.16 (s, 1H), 2.82 (d, J = 11.1 Hz, 1H), 1.81 - 1.59 (m, 2H), 1.39 (s, 9H).

Tert-butyl 4-hydroxy-5-(methoxy-d3)-7-methyl-1H-indole-1-carboxylate (200 mg), tert-butyl (3R,4S)-4-hydroxy-3-(4-(methoxycarbonyl)phenyl)piperidine-1-carboxylate (250.8 mg), and triphenylphosphine (187.7 mg) were added to a 50 mL three-necked flask. The mixture was purged with nitrogen for protection. Anhydrous tetrahydrofuran (20 mL) was added. Diisopropyl azodicarboxylate (144.7 mg) was added dropwise under an ice-water bath. The reaction system was slowly warmed to room temperature and stirred overnight. The reaction was quenched by adding water (20 mL). The mixture was extracted with ethyl acetate (20 mL*3). The organic phases were combined, washed with saturated brine (20 mL*1), dried over anhydrous sodium sulfate, and filtered. The filtrate was concentrated under reduced pressure to yield a powdery residue, which was purified by column chromatography (mobile phase: petroleum ether/ethyl acetate = 90/10 (V/V)) to obtain 171 mg of the title compound.

### f) Preparation of tert-butyl 5-(methoxy-d3)-4-(((3R,4R)-3-(4-(methoxycarbonyl)phenyl)piperidin-4-yl)oxy)-7-methyl-1H-indole-1-carboxylate

Tert-butyl 4-(((3R,4R)-1-(tert-butoxycarbonyl)-3-(4-(methoxycarbonyl)phenyl)piperidin-4-yl)oxy)-5-(methoxy-d3)-7-methyl-1H-indole-1-carboxylate (171 mg) was dissolved in 1,4-dioxane (2 mL). A solution of hydrochloric acid in 1,4-dioxane (1 mL, 4 M) was added. The mixture was purged with nitrogen for protection. The mixture was stirred at room temperature for 3.5 hours. Saturated sodium bicarbonate solution (10 mL) was added to the reaction solution. The mixture was extracted with ethyl acetate (20 mL*3). The organic phases were combined, washed with saturated brine (20 mL*1), dried over anhydrous sodium sulfate, and filtered. The filtrate was concentrated under reduced pressure to obtain 141 mg of the title compound.

LCMS m/z = 498.3 [M+H]+.

### g) Preparation of tert-butyl 4-(((3R,4R)-1-(2,2-difluoroethyl)-3-(4-(methoxycarbonyl)phenyl)piperidin-4-yl)oxy)-5-(methoxy-d3)-7-methyl-1H-indole-1-carboxylate

Tert-butyl 5-(methoxy-d3)-4-(((3R,4R)-3-(4-(methoxycarbonyl)phenyl)piperidin-4-yl)oxy)-7-methyl-1H-indole-1-carboxylate (75 mg) was added to a 50 mL reaction flask. Tetrahydrofuran (5 mL) and N,N-diisopropylethylamine (78.8 mg) were added. The mixture was purged with nitrogen for protection. 2,2-Difluoroethyl trifluoromethanesulfonate (97 mg) was added, and the mixture was stirred at 50 °C overnight. The reaction solution was concentrated to dryness under reduced pressure to yield a powdery residue, which was purified by column chromatography (mobile phase: petroleum ether/ethyl acetate = 90/10 (V/V)) to obtain 61 mg of the title compound.

### h) Preparation of 4-((3R,4R)-1-(2,2-difluoroethyl)-4-((5-(methoxy-d3)-7-methyl-1H-indol-4-yl)oxy)piperidin-3-yl) benzoic acid

Tert-butyl 4-(((3R,4R)-1-(2,2-difluoroethyl)-3-(4-(methoxycarbonyl)phenyl)piperidin-4-yl)oxy)-5-(methoxy-d3)-7-methyl-1H-indole-1-carboxylate (60 mg) was dissolved in tetrahydrofuran/methanol (2 mL/2 mL). An aqueous solution of lithium hydroxide (1 mL, 2 N) was added. The mixture was stirred at 70 °C for 3.5 hours and then concentrated to dryness under reduced pressure. Dilute hydrochloric acid aqueous solution was added to adjust the pH to neutral. The mixture was extracted with ethyl acetate (10 mL*3). The organic phases were combined, washed with saturated brine (20 mL*1), dried over anhydrous sodium sulfate, and filtered. The filtrate was concentrated under reduced pressure to yield a powdery residue, which was purified by column chromatography (mobile phase: dichloromethane/methanol = 20/1 (V/V)) to obtain 25.2 mg of the title compound.

¹H NMR (400 MHz, DMSO-d6) δ 12.80 (s, 1H), 10.83 (s, 1H), 7.91 (d, J = 8.2 Hz, 2H), 7.58 (d, J = 8.2 Hz, 2H), 7.17 (s, 1H), 6.62 (s, 1H), 6.17 (s, 1H), 6.33 - 5.96 (m, 1H), 4.45 (dd, J = 15.1, 9.0 Hz, 1H), 3.13 (td, J = 10.2, 3.8 Hz, 1H), 2.93 (dd, J = 23.5, 11.9 Hz, 2H), 2.78 (t, J = 15.5 Hz, 2H), 2.36 (s, 3H), 2.32 - 2.18 (m, 1H), 2.08 - 1.91 (m, 1H), 1.70 (s, 2H).

LCMS m/z = 448.2 [M+H]+.

### Example 27: 4-((3R,4R)-4-((5-(methoxy-d3)-7-methyl-1H-indol-4-yl)oxy)-1-(2,2,2-trifluoroethyl)piperidin-3-yl) benzoic acid

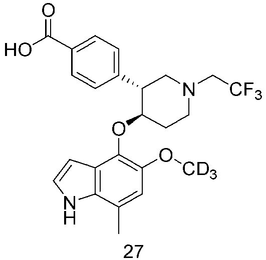

It was prepared by referring to the preparation method of Example 26, and 2,2-difluoroethyl trifluoromethanesulfonate in step g) was replaced with 2,2,2-trifluoroethyl trifluoromethanesulfonate.

¹H NMR (400 MHz, DMSO-d6) δ 12.76 (s, 1H), 10.84 (s, 1H), 7.91 (d, J = 7.9 Hz, 2H), 7.58 (d, J = 7.9 Hz, 2H), 7.17 (s, 1H), 6.62 (s, 1H), 6.18 (s, 1H), 4.49 - 4.45 (m, 1H), 3.26 (d, J = 10.1 Hz, 3H), 3.15 (d, J = 10.0 Hz, 1H), 2.95 (t, J = 13.0 Hz, 2H), 2.68 (t, J = 11.1 Hz, 1H), 2.36 (s, 3H), 1.71 (s, 2H).

LCMS m/z = 466.2 [M+H]+.

### Example 28: 4-((3R,4R)-4-((5-(difluoromethoxy)-7-methyl-1H-indol-4-yl)oxy)-1-(2,2,2-trifluoroethyl)piperidin-3 -yl)benzoic acid

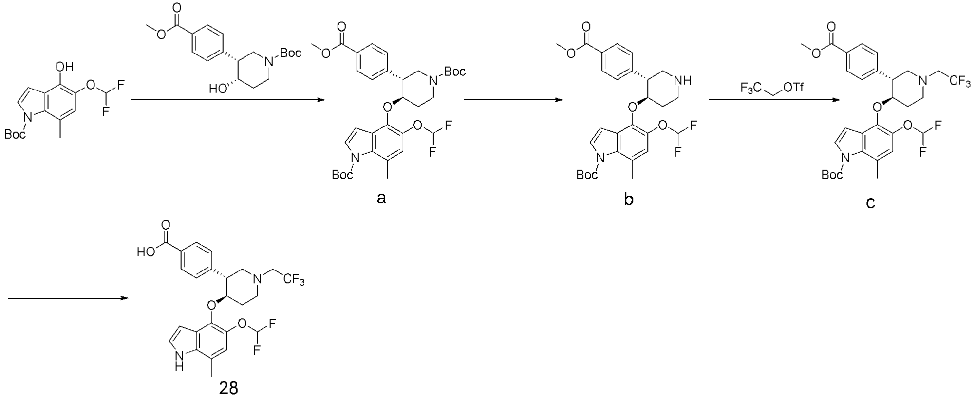

### a) Preparation of tert-butyl 4-(((3R,4R)-1-(tert-butoxycarbonyl)-3-(4-(methoxycarbonyl)phenyl)piperidin-4-yl)oxy)-5-(difluoromethoxy)-7-methyl-1H-indole-1-carboxylate

Tert-butyl 5-(difluoromethoxy)-4-hydroxy-7-methyl-1H-indole-1-carboxylate (80 mg), tert-butyl (3R,4S)-4-hydroxy-3-(4-(methoxycarbonyl)phenyl)piperidine-1-carboxylate (172 mg), and triphenylphosphine (134 mg) were added to a 25 mL three-necked flask. The mixture was purged with nitrogen for protection. Anhydrous tetrahydrofuran (10 mL) was added. Diisopropyl azodicarboxylate (104 mg) was added dropwise under an ice-water bath. The reaction system was slowly warmed to room temperature and stirred overnight. The reaction was quenched by adding water (20 mL). The mixture was extracted with ethyl acetate (20 mL*3). The organic phases were combined, washed with saturated brine (20 mL*1), dried over anhydrous sodium sulfate, and filtered. The filtrate was concentrated under reduced pressure to yield a powdery residue, which was purified by column chromatography (mobile phase: petroleum ether/ethyl acetate = 90/10 (V/V)) to obtain 80 mg of the title compound.

LCMS m/z = 631.3 [M+H]+.

### b) Preparation of tert-butyl 5-(difluoromethoxy)-4-(((3R,4R)-3-(4-(methoxycarbonyl)phenyl)piperidin-4-yl)oxy)-7-methyl-1H-indole-1-carboxylate

Tert-butyl 4-(((3R,4R)-1-(tert-butoxycarbonyl)-3-(4-(methoxycarbonyl)phenyl)piperidin-4-yl)oxy)-5-(difluoromethoxy) -7-methyl-1H-indole-1-carboxylate (80 mg) was dissolved in 1,4-dioxane (5 mL). A solution of hydrochloric acid in 1,4-dioxane (5 mL, 4 M) was added. The mixture was purged with nitrogen for protection. The mixture was stirred at room temperature for 3.5 hours. Saturated sodium bicarbonate solution (10 mL) was added to the reaction solution. The mixture was extracted with ethyl acetate (20 mL*3). The organic phases were combined, washed with saturated brine (20 mL*1), dried over anhydrous sodium sulfate, and filtered. The filtrate was concentrated under reduced pressure to obtain 65 mg of the title compound.

LCMS m/z = 531 [M+H]+.

### c) Preparation of tert-butyl 5-(difluoromethoxy)-4-(((3R,4R)-3-(4-(methoxycarbonyl)phenyl)-1-(2,2,2-trifluoroethyl) piperidin-4-yl)oxy)-7-methyl-1H-indole-1-carboxylate

Tert-butyl 5-(difluoromethoxy)-4-(((3R,4R)-3-(4-(methoxycarbonyl)phenyl)piperidin-4-yl)oxy)-7-methyl-1H-indole-1-carboxylate (37 mg) was added to a 50 mL reaction flask. Tetrahydrofuran (5 mL) and N,N-diisopropylethylamine (36 mg) were added. The mixture was purged with nitrogen for protection. 2,2,2-Trifluoroethyl trifluoromethanesulfonate (49 mg) was added, and the mixture was stirred at 50 °C for 2 hours. The reaction solution was concentrated to dryness under reduced pressure to yield a powdery residue, which was purified by column chromatography (mobile phase: petroleum ether/ethyl acetate = 90/10 (V/V)) to obtain 28 mg of the title compound.

### d) 4-((3R,4R)-4-((5-(difluoromethoxy)-7-methyl-1H-indol-4-yl)oxy)-1-(2,2,2-trifluoroethyl)piperidin-3-yl)benzoic acid

Tert-butyl 5-(difluoromethoxy)-4-(((3R,4R)-3-(4-(methoxycarbonyl)phenyl)-1-(2,2,2-trifluoroethyl)piperidin-4-yl)oxy) -7-methyl-1H-indole-1-carboxylate (28 mg) was dissolved in tetrahydrofuran/methanol (5 mL/5 mL). An aqueous solution of lithium hydroxide (5 mL, 2 N) was added. The mixture was stirred at 70 °C for 2 hours and then concentrated to dryness under reduced pressure. Dilute hydrochloric acid aqueous solution was added to adjust the pH to neutral. The mixture was extracted with ethyl acetate (10 mL*3). The organic phases were combined, washed with saturated brine (20mL*1), dried over anhydrous sodium sulfate, and filtered. The filtrate was concentrated under reduced pressure to yield a powdery residue, which was purified by column chromatography (mobile phase: dichloromethane/methanol = 20/1 (V/V)) to obtain 12 mg of the title compound.

¹H NMR (400 MHz, DMSO-d6) δ 12.83 (s, 1H), 11.17 (s, 1H), 7.89 (d, J = 8.3 Hz, 2H), 7.57 (d, J = 8.3 Hz, 2H), 7.29 (t, J = 2.8 Hz, 1H), 6.94-6.56 (t, 1H), 6.67 (s, 1H), 6.28 - 6.21 (m, 1H), 4.56 (td, J = 9.5, 4.8 Hz, 1H), 3.24 (d, J = 10.2 Hz, 1H), 3.19 - 3.11 (m, 1H), 2.96 (t, J = 12.7 Hz, 2H), 2.74 (t, J = 11.4 Hz, 1H), 2.36 (s, 3H), 1.80 - 1.66 (m, 2H), 1.23 (s, 2H).

LCMS m/z = 499 [M+H]+.

### Example 29: 4-((3R,4R)-1-(2,2-difluoroethyl)-4-((5-(difluoromethoxy)-7-methyl-1H-indol-4-yl)oxy)piperidin-3-yl) benzoic acid

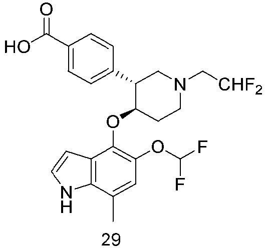

It was prepared by referring to the preparation method of Example 28, and 2,2,2-trifluoroethyl trifluoromethanesulfonate in step g) was replaced with 2,2-difluoroethyl trifluoromethanesulfonate.

¹H NMR (400 MHz, DMSO-d6) δ 12.77 (s, 1H), 11.17 (s, 1H), 7.89 (d, J = 8.3 Hz, 2H), 7.57 (d, J = 8.3 Hz, 2H), 7.28 (t, J = 2.8 Hz, 1H), 6.94-6.56 (t, 1H),6.67 (s, 1H), 6.29-6.01 (tt, J = 4.2 Hz, 1H), 6.22 (dd, J = 2.9, 2.0 Hz, 1H), 4.53 (td, J = 9.7, 5.0 Hz, 1H), 3.14 (td, J = 10.4, 3.9 Hz, 1H), 2.94 (dd, J = 20.9, 10.8 Hz, 2H), 2.79 (td, J = 15.9, 3.0 Hz, 2H), 2.56 (t, J = 11.3 Hz, 1H), 2.36 (s, 3H), 1.74 (d, J = 3.7 Hz, 1H), 1.25 (d, J = 9.2 Hz, 2H).

LCMS m/z = 481 [M+H]+.

### Example 30: 4-((3R,4R)-1-(2,2-difluoroethyl)-4-((7-methyl-5-(trifluoromethoxy)-1H-indol-4-yl)oxy)piperidin-3-yl)benzoic acid

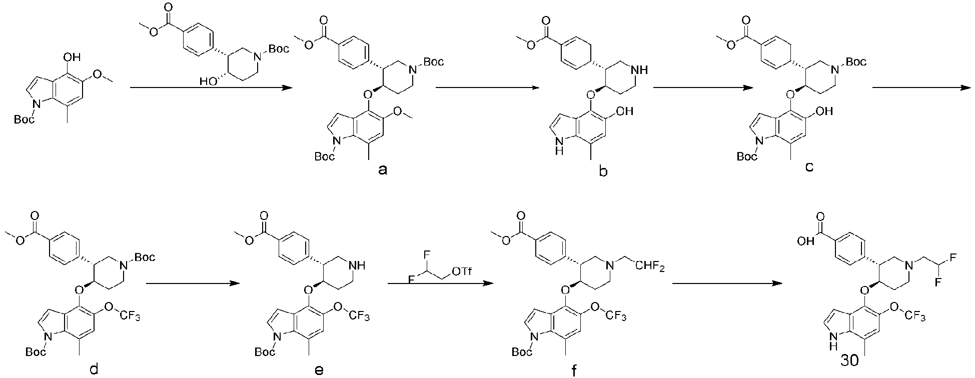

### a) Preparation of tert-butyl 4-(((3R,4R)-1-(tert-butoxycarbonyl)-3-(4-(methoxycarbonyl)phenyl)piperidin-4-yl)oxy) -5-methoxy-7-methyl-1H-indole-1-carboxylate

Tert-butyl 4-hydroxy-5-methoxy-7-methyl-1H-indole-1-carboxylate (1.49 g), tert-butyl (3R,4S)-4-hydroxy-3-(4-(methoxycarbonyl)phenyl)piperidine-1-carboxylate (1.2 g), and triphenylphosphine (1.13 g) were added to a 100 mL three-necked flask. The mixture was purged with nitrogen for protection. Anhydrous tetrahydrofuran (40 mL) was added. Diisopropyl azodicarboxylate (869 mg) was added dropwise under an ice-water bath. The reaction system was slowly warmed to room temperature and stirred overnight. The reaction was quenched by adding water (20 mL). The mixture was extracted with ethyl acetate (20 mL*3). The organic phases were combined, washed with saturated brine (20 mL*1), dried over anhydrous sodium sulfate, and filtered. The filtrate was concentrated under reduced pressure to yield a powdery residue, which was purified by column chromatography (mobile phase: petroleum ether/ethyl acetate = 85/15 (V/V)) to obtain 1.1 g of the title compound.

### b) Preparation of methyl 4-((3R,4R)-4-((5-hydroxy-7-methyl-1H-indol-4-yl)oxy)piperidin-3-yl)benzoate

Tert-butyl 4-(((3R,4R)-1-(tert-butoxycarbonyl)-3-(4-(methoxycarbonyl)phenyl)piperidin-4-yl)oxy)-5-methoxy-7-methyl-1H-indole-1-carboxylate (1.1 g) was added to a reaction flask. The mixture was purged with nitrogen for protection. Dichloromethane (20 mL) was added. Boron tribromide (187.7 mg) was added dropwise under an ice-water bath. The reaction system was slowly warmed to room temperature and the reaction continued for about 6 hours. Triethylamine was added dropwise to the reaction mixture under an ice-water bath until the reaction became weakly basic. The reaction solution was concentrated to dryness under reduced pressure to obtain 0.8 g of the title compound.

LCMS m/z = 381.2 [M+H]+.

### c) Preparation of tert-butyl 4-(((3R,4R)-1-(tert-butoxycarbonyl)-3-(4-(methoxycarbonyl)phenyl)piperidin-4-yl)oxy)-5-hydroxy-7-methyl-1H-indole-1-carboxylate

5-Hydroxy-7-methyl-1H-indole-4-carbaldehyde (0.8 g) was added to dichloromethane (40 mL). Then di-tert-butyl dicarbonate (2.0 g) and 4-dimethylaminopyridine (102 mg) were added. The mixture was purged with nitrogen for protection. The mixture was stirred at room temperature overnight. Water and ethyl acetate were added for extraction and separation. The organic phase was dried over anhydrous sodium sulfate and filtered. The filtrate was concentrated under reduced pressure to yield a powdery residue, which was purified by column chromatography (mobile phase: petroleum ether/ethyl acetate = 85/15 (V/V)) to obtain 447 mg of the title compound.

### d) Preparation of tert-butyl 4-(((3R,4R)-1-(tert-butoxycarbonyl)-3-(4-(methoxycarbonyl)phenyl)piperidin-4-yl)oxy) -7-methyl-5-(trifluoromethoxy)-1H-indole-1-carboxylate

Tert-butyl 4-(((3R,4R)-1-(tert-butoxycarbonyl)-3-(4-(methoxycarbonyl)phenyl)piperidin-4-yl)oxy)-5-hydroxy-7-methyl-1H-indole-1-carboxylate (447 mg), 2,8-difluoro-5-(trifluoromethyl)-5H-dibenzo[B,D]thiophen-5-ium trifluoromethanesulfonate (405 mg), and potassium carbonate (159.6 mg) were added to a reaction flask, followed by the addition of N,N-dimethylformamide (20 mL). The mixture was stirred at room temperature overnight. The reaction was quenched by adding water (20 mL). The mixture was extracted with ethyl acetate (20 mL*3). The organic phases were combined, washed with saturated brine (20mL*1), dried over anhydrous sodium sulfate, and filtered. The filtrate was concentrated under reduced pressure to yield a powdery residue, which was purified by column chromatography (mobile phase: petroleum ether/ethyl acetate = 90/10 (V/V)) to obtain 158 mg of the title compound.

### e) Tert-butyl 4-(((3R,4R)-3-(4-(methoxycarbonyl)phenyl)piperidin-4-yl)oxy)-7-methyl-5-(trifluoromethoxy)-1H-indole -1-carboxylate

Tert-butyl 4-(((3R,4R)-1-(tert-butoxycarbonyl)-3-(4-(methoxycarbonyl)phenyl)piperidin-4-yl)oxy)-7-methyl-5-(trifluoromethoxy)-1H-indole-1-carboxylate (158 mg) was dissolved in 1,4-dioxane (5 mL). A solution of hydrochloric acid in 1,4-dioxane (5 mL, 4 M) was added. The mixture was purged with nitrogen for protection. The mixture was stirred at room temperature for 3.5 hours. Saturated sodium bicarbonate solution (10 mL) was added to the reaction solution. The mixture was extracted with ethyl acetate (20 mL*3). The organic phases were combined, washed with saturated brine (20 mL*1), dried over anhydrous sodium sulfate, and filtered. The filtrate was concentrated under reduced pressure to obtain 105 mg of the title compound.

### f) Preparation of tert-butyl 4-(((3R,4R)-1-(2,2-difluoroethyl)-3-(4-(methoxycarbonyl)phenyl)piperidin-4-yl)oxy)-7-methyl-5-(trifluoromethoxy)-1H-indole-1-carboxylate

Tert-butyl 4-(((3R,4R)-3-(4-(methoxycarbonyl)phenyl)piperidin-4-yl)oxy)-7-methyl-5-(trifluoromethoxy)-1H-indole -1-carboxylate (65 mg) was added to a 50 mL reaction flask. Tetrahydrofuran (5 mL) and N,N-diisopropylethylamine (61 mg) were added. The mixture was purged with nitrogen for protection. 2,2-Difluoroethyl trifluoromethanesulfonate (75.8 mg) was added, and the mixture was stirred at 50 °C overnight. The reaction solution was concentrated to dryness under reduced pressure to yield a powdery residue, which was purified by column chromatography (mobile phase: petroleum ether/ethyl acetate = 80/20 (V/V)) to obtain 49 mg of the title compound.

### g)4-((3R,4R)-1-(2,2-Difluoroethyl)-4-((7-methyl-5-(trifluoromethoxy)-1H-indol-4-yl)oxy)piperidin-3-yl)benzoic acid

Tert-butyl 4-(((3R,4R)-1-(2,2-difluoroethyl)-3-(4-(methoxycarbonyl)phenyl)piperidin-4-yl)oxy)-7-methyl-5-(trifluoromethoxy)-1H-indole-1-carboxylate (49 mg) was dissolved in tetrahydrofuran/methanol (5 mL/5 mL). An aqueous solution of lithium hydroxide (5 mL, 2 N) was added. The mixture was stirred at 70 °C for 3.5 hours and then concentrated to dryness under reduced pressure. Dilute hydrochloric acid aqueous solution was added to adjust the pH to neutral. The mixture was extracted with ethyl acetate (10 mL*3). The organic phases were combined, washed with saturated brine (20 mL*1), dried over anhydrous sodium sulfate, and filtered. The filtrate was concentrated under reduced pressure to yield a powdery residue, which was purified by column chromatography (mobile phase: dichloromethane/methanol = 20/1 (V/V)) to obtain 18.3 mg of the title compound.

¹H NMR (400 MHz, DMSO-d6) δ 12.92 (s, 1H), 10.93 (s, 1H), 8.00 (d, J = 8.4 Hz, 2H), 7.67 (d, J = 8.4 Hz, 2H), 7.25 (t, J = 2.8 Hz, 1H), 6.72 (s, 1H), 6.44 - 6.02 (m, 2H), 4.45 (dd, J = 16.1, 9.5 Hz, 1H), 3.23 (td, J = 10.7, 4.2 Hz, 1H), 3.03 (dd, J = 21.3, 10.7 Hz, 2H), 2.85 (dd, J = 33.4, 21.7 Hz, 2H), 2.43 (d, J = 17.8 Hz, 3H), 2.39 (d, J = 14.4 Hz, 1H), 1.80 (s, 2H), 1.33 (s, 1H).

LCMS m/z = 499.2[M+H]+.

### Example 31: 4-((3R,4R)-4-((7-methyl-5-(trifluoromethoxy)-1H-indol-4-yl)oxy)-1-(2,2,2-trifluoroethyl)piperidin-3 -yl)benzoic acid

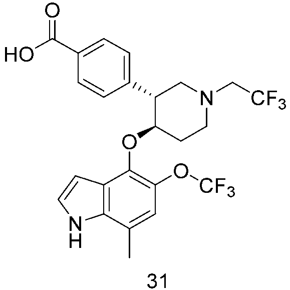

It was prepared by referring to the preparation method of Example 30, and 2,2-difluoroethyl trifluoromethanesulfonate in step f) was replaced with 2,2,2-trifluoroethyl trifluoromethanesulfonate.

¹H NMR (400 MHz, DMSO-d6) δ 12.90 (s, 1H), 10.93 (s, 1H), 8.00 (d, J = 8.1 Hz, 2H), 7.68 (d, J = 8.1 Hz, 2H), 7.27 (s, 1H), 6.72 (s, 1H), 6.28 (s, 1H), 4.58 (d, J = 7.4 Hz, 1H), 3.36 (d, J = 9.2 Hz, 3H), 3.24 (dd, J = 9.5, 6.8 Hz, 1H), 3.05 (t, J = 12.4 Hz, 2H), 2.78 (t, J = 11.1 Hz, 1H), 2.45 (s, 3H), 1.81 (s, 1H), 1.33 (s, 1H).

LCMS m/z = 517.2[M+H]+.

### Example 32: 4-((2R,5S)-5-ethoxy-2-((5-methoxy-7-methyl-1H-indol-4-yl)methyl)piperidin-1-yl)benzoic acid and 4-((2S,5R)-5-ethoxy-2-((5-methoxy-7-methyl-1H-indol-4-yl)methyl)piperidin-1-yl)benzoic acid and 4-((2S,5S)-5-ethoxy-2-((5-methoxy-7-methyl-1H-indol-4-yl)methyl)piperidin-1-yl)benzoic acid and 4-((2R,5R)-5-ethoxy-2-((5-methoxy-7-methyl-1H-indol-4-yl)methyl)piperidin-1-yl)benzoic acid

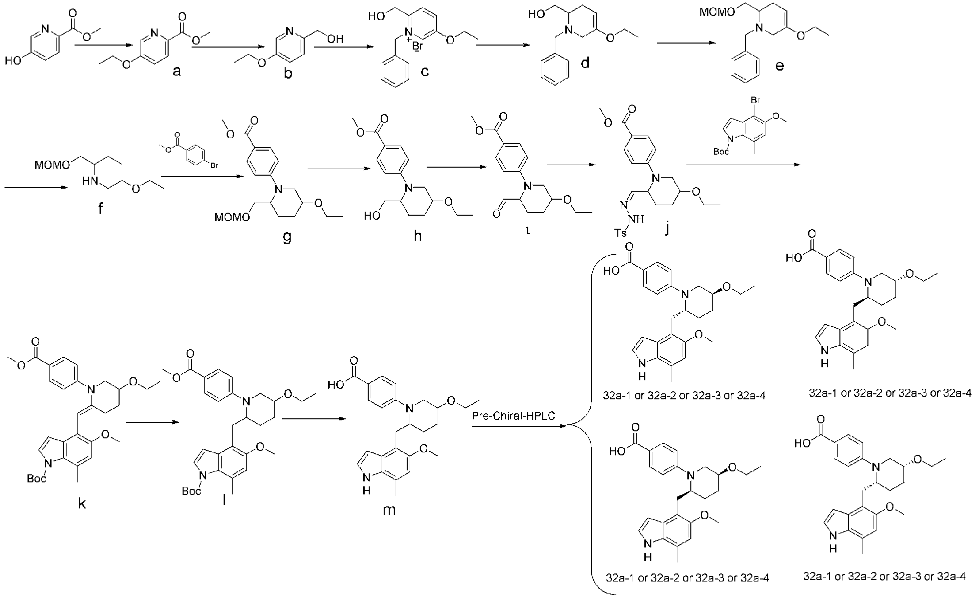

### a) Preparation of methyl 5-ethoxypyridine-2-carboxylate

Methyl 5-hydroxypyridine-2-carboxylate (49 g), ethyl iodide (54.90 g), and potassium carbonate (66 g) were added to a reaction flask, followed by the addition of N,N-dimethylformamide (490 mL). The mixture was purged with nitrogen for protection and stirred at room temperature overnight. The reaction was quenched by adding water. The mixture was extracted with ethyl acetate (5 × 100 mL). The organic phases were combined, dried over anhydrous sodium sulfate, and filtered. The filtrate was concentrated under reduced pressure to obtain 54 g of the title compound.

LCMS m/z = 182[M+H]+.

### b) Preparation of (5-ethoxypyridin-2-yl)methanol

Methyl 5-ethoxypyridine-2-carboxylate (54 g), sodium borohydride (33.82 g), and calcium chloride (46.6 g) were added to a reaction flask, followed by the addition of ethanol (540 mL). The mixture was stirred at room temperature overnight. The reaction solution was concentrated under reduced pressure to obtain a residue. Water was added, and the mixture was extracted with ethyl acetate (3 × 100 mL). The organic phases were combined, dried over anhydrous sodium sulfate, and filtered. The filtrate was concentrated under reduced pressure to obtain 36.9 g of the title compound.

LCMS m/z = 154[M+H]+.

### c) Preparation of 1-benzyl-5-ethoxy-2-(hydroxymethyl)pyridin-1-ium bromide

(5-Ethoxypyridin-2-yl)methanol (34.4 g) and benzyl bromide (57.61 g) were added to a reaction flask, followed by the addition of acetonitrile (344 mL). The mixture was stirred at 90 °C overnight. The mixture was cooled to room temperature. The precipitated solid was collected by filtration and washed with ethyl acetate (3 × 20 mL) to obtain 64 g of the title compound.

LCMS m/z = 244[M+H]+.

### d) Preparation of (1-benzyl-5-ethoxy-3,6-dihydro-2H-pyridin-2-yl)methanol

1-Benzyl-5-ethoxy-2-(hydroxymethyl)pyridin-1-ium bromide (40 g) was added to a reaction flask. A mixed solution of methanol/tetrahydrofuran (200 mL/200 mL) was added. The temperature was cooled to -60 °C, and sodium borohydride (11.6 g) was added. Then the temperature was slowly warmed to room temperature and the mixture was stirred for 2 hours. The reaction was quenched by adding water (200 mL). The mixture was extracted with ethyl acetate (200 mL*3). The organic phases were combined, washed with saturated brine (200 mL*1), dried over anhydrous sodium sulfate, and filtered. The filtrate was concentrated under reduced pressure to yield a powdery residue, which was purified by column chromatography (mobile phase: dichloromethane/methanol = 10/1 (V/V)) to obtain 29 g of the title compound.

LCMS m/z = 248[M+H]+.

### e) Preparation of 1-benzyl-5-ethoxy-2-((methoxymethoxy)methyl)-1,2,3,6-tetrahydropyridine

(1-Benzyl-5-ethoxy-3,6-dihydro-2H-pyridin-2-yl)methanol (28 g) was added to a reaction flask, followed by the addition of tetrahydrofuran (311 mL). Sodium hydride (13.58 g) was added under an ice-bath, and the mixture was stirred at 0 °C for 30 minutes. Then bromo(methoxy)methane (16.98 g) was added dropwise. The reaction was slowly warmed to room temperature and stirred for 2 hours. The reaction was quenched by adding water/ice (20 mL). The mixture was extracted with ethyl acetate (30 mL*3). The organic phases were combined, washed with saturated brine (30 mL*1), dried over anhydrous sodium sulfate, and filtered. The filtrate was concentrated under reduced pressure to yield a powdery residue, which was purified by column chromatography (mobile phase: petroleum ether/ethyl acetate = 1/1 (V/V)) to obtain 29 g of the title compound.

LCMS m/z = 292[M+H]+.

### f) Preparation of 5-ethoxy-2-((methoxymethoxy)methyl)piperidine

1-Benzyl-5-ethoxy-2-((methoxymethoxy)methyl)-1,2,3,6-tetrahydropyridine (29 g) and palladium on carbon (2.86 g) were added to a reaction flask, followed by the addition of methanol (193 mL). The mixture was purged with hydrogen and stirred at room temperature for 3 hours. Then it was filtered, and the filter cake was washed with methanol (3 × 5 mL). The filtrate was concentrated to dryness under reduced pressure to obtain 16.2 g of the title compound.

LCMS m/z = 204[M+H]+.

### g) Preparation of methyl 4-(5-ethoxy-2-((methoxymethoxy)methyl)piperidin-1-yl)benzoate

5-Ethoxy-2-((methoxymethoxy)methyl)piperidine (5 g), methyl 4-bromobenzoate (4.23 g), tris(dibenzylideneacetone)dipalladium(0) (2.55 g), 2-dicyclohexylphosphino-2',6'-diisopropoxy-1,1'-biphenyl (4.59 g), and cesium carbonate (24.04 g) were added to a reaction flask, followed by the addition of toluene (50 mL). The mixture was purged with nitrogen for protection and stirred at 100 °C for 24 hours. The reaction solution was concentrated under reduced pressure to yield a powdery residue, which was purified by column chromatography (mobile phase: petroleum ether/ethyl acetate = 1/1 (V/V)) to obtain 5.6 g of the title compound.

LCMS m/z = 338[M+H]+.

### h) Preparation of methyl 4-(5-ethoxy-2-(hydroxymethyl)piperidin-1-yl)benzoate

Methyl 4-(5-ethoxy-2-((methoxymethoxy)methyl)piperidin-1-yl)benzoate (5 g) was dissolved in methanol (20 mL). A solution of hydrogen chloride in 1,4-dioxane (4 M, 20.0 mL) was added. The mixture was stirred at room temperature for 2 hours. The reaction solution was concentrated to dryness under reduced pressure to obtain 3 g of the title compound.

LCMS m/z = 294[M+H]+.

### i) Preparation of methyl 4-(5-ethoxy-2-formylpiperidin-1-yl)benzoate

Methyl 4-(5-ethoxy-2-(hydroxymethyl)piperidin-1-yl)benzoate (4 g) and Dess-Martin periodinane (8.67 g) were added to a reaction flask, followed by the addition of dichloromethane (40.0 mL). The mixture was stirred at room temperature for 2 hours. Then it was filtered, and the filter cake was washed with dichloromethane (3 × 10 mL). The filtrate was concentrated under reduced pressure to yield a powdery residue, which was purified by column chromatography (mobile phase: petroleum ether/ethyl acetate = 5/1 (V/V)) to obtain 3 g of the title compound.

LCMS m/z = 292[M+H]+.

### j) Preparation of methyl (Z)-4-(5-ethoxy-2-((2-tosylhydrazineylidene)methyl)piperidin-1-yl)benzoate

Methyl 4-(5-ethoxy-2-formylpiperidin-1-yl)benzoate (2 g) and p-toluenesulfonylhydrazide (1.4 g) were dissolved in methanol (20 mL). The mixture was stirred at room temperature overnight. The reaction solution was concentrated under reduced pressure to yield a powdery residue, which was purified by column chromatography (mobile phase: petroleum ether/ethyl acetate = 1/1 (V/V)) to obtain 2.2 g of the title compound.

LCMS m/z = 460 [M+H]+.

### k) Preparation of tert-butyl (E)-4-((5-ethoxy-1-(4-(methoxycarbonyl)phenyl)piperidin-2-ylidene)methyl)-5-methoxy-7-methyl-1H-indole-1-carboxylate

Methyl (Z)-4-(5-ethoxy-2-((2-tosylhydrazineylidene)methyl)piperidin-1-yl)benzoate (600 mg), tert-butyl 4-bromo-5-methoxy-7-methylindole-1-carboxylate (666.2 mg)), tris(dibenzylideneacetone)dipalladium(0) (135.1 mg), 2-dicyclohexylphosphino-2',4',6'-triisopropylbiphenyl (62.2 mg), and lithium tert-butoxide (313.5 mg) were added to a reaction flask, followed by the addition of 1,4-dioxane (10 mL). Under nitrogen protection, the mixture was stirred at 100 °C for 3 hours. Water was added, and the mixture was extracted with ethyl acetate (20 mL*3). The organic phases were combined, washed with saturated brine (30 mL*1), dried over anhydrous sodium sulfate, and filtered. The filtrate was concentrated to dryness under reduced pressure to obtain 900 mg of the title compound.

LCMS m/z = 535[M+H]+.

### l) Preparation of tert-butyl 4-((5-ethoxy-1-(4-(methoxycarbonyl)phenyl)piperidin-2-yl)methyl)-5-methoxy-7-methyl -1H-indole-1-carboxylate

### Tert-butyl

(E)-4-((5-ethoxy-1-(4-(methoxycarbonyl)phenyl)piperidin-2-ylidene)methyl)-5-methoxy-7-methyl-1H-indole-1-carboxylate (900 mg), sodium cyanoborohydride (126.9 mg), acetic acid (1.35 mL), and methanol (13.5 mL) were added to a reaction flask. The mixture was stirred at room temperature for 20 hours, then concentrated under reduced pressure to yield a powdery residue, which was purified by column chromatography (mobile phase: petroleum ether/ethyl acetate = 1/1 (V/V)) to obtain 80 mg of the title compound.

LCMS m/z = 537 [M+H]+.

### m) Preparation of 4-(5-ethoxy-2-((5-methoxy-7-methyl-1H-indol-4-yl)methyl)piperidin-1-yl)benzoic acid

### Tert-butyl

4-((5-ethoxy-1-(4-(methoxycarbonyl)phenyl)piperidin-2-yl)methyl)-5-methoxy-7-methyl-1H-indole-1-carboxylate (80 mg) was added to a reaction flask, followed by the addition of an aqueous solution of lithium hydroxide (1.8 mL, 2 N) and tetrahydrofuran (1 mL). The mixture was stirred at 70 °C for 30 hours. The residue was acidified to pH 4 with concentrated sulfuric acid. The precipitated solid was collected by filtration, and the filter cake was washed with water (3 × 5 mL) to obtain 32 mg of the title compound.

The mixture of isomers was resolved by chiral SFC, obtaining 32a-1, tr = 5.47 min and 32a-2, tr = 7.57 min, 32a-3, tr = 10.6 min and 32a-4, tr = 15.2 min (The resolution method: chromatographic column: CHIRALPAK-IG 2*25cm; mobile phase A: n-Hexane (0.1% formic acid); mobile phase B: Ethanol : Dichloromethane = 1: 1;A:B = 1:1; flow rate: 20 mL/min; detection wavelength: 220/254nm)

32a-1, tr = 5.47 min

¹H NMR (400 MHz, DMSO-d6) δ 10.86 (s, 1H), 7.82 - 7.73 (m, 2H), 7.30 (t, J = 2.8 Hz, 1H), 7.03 (d, J = 8.8 Hz, 2H), 6.71 (s, 1H), 6.44 (dd, J = 3.1, 1.9 Hz, 1H), 4.30 (s, 1H), 3.88 (d, J = 13.9 Hz, 1H), 3.82 (s, 3H), 3.67 (s, 1H), 3.49 - 3.41 (m, 2H), 3.21 (t, J = 11.8 Hz, 2H), 2.75 (dd, J = 12.7, 4.1 Hz, 1H), 2.46 - 2.39 (m, 3H), 2.08 (dd, J = 14.7, 11.4 Hz, 1H), 1.66 (q, J = 15.2, 14.4 Hz, 2H), 1.15 (d, J = 8.2 Hz, 1H), 1.05 (t, J = 7.0 Hz, 3H). Lack of active hydrogen.

32a-2, tr = 7.57 min

¹H NMR (400 MHz, DMSO-d6) δ 12.04 (s, 1H), 10.86 (s, 1H), 7.77 (d, J = 8.9 Hz, 2H), 7.30 (t, J = 2.8 Hz, 1H), 7.03 (d, J = 8.8 Hz, 2H), 6.71 (s, 1H), 6.49 - 6.40 (m, 1H), 4.30 (s, 1H), 3.88 (d, J = 14.3 Hz, 1H), 3.82 (s, 3H), 3.67 (s, 1H), 3.45 (dt, J = 7.0, 3.3 Hz, 2H), 3.19 (d, J = 11.8 Hz, 2H), 2.75 (dd, J = 12.8, 4.0 Hz, 1H), 2.43 (s, 3H), 2.08 (t, J = 13.3 Hz, 1H), 1.73 - 1.59 (m, 2H), 1.15 (d, J = 13.0 Hz, 1H), 1.05 (t, J = 7.0 Hz, 3H).

32a-3, tr = 10.6 min

¹H NMR (400 MHz, DMSO-d6) δ 12.12 (s, 1H), 10.87 (s, 1H), 7.79 (d, J = 8.6 Hz, 2H), 7.30 (t, J = 2.9 Hz, 1H), 7.05 (d, J = 8.8 Hz, 2H), 6.70 (s, 1H), 6.37 (t, J = 2.4 Hz, 1H), 4.28 (d, J = 10.2 Hz, 1H), 3.88 (dd, J = 12.4, 4.8 Hz, 1H), 3.80 (s, 3H), 3.60 (qd, J = 7.0, 4.9 Hz, 2H), 3.20 (t, J = 11.8 Hz, 1H), 2.93 (t, J = 11.4 Hz, 1H), 2.71 (dd, J = 12.8, 4.3 Hz, 2H), 2.43 (s, 3H), 1.87 (d, J = 11.0 Hz, 1H), 1.76 (p, J = 10.4 Hz, 1H), 1.49 - 1.37 (m, 1H), 1.16 (t, J = 6.9 Hz, 3H).

32a-4, tr = 15.2 min

¹H NMR (400 MHz, DMSO-d6) δ 12.15 (s, 1H), 10.87 (s, 1H), 7.84 - 7.72 (m, 2H), 7.30 (t, J = 2.8 Hz, 1H), 7.05 (d, J = 9.1 Hz, 2H), 6.70 (s, 1H), 6.37 (dd, J = 3.2, 1.9 Hz, 1H), 4.29 (d, J = 9.3 Hz, 1H), 3.88 (dd, J = 12.4, 4.8 Hz, 1H), 3.80 (s, 3H), 3.60 (qd, J = 6.9, 4.8 Hz, 2H), 3.18 (d, J = 11.7 Hz, 2H), 2.93 (t, J = 11.4 Hz, 1H), 2.71 (dd, J = 12.7, 4.1 Hz, 1H), 2.43 (s, 3H), 1.87 (d, J = 9.5 Hz, 1H), 1.76 (p, J = 11.6, 10.7 Hz, 1H), 1.43 (d, J = 5.6 Hz, 2H), 1.16 (t, J = 7.0 Hz, 3H).

### Example 33: 5-(4-((3R,4R)-4-((5-methoxy-7-methyl-1H-indol-4-yl)oxy)-1-(2,2,2-trifluoroethyl)piperidin-3-yl) phenyl)-1,3,4-oxadiazol-2(3H)-one

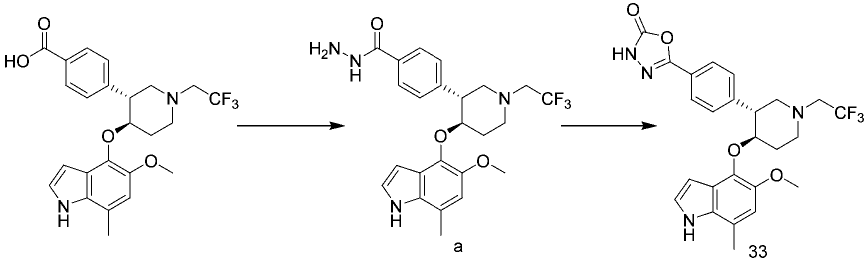

### a) Preparation of 4-((3R,4R)-4-((5-methoxy-7-methyl-1H-indol-4-yl)oxy)-1-(2,2,2-trifluoroethyl)piperidin-3-yl) benzohydrazide

4-((3R,4R)-4-(((5-Methoxy-7-methyl-1H-indol-4-yl)oxy)-1-(2,2,2-trifluoroethyl)piperidin-3-yl)benzoic acid (50 mg) was added to a reaction flask, followed by the addition of tetrahydrofuran (5 mL). N,N'-Carbonyldiimidazole (26.3 mg) was added, and the mixture was stirred at 50 °C for 2 hours. Then hydrazine hydrochloride (14.8 mg) and 1,8-diazabicyclo[5.4.0]undec-7-ene (41.2 mg) were added in sequence. The mixture was reacted at 50 °C overnight. After cooling, water was added. The mixture was extracted with ethyl acetate (20 mL*3). The organic phases were combined, washed with saturated brine (30 mL*1), dried over anhydrous sodium sulfate, and filtered. The filtrate was concentrated to dryness under reduced pressure to obtain 25 mg of the title compound.

LCMS m/z = 477.2[M+H]+.

### b) Preparation of 5-(4-((3R,4R)-4-((5-methoxy-7-methyl-1H-indol-4-yl)oxy)-1-(2,2,2-trifluoroethyl)piperidin-3-yl) phenyl)-1,3,4-oxadiazol-2(3H)-one

4-((3R,4R)-4-((5-Methoxy-7-methyl-1H-indol-4-yl)oxy)-1-(2,2,2-trifluoroethyl)piperidin-3-yl)benzohydrazide (25 mg) was added to a reaction flask, followed by the addition of acetonitrile (10 mL), N,N'-carbonyldiimidazole (12.8 mg), and 1,8-diazabicyclo[5.4.0]undec-7-ene (32 mg). The mixture was heated to 50 °C and reacted overnight. The reaction solution was concentrated under reduced pressure to yield a powdery residue, which was purified by column chromatography (mobile phase: dichloromethane/methanol = 90/10 (V/V)) to obtain 14.4 mg of the title compound.

¹H NMR (400 MHz, DMSO-d6) δ 12.43 (s, 1H), 10.83 (s, 1H), 7.76 (d, J = 8.2 Hz, 2H), 7.63 (d, J = 8.2 Hz, 2H), 7.17 (s, 1H), 6.63 (s, 1H), 6.20 (s, 1H), 4.47 (dd, J = 15.4, 8.5 Hz, 1H), 3.61 (s, 3H), 3.23 (d, J = 10.0 Hz, 2H), 3.14 (td, J = 10.1, 3.6 Hz, 1H), 3.02 - 2.87 (m, 2H), 2.69 (t, J = 11.2 Hz, 1H), 2.44 (s, 1H), 2.36 (s, 3H), 1.71 (d, J = 3.4 Hz, 2H).

LCMS m/z = 503.2[M+H]+.

### Experimental Example 1: TR-FRET assay method of human complement factor B

Competitive binding assays were performed using the Cy5 fluorescently labeled small-molecule inhibitor (+) or (-)-2-((1E,3E,5E)-5-(1-(6-((2-(3-(4-((R)-3-amino-3-phenylpropanoyl)-1-(4-amino-6,7-dimethoxyquinazolin-2-yl)piperazin-2-yl)phenoxy)ethyl)amino)-6-oxohexyl)-3,3-dimethyl-5-sulfoindolin-2-ylidene)penta-1,3-dien-1-yl)-1-ethyl-3,3-dimethyl-5-sulfo-3H-indol-1-ium (prepared according to Biological Example 2 of CN201480050471.1) as probes to test the inhibitory activity of the compounds against complement factor B. Complement factor B (ComplementTech, A135) and EZ-Link^{™} Sulfo-NHS-LC-LC-Biotin (Thermo, 21338) were incubated at a ratio of 1:20 on ice for 2 hours, and then 1 M Tris (pH 7.5) was added to terminate the reaction. Subsequently, the mixture was purified twice using a 2 mL Zeba^{™} desalt spin column (Thermo, 89890) to obtain biotin-labeled complement factor B. During the experiment, biotin-labeled complement factor B at a final concentration of 25 nM was incubated with compounds at different concentrations in a buffer (PBS containing 10 mM MgCl₂ and 0.05% Chaps) at 4 °C for 30 minutes. Then, a Cy5 fluorescently labeled probe and europium chelate-labeled streptavidin (PerkinElmer, AD0060) at final concentrations of 75 nM and 0.225 nM were added respectively, and the mixture was reacted at 4 °C for 2 hours. After the reaction, time-resolved fluorescence energy transfer (TR-FRET) data were read on a microplate reader (Tecan, SPARK; 337 nm excitation wavelength, 615 nm and 665 nm emission wavelengths), and the IC₅₀ values were determined. The test results were shown in Table 1 below.

Reference compound LNP023: synthesized according to Example 26 of CN20 1480050471.1, the structure thereof is as follows:

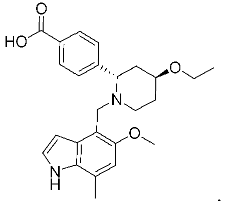

**Table 1. Inhibitory activity of the compounds against complement factor B**

| Example | IC₅₀ (nM) | Example | IC₅₀ (nM) | Example | IC₅₀ (nM) | Example | IC₅₀ (nM) |
|---|---|---|---|---|---|---|---|
| 1-2 | 89.1 | 13a-1 | 982 | 19a-2 | 223.5 | 28 | 45.97 |
| 3 | > 1000 | 13a-2 | 6 | 19a-4 | 257.4 | 29 | 31.22 |
| 4 | > 1000 | 14a-2 | 10.2 | 19b-2 | 8 | 30 | 72.15 |
| 5 | 202 | 15a-2 | 6.9 | 19b-4 | 178.4 | 31 | 426.6 |
| 6-1 | > 1000 | 15b-2 | 596.1 | 20a-4 | 12.2 | 32a-2 | 601.17 |
| 9 | 28.2 | 16-2 | 12.4 | 25-2 | 24.01 | 32a-4 | 285.1 |
| 10-2 | 117.5 | 16-4 | 195.7 | 26 | 12.16 | 33 | 23.71 |
| 11-2 | 70.7 | 17a-2 | 17.19 | 27 | 9.13 | LNP023 | 29.82 |

### Experimental Example 2: Complement deposition assay of serum alternative pathway

The Wieslab^{®} Complement system Alternative pathway kit (WIESLAB^{®} Complement system Alternative pathway AP330 RUO) was used to detect the inhibitory activity of the compounds on the alternative complement pathway in human serum. Human serum was diluted 18-fold with the kit diluent. The diluted serum was added to a 96-well plate at 130 µL/well. Using a compound titrator (Tecan, D300e), test compounds at corresponding concentrations were added. The test concentration of the compounds started at 10 µM, with 3-fold serial dilution for 6 concentration points, detected in single wells. The DMSO ratio was uniformly adjusted to 0.1% using DMSO in all drug-treated wells. In the positive control wells, 0.1% DMSO and 130 µL of diluted serum were added; in the negative control wells, 0.1% DMSO and 130 µL of the kit diluent were added. The mixtures were pre-incubated at room temperature for 15 min. The incubated mixtures were transferred to the 96-well plate provided by the kit at 100 µL per well, incubated at 37 °C for 60 minutes, and the liquid in the wells was discarded. 300 µL of the washing solution in the kit was added to each well. The wells were washed three times. 100 µL of the conjugated antibody Conjugate in the kit was added to each well, and it was incubated at room temperature for 30 minutes. The liquid in the wells was removed. 300 µL of the washing solution in the kit was added to each well and the wells were washed three times. 100 µL of the substrate solution was added to each well and incubated at room temperature for 30 minutes. It was detected by a microplate reader (Tecan, SPARK), the absorbance values at 405 nm were read. The experimental data were shown in Table 2 below.

**Table 2. Results of complement deposition in serum alternative pathway**

| Example | IC50 (nM) | Example | IC50 (nM) |
|---|---|---|---|
| 1-2 | 132.0 | 16-2 | 125.0 |
| 6-2 | 214.8 | 17a-2 | 68.2 |
| 13a-2 | 54.1 | 19b-2 | 103.6 |
| 14a-2 | 59.3 | 20a-4 | 86.7 |
| 15a-2 | 54.8 | LNP023 | 105.9 |

### Experimental Example 3: Pharmacokinetic study in mice

Purpose of the experiment: To investigate the plasma pharmacokinetics in male ICR (CD-1) mice after single intravenous administration and gavage administration of the compounds of the present disclosure.

Experimental animals: Male ICR (CD-1) mice, body weight 32-35 g; supplier: Vital River Laboratory Animal Technology Co., Ltd.

Experimental procedures: Intravenous Administration (IV); Oral Gavage (PO); the dose was 10 mg/kg (vehicle: water (containing 0.5% methylcellulose (w/v) and 0.5% Tween 80 (v/v)).

Sample collection: At each set time point, 40 µL of blood was collected from the orbital sinus of the experimental animals. Whole blood samples were placed in anticoagulant tubes containing EDTA-K2. The whole blood samples were centrifuged at 1500 g for 10 min to separate plasma. The upper-layer plasma samples were collected into sample tubes for LC-MS/MS analysis.

Data analysis: Plasma concentrations were processed using the non-compartmental model in WinNonlin^{™}Version6.3 (Pharsight, Mountain View, CA) pharmacokinetic software. Pharmacokinetic parameters Cl, T_{1/2}, Cₘₐₓ, and AUC₀₋₂₄ were calculated using the linear-log trapezoidal method, and the results were shown in the table below.

**Table 3. Results of the pharmacokinetic study**

| Compound | PO (10 mpk) | |
|---|---|---|
| | Cₘₐₓ (ng/mL) | AUC₀₋₂₄ (ng·h/mL) |
| Compound 13a-2 | 1290 | 9680 |
| Compound 14a-2 | 1370 | 6690 |
| Compound 16-2 | 601 | 5750 |
| Compound 17a-2 | 679 | 6480 |
| Compound 26 | 944 | 4960 |
| LNP023 | 1160 | 4110 |

### Experimental Example 4: Collagen antibody-induced mouse arthritis model

Purpose of the experiment: To investigate the efficacy of the compounds of the present disclosure on collagen antibody and lipopolysaccharide-induced arthritis (CAIA) in BALB/c mice.

Experimental animals: BALB/c mice, male, 6-8 weeks, 18-20 g; supplier: Beijing Vital River Laboratory Animal Technology Co., Ltd.

### Experimental procedures:

1. Induction of the mice CAIA Model: on Day 0, all mice were intravenously injected via the tail vein with 0.15 mL of Arthrogen-CIA 5-clone mixed type II collagen antibody (10 mg/mL, Chondrex); on Day 3, the mice were intraperitoneally injected with 0.2 mL of LPS (E. coli 0111: B4 lipopolysaccharide, 0.5 mg/mL; Chondrex).
2. Administration:

**Table 4. Administration groups and administration schemes**

| Group | Test article | N | Concentr ation mg/mL | Administration dose | | Admini stration route | Dosing regimen^{b} |
|---|---|---|---|---|---|---|---|
| | | | | mL/kg | mg/kg | | |
| 1 | Model control^{a} | 6 | N/A | 10 | N/A | Oral | Prophylactic administration: |
| 2 | LNP023 | 6 | 2 | 10 | 20 | Oral | |
| 3 | 13a-2 | 6 | 2 | 10 | 20 | Oral | administered 15 min after collagen antibody injection; |
| | | | | | | | administered 15 min after LPS injection on day 3; |
| | | | | | | | twice a day, 6h apart |

| | | | | | | | |
|---|---|---|---|---|---|---|---|
| *a: An aqueous solution of 0.5% methylcellulose (w*/*v) and 0.5% Tween 80 (v*/*v)* *b: On the day of sampling, only a single dose was administered in the morning, and the blood sampling and data analysis were completed within 4 hours after administration.* | | | | | | | |

The data were analyzed by One-way ANOVA/Dunnett's using Graphpad Prism and by Repeat Measurement ANOVA/Bonferroni using SPSS. *P<* 0.05 was considered to indicate a significant difference.

Arthritis scoring: Starting from the day of modeling, the incidence of arthritis in the limbs of the animals in each group was observed three times a week until the end of the experiment. According to the different degrees of lesions (erythema and swelling), the scoring was carried out according to the standard of 0-4 points. The scoring criteria are as follows: 0, no signs of erythema or swelling; 1, erythema or slight swelling of the midfoot (ankle); 2, erythema and slight swelling extending from the ankle to the midfoot; 3, erythema and moderate swelling from the ankle joint to the metatarsophalangeal joint; 4, erythema and severe swelling from the toes or fingers to the ankle joint or wrist joint.

Joint swelling: Starting from the day of modeling, the thickness of the left and right foot pads of the animals was measured three times a week using a micrometer (Mitutoyo, 0-25 mm, minimal 0.001 mm) until the end of the experiment.

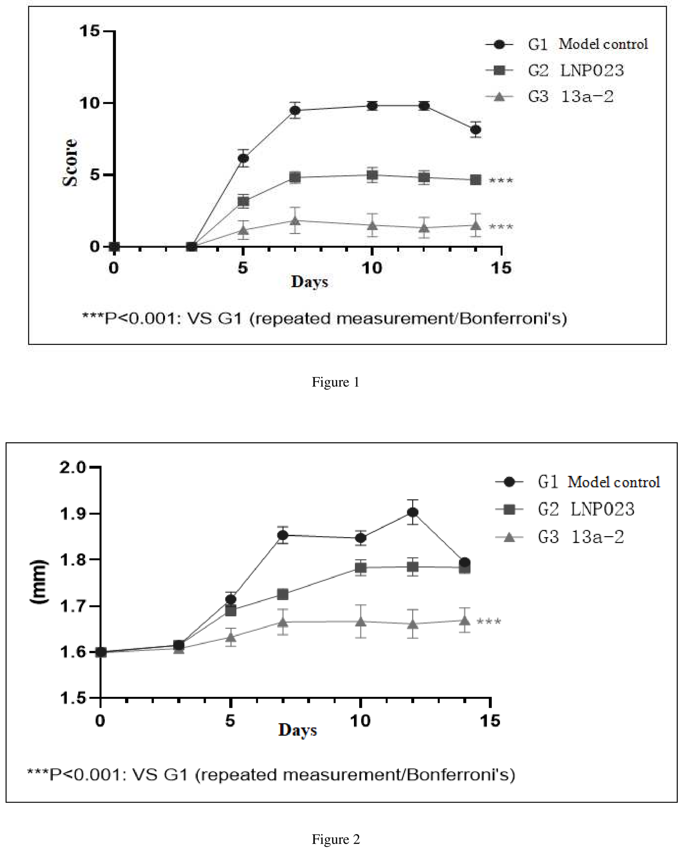

Experimental results: The experimental data were shown in Table 5, Table 6 and Figure 1, Figure 2.

**Table 5. Arthritis score**

| Group | | Day0 | Day3 | Day5 | Day7 | Day10 | Day12 | Day14 |
|---|---|---|---|---|---|---|---|---|
| Model control | Mean | 0.00 | 0.00 | 6.17 | 9.50 | 9.83 | 9.83 | 8.17 |
| | SEM | 0.00 | 0.00 | 0.60 | 0.56 | 0.31 | 0.31 | 0.54 |
| LNP023 | Mean | 0.00 | 0.00 | 3.17 | 4.83 | 5.00 | 4.83 | 4.67 |
| | SEM | 0.00 | 0.00 | 0.48 | 0.40 | 0.52 | 0.48 | 0.33 |
| 13a-2 | Mean | 0.00 | 0.00 | 1.17 | 1.83 | 1.50 | 1.33 | 1.50 |
| | SEM | 0.00 | 0.00 | 0.65 | 0.91 | 0.81 | 0.71 | 0.81 |

**Table 6. Thickness of foot pads (mm)**

| Group | | Day0 | Day3 | Day5 | Day7 | Day10 | Day12 | Day14 |
|---|---|---|---|---|---|---|---|---|
| Model control | Mean | 1.60 | 1.615 | 1.714 | 1.853 | 1.847 | 1.903 | 1.795 |
| | SEM | 0.00 | 0.005 | 0.015 | 0.018 | 0.016 | 0.027 | 0.007 |
| LNP023 | Mean | 1.60 | 1.614 | 1.690 | 1.725 | 1.783 | 1.785 | 1.783 |
| | SEM | 0.00 | 0.005 | 0.012 | 0.012 | 0.017 | 0.020 | 0.013 |
| 13a-2 | Mean | 1.60 | 1.607 | 1.632 | 1.665 | 1.666 | 1.661 | 1.669 |
| | SEM | 0.00 | 0.006 | 0.019 | 0.027 | 0.035 | 0.031 | 0.027 |

Experimental conclusion: According to the scoring results, compared with the model group, all the compounds of the present disclosure can significantly improve the degree of arthritis lesions in model animals, reduce joint swelling, and exhibit a better effect than LNP023.

### Experimental Example 5: Pharmacodynamic study of the test substance in the mouse CAIA model

Purpose of the experiment: Evaluate the efficacy of the test drug on collagen antibody and lipopolysaccharide-induced arthritis (CAIA) in BALB/c mice.

Experimental Animals: BALB/c mice, male, 6-8 weeks, 18-20 g; Supplier: Shanghai Jihui Laboratory Animal Care Co., Ltd

### Experimental Procedures:

1. Induction of the mouse CAIA model: On Day 0, all mice were intravenously injected via the tail vein with 0.15 mL of Arthrogen-CIA 5-clone mixed type II collagen antibody (10 mg/mL, Chondrex). On Day 3, the mice were intraperitoneally injected with 0.2 mL of LPS (E. coli 0111: B4 lipopolysaccharide, 0.5 mg/mL; Chondrex).
2. Administration:

**Table 7. Administration groups and administration schemes**

| **Gro up** | **Test article** | **N** | **Concent ration mg/mL** | **Administration dosage** | | **Administra tion route** | **Dosing regimen** |
|---|---|---|---|---|---|---|---|
| | | | | **mL/kg** | **mg/kg** | | |
| 1 | Model control^{a} | 6 | N/A | 10 | N/A | oral, Bid | Prophylactic administration; |
| 2 | LNP023 | 6 | 2 | 10 | 20 | oral, Bid | |
| 3 | 16-2 | 6 | 2 | 10 | 20 | oral, Bid | administered 15 min after collagen antibody injection; |
| 5 | 14a-2 | 6 | 2 | 10 | 20 | oral, Bid | |
| | | | | | | | administered 15 min after LPS injection on Day 3; |
| | | | | | | | (The administration interval of Bid is approximately 6h) |
| | | | | | | | Day0-Day14 |

| | | | | | | | |
|---|---|---|---|---|---|---|---|
| *a: An aqueous solution of 0.5% methylcellulose (w*/*v) and 0.5% Tween 80 (v*/*v)* | | | | | | | |

The experimental data are expressed as mean±standard error (mean ± S.E.M.). The data were analyzed by One-way ANOVA/Dunnett's using Graphpad Prism and by Repeat Measurement ANOVA/Bonferroni using SPSS. *P<* 0.05 was considered to indicate a significant difference.

Arthritis scoring: Starting from the day of modeling, the incidence of arthritis in the limbs of the animals in each group was observed three times a week until the end of the experiment. According to the different degrees of lesions (erythema and swelling), the scoring was carried out according to the standard of 0-4 points. The scoring criteria are as follows: 0, no signs of erythema or swelling; 1, erythema or slight swelling of the midfoot (ankle); 2, erythema and slight swelling extending from the ankle to the midfoot; 3, erythema and moderate swelling from the ankle joint to the metatarsophalangeal joint; 4, erythema and severe swelling from the toes or fingers to the ankle joint or wrist joint.

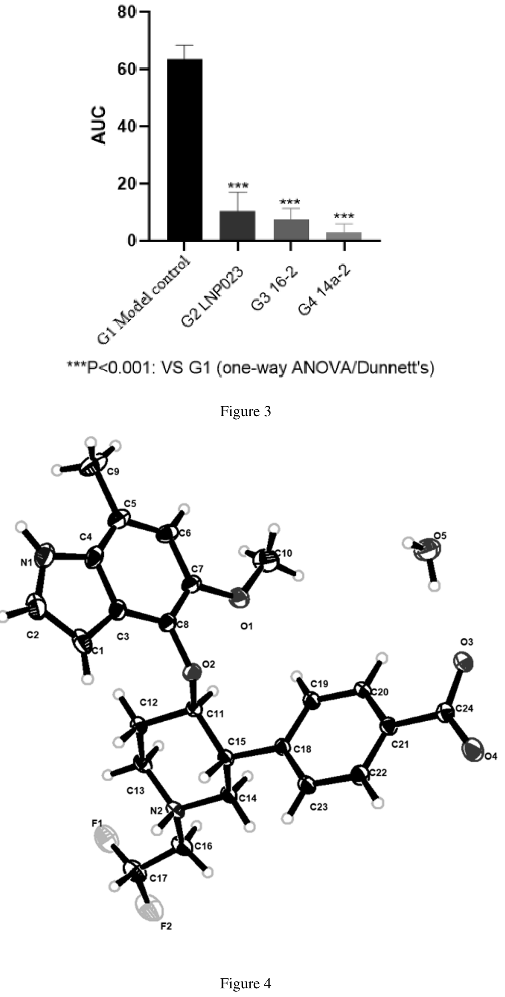

Score AUC: After the experiment was completed, by using GraphPad Prism 8.4.3 software, the mean arthritis score of each group of animals was taken to be analyzed with each time-point, the Area under curve in XY analyses was selected, and the arthritis score AUC of each group of animals was calculated. The larger the AUC, the greater the severity of arthritis was. The experimental results of AUC were shown in Table 8 and Figure 3.

**Table 8. Arthritis score AUC**

| Group | | AUC |
|---|---|---|
| G1 Model control | Mean | 63.25 |
| | SEM | 5.10 |
| LNP023 20mg/kg bid | Mean | 10.17 |
| | SEM | 6.56 |
| 16-2 20mg/kg bid | Mean | 7.25 |
| | SEM | 4.00 |
| 14a-2 20mg/kg bid | Mean | 3.00 |
| | SEM | 3.00 |

Experimental conclusion: According to the scoring results, compared with the model group, all the compounds of the present disclosure can significantly improve the degree of arthritis lesions in model animals, and the scoring AUC was significantly lower than that of LNP023.

### Experimental Example 6: X-ray single crystal diffraction experiment

Preparation Method: 5 mg of Compound 4-((3R,4R)-1-(2,2-difluoroethyl)-4-((5-methoxy-7-methyl-1H-indol-4-yl)oxy) piperidin-3-yl)benzoic acid (13a-2, tᵣ = 1.95min) were placed in a small glass vial. 0.6 mL mixed solvent of acetone/water (2:1, v/v) was added. After dissolving, the solution was filtered into another clean vial. The vial was sealed with parafilm and a small hole was pierced with a syringe needle. After allowing the solvent to evaporate at room temperature for 3 days, rod-shaped single crystal samples were obtained.

After the diffraction data were integrated and reduced using the SAINT program, the data were subjected to empirical absorption correction by the SADABS program. The single crystal structure was solved by the direct method using SHELXT2014, and the structure was refined using the least-squares method. The hydrogen atoms were obtained by isotropic calculation processing during the refinement process. The hydrogen atoms on C-H were obtained by computational hydrogenation and refined by the riding model. Figure 4 and Table 9 below is the single crystal data for the monohydrate of 4-((3R,4R)-1-(2,2-difluoroethyl)-4-((5-methoxy-7-methyl-1H-indol-4-yl)oxy)piperidin-3-yl)benzoic acid of Example 13a-2. The Flack parameter is 0.10 (9), and C11 and C15 have an R configuration.

**Table 9. Data of single crystal diffraction**

| | |
|---|---|
| C₂₄H₂₆F₂N₂O₄·H₂O | Dₓ = 1.353 Mg m⁻³ |
| Mᵣ = 462.48 | Cu Kα radiation, λ = 1.54178 Å |
| Orthorhombic, P2₁2₁2₁ | Cell parameters from 6580 reflections |
| a = 10.5507 (3) Å | θ= 4.5-74.6° |
| b = 11.7312 (4) Å | µ= 0.89 mm⁻¹ |
| c = 18.3406 (5) Å | T = 170 K |
| V = 2270.06 (12) Å³ | Block, colourless |
| Z = 4 | 0.12 × 0.08 × 0.05 mm |
| F(000) = 976 | |

### Experimental Example 7: X-ray single crystal diffraction experiment

Preparation Method: A saturated solution of compound 4-((3R,4R)-4-((5-methoxy-7-methyl-1H-indol-4-yl)oxy)-1-(2,2,2-trifluoroethyl)piperidin-3-yl)benzoic acid (14a-2, tr = 2.73 min) at high temperature (approximately 60 °C) was prepared in a 3 mL glass vial using acetonitrile (1 mL) as the solvent. While hot, the clear solution mentioned above was aspirated with a 2 mL syringe and filtered through a hydrophilic PTFE needle filter (13 mm*0.45 µm). The filtrate was transferred to another clean 3 mL glass vial, which was then left to stand at room temperature. Transparent single crystals were obtained after one day.

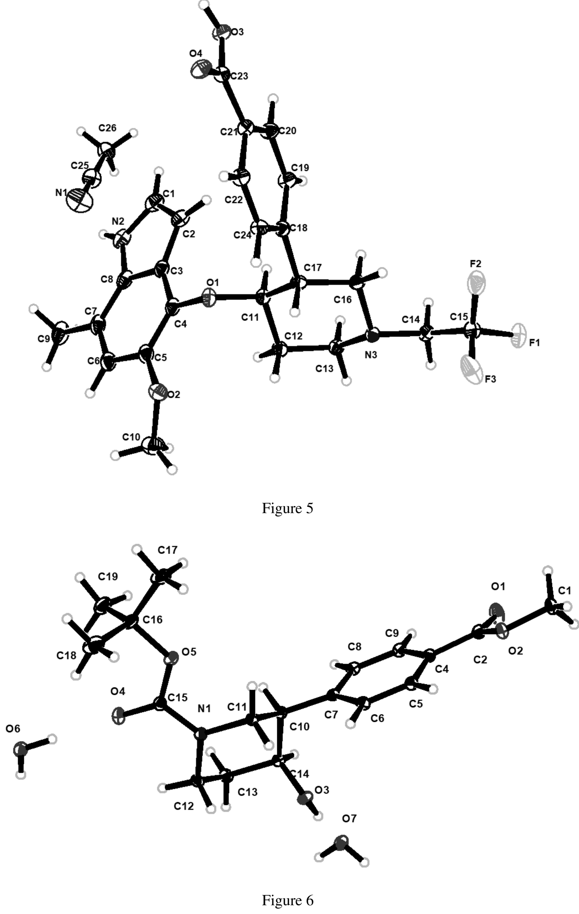

After the diffraction data were integrated and reduced using the SAINT program, the data were subjected to empirical absorption correction by the SADABS program. The single crystal structure was solved by the direct method using SHELXT2014, and the structure was refined using the least-squares method. The hydrogen atoms were obtained by isotropic calculation processing during the refinement process. The hydrogen atoms on C-H were obtained by computational hydrogenation and refined by the riding model. The Flack parameter is 0.09 (10), and C11 and C17 have an R configuration. Figure 5 and Table 10 below is the single crystal results for the acetonitrile solvate of 4-((3R,4R)-4-((5-methoxy-7-methyl-1H-indol-4-yl)oxy)-1-(2,2,2-trifluoroethyl)piperidin-3-yl)benzoic acid of Example 14a-2.

**Table 10. Data of single crystal diffraction**

| | |
|---|---|
| C₂₄H₂₅F₃N₂O₄·C₂H₃N | *D*ₓ = 1.308 Mg m⁻³ |
| *Mᵣ* = 503.51 | Cu *K*α radiation, λ = 1.54178 Å |
| Orthorhombic, *P*2₁2₁2₁ | Cell parameters from 9188 reflections |
| *a* = 10.6123 (4) Å | θ = 4.3-74.4° |
| *b* = 12.0219 (4) Å | µ = 0.87 mm⁻¹ |
| *c* = 20.0375 (6) Å | *T = 100 K* |
| *V*= 2556.39 (15) Å³ | Block, colourless |
| *Z* = 4 | 0.15 × 0.08 × 0.05 mm |
| *F*(000) = 1056 | |

### Experimental Example 8: X-ray single crystal diffraction experiment

Preparation Method: A saturated solution of compound (3S,4R)-tert-butyl 4-hydroxy-3-(4-(methoxycarbonyl)phenyl)piperidine-1-carboxylate (tr = 16.51 min) at high temperature (approximately 50 °C) was prepared using 1 milliliter of ethanol/water (1:2, v/v) as the solvent. The solution was then transferred to room temperature and left to stand. Transparent single crystals were obtained after one day.

After the diffraction data were integrated and reduced using the SAINT program, the data were subjected to empirical absorption correction by the SADABS program. The single crystal structure was solved by the direct method using SHELXT2014, and the structure was refined using the least-squares method. The hydrogen atoms were obtained by isotropic calculation processing during the refinement process. The hydrogen atoms on N and O were obtained by residual electron density. The hydrogen atoms on C-H were obtained by computational hydrogenation and refined by the riding model. The Flack parameter is 0.08(7), C10 has an R configuration and C14 has an S configuration. Figure 6 and Table 11 below is the single crystal results for the dihydrate of the intermediate compound (3R,4S)-tert-butyl 4-hydroxy-3-(4-(methoxycarbonyl)phenyl)piperidin-1-yl)carboxylate (tr = 16.51 min) obtained by resolution in Example 26, Step e).

**Table 11. Data of single crystal diffraction**

| | |
|---|---|
| C₁₈H₂₅NO₅·2(H₂O) | *D*ₓ = 1.227 Mg m⁻³ |
| *Mᵣ* = 371.42 | Cu *K*αradiation, λ = 1.54178 Å |
| Orthorhombic, *P*2₁2₁2₁ | Cell parameters from 9881 reflections |
| *a* = 6.4578 (2) Å | θ = 3.3-74.4° |
| *b* = 11.7232 (3) Å | µ= 0.78 mm⁻¹ |
| *c* = 26.5603 (6) Å | *T = 100 K* |
| *V* = 2010.78 (9) Å³ | Block, colourless |
| *Z=4* | 0.12 × 0.08 × 0.05 mm |
| *F(000)* = 800 | |

The foregoing description of the specific exemplary embodiments of the present disclosure is for the purpose of illustration and example. These descriptions are not intended to limit the present disclosure to the precise form disclosed, and it is obvious that many changes and variations can be made according to the above teachings. The purpose of selecting and describing the exemplary examples is to explain the specific principles of the present disclosure and their practical applications, so that those skilled in the art can implement and utilize various exemplary embodiments of the present disclosure as well as various selections and changes. The scope of the present disclosure is intended to be defined by the claims and equivalents thereof.

## Claims

1. A compound represented by General Formula (I) or an isomer or a pharmaceutically acceptable salt thereof: 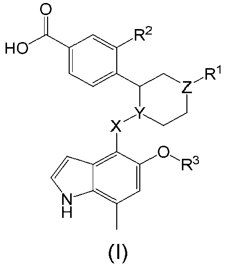 wherein:
X is selected from CH₂, NH, S, O or 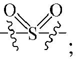
Y and Z are each independently selected from CH or N;
and when X is CH₂, Z is N;
R¹ is selected from C1-C6 alkyl, C1-C6 alkoxy, C3-C6 cycloalkyl or C3-C6 heterocyclic group, the C1-C6 alkyl or C3-C6 cycloalkyl is optionally substituted with halogen, oxo group, C1-C6 haloalkyl, C3-C6 cycloalkyl, C3-C6 heterocyclic group or C3-C6 halocycloalkyl;
R² is hydrogen, or R¹ and R² together with the atoms to which they are attached, form a C10-15 heterocyclic group, the heterocyclic group is optionally substituted with halogen;
R³ is selected from C1-C6 alkyl or C3-C6 cycloalkyl, the C1-C6 alkyl is optionally substituted with deuterium or halogen.

2. The compound or isomer or pharmaceutically acceptable salt thereof according to claim 1, wherein, the compound is as represented by General Formula (II-1), (II-2), or (II-3): 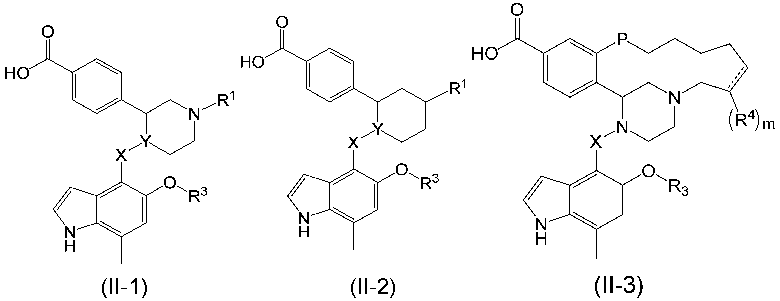 wherein:
X, Y, R¹, R³ are as defined in claim 1;
P is selected from CH₂, NH, S or O;
R⁴ is selected from hydrogen, halogen or C1-C3 alkyl;
m is 0, 1 or 2.

3. The compound or isomer or pharmaceutically acceptable salt thereof according to claim 1, wherein, the compound is as represented by General Formula (II-1-1) or (II-1-2): 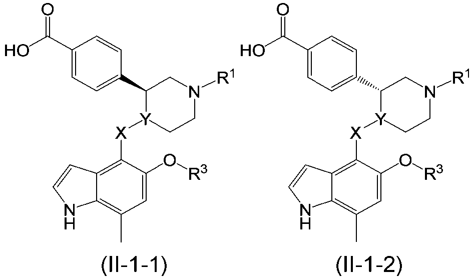 wherein:
X, Y, R¹, R³ are as defined in claim 1.

4. The compound or isomer or pharmaceutically acceptable salt thereof according to claim 1, wherein, the compound is as represented by General Formula (II-2-1) or (II-2-2): 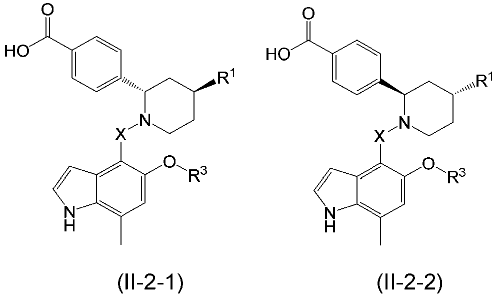 wherein:
X, Y, R¹, R³ are as defined in claim 1.

5. The compound or isomer or pharmaceutically acceptable salt thereof according to claim 1, wherein, the compound is as represented by General Formula (III-1) or (III-2): 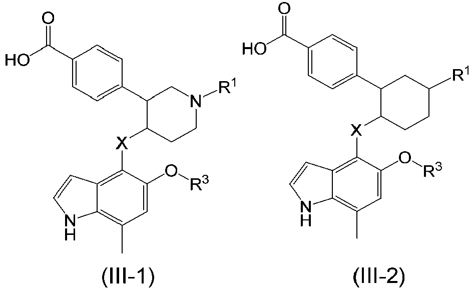 wherein:
X, R¹, R³ are as defined in claim 1;
preferably, the compound or isomer or pharmaceutically acceptable salt thereof is as represented by General Formula (III-1-1), (III-1-2), (III-1-3) or (III-1-4): 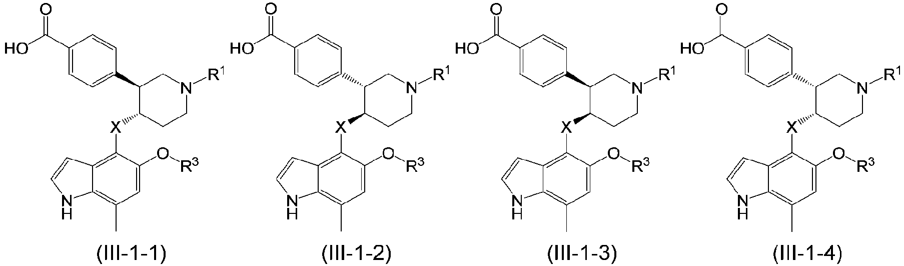 wherein:
X, R¹, R³ are as defined in claim 1.

6. The compound or isomer or pharmaceutically acceptable salt thereof according to claim 1, wherein, the compound is as represented by General Formula (III-2-1), (III-2-2), (III-2-3), (III-2-4), (III-2-5), (III-2-6), (III-2-7) or (III-2-8): 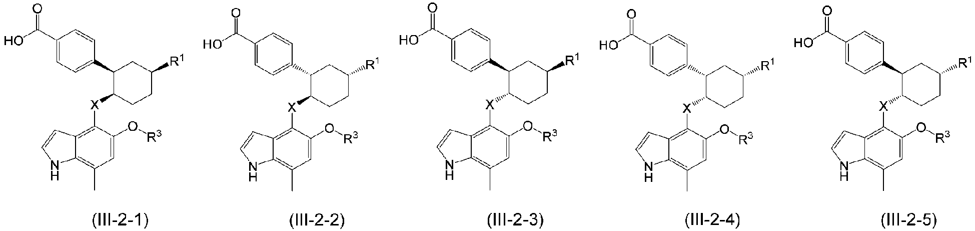 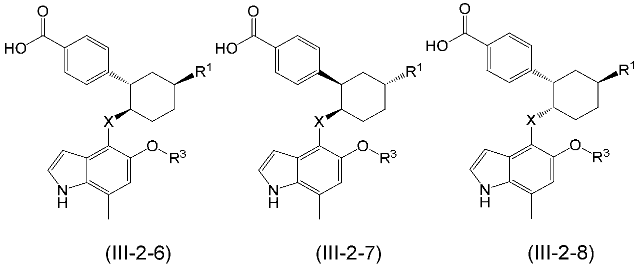 wherein:
X, R¹, R³ are as defined in claim 1.

7. The compound or isomer or pharmaceutically acceptable salt thereof according to any one of claims 1-6, wherein, X is selected from CH₂, S or O; preferably S or O;
Y is selected from CH or N; preferably CH;
Z is selected from CH or N; preferably N;
R¹ is selected from C1-C3 alkyl, C1-C3 alkoxy, C3-C4 cycloalkyl or C3-C4 heterocyclic group, the C1-C3 alkyl or C3-C4 cycloalkyl is optionally substituted with fluorine, chlorine, bromine, iodine, oxo group, C1-C3 haloalkyl, C3-C4 cycloalkyl, C3-C4 heterocyclic group or C3-C4 halocycloalkyl;
preferably, R¹ is selected from methyl, ethyl, propyl, ethoxy, cyclobutyl or oxetane, the ethyl, propyl or cyclobutyl is optionally substituted with fluorine, oxo group, trifluoromethyl, cyclopropyl, fluorocyclopropyl or oxetane; more preferably, R¹ is selected from 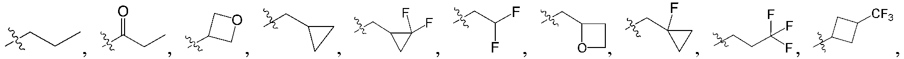 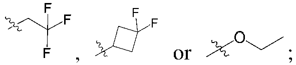 preferably 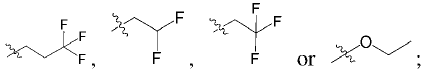 more preferably 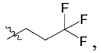 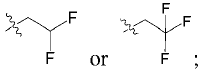 further preferably, R¹ is selected from 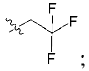
R³ is selected from C1-C3 alkyl or C3-C4 cycloalkyl, the C1-C3 alkyl is optionally substituted with deuterium, fluorine, chlorine, bromine or iodine; preferably, R³ is selected from methyl or cyclopropyl, the methyl is optionally substituted with deuterium or fluorine; more preferably, R³ is selected from methyl, -CD₃, 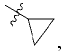 -CF₃ or 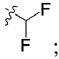 further preferably methyl;
P is selected from CH₂, NH, S or O; preferably O;
R⁴ is selected from fluorine, chlorine, bromine or iodine; preferably fluorine;
M is 0, 1 or 2; preferably 2.

8. The compound or isomer or pharmaceutically acceptable salt thereof according to any one of claims 1-6, wherein, X is selected from NH, S, O or 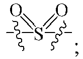
Y is selected from CH or N; preferably CH;
Z is selected from CH or N; preferably N;
R¹ is selected from C1-C3 alkyl, C1-C3 alkoxy, C3-C4 cycloalkyl or C3-C4 heterocyclic group, the C1-C3 alkyl or C3-C4 cycloalkyl is optionally substituted with fluorine, chlorine, bromine, iodine, oxo group, C1-C3 haloalkyl, C3-C4 cycloalkyl, C3-C4 heterocyclic group or C3-C4 halocycloalkyl;
preferably, R¹ is selected from methyl, ethyl, propyl, ethoxy, cyclobutyl or oxetane, the ethyl, propyl or cyclobutyl is optionally substituted with fluorine, oxo group, trifluoromethyl, cyclopropyl, fluorocyclopropyl or oxetane; more preferably, R¹ is selected from 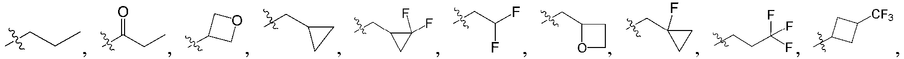 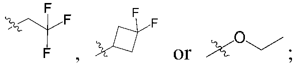 preferably 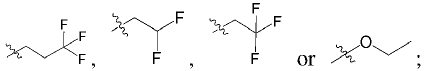 more preferably 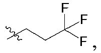 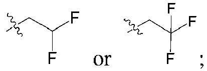 further preferably, R¹ is selected from 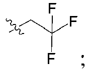
R³ is selected from C1-C3 alkyl or C3-C4 cycloalkyl, the C1-C3 alkyl is optionally substituted with deuterium, fluorine, chlorine, bromine or iodine; preferably, R³ is selected from methyl or cyclopropyl, the methyl is optionally substituted with deuterium or fluorine; more preferably, R³ is selected from methyl, -CD₃, 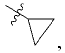 -CF₃ or 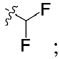 further preferably methyl;
P is selected from CH₂, NH, S or O; preferably O;
R⁴ is selected from fluorine, chlorine, bromine or iodine; preferably fluorine;
m is 0, 1 or 2; preferably 2.

9. The following compound or an isomer or a pharmaceutically acceptable salt thereof, wherein the compound has the following structure:

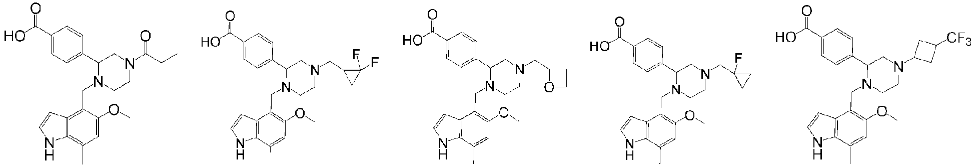

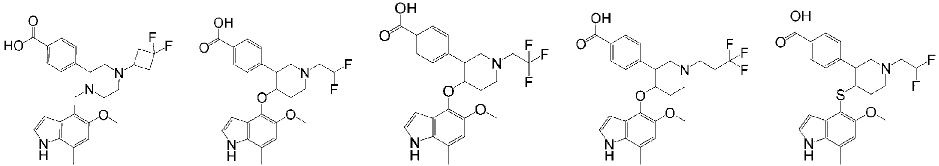

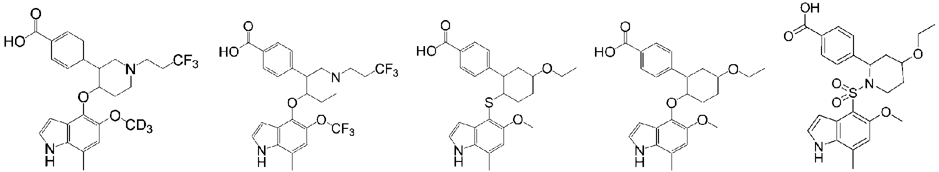

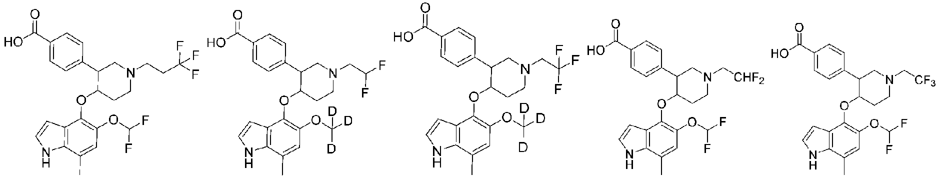

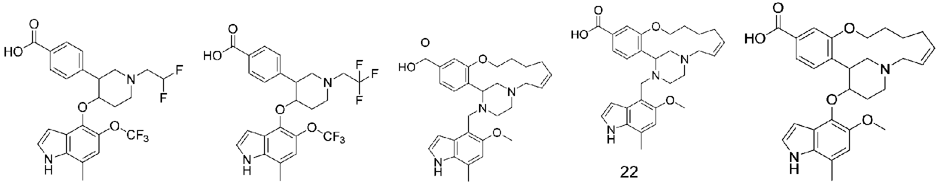

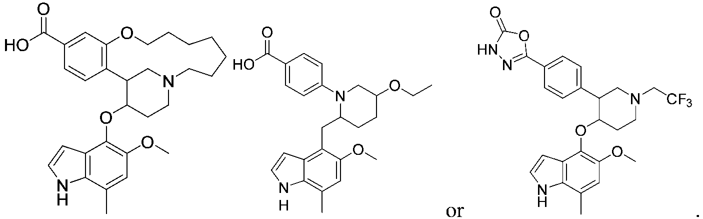

10. The following compound or an isomer or a pharmaceutically acceptable salt thereof, wherein the compound has the following structure: 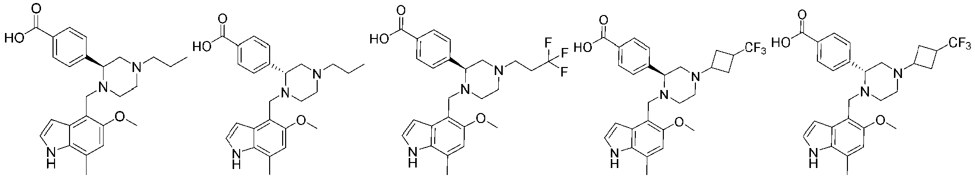 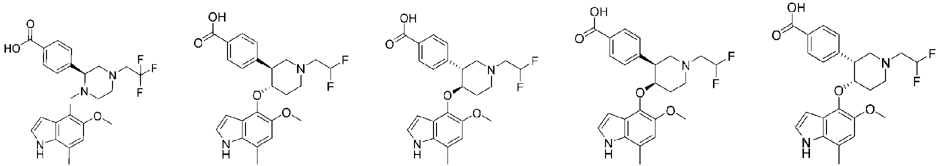 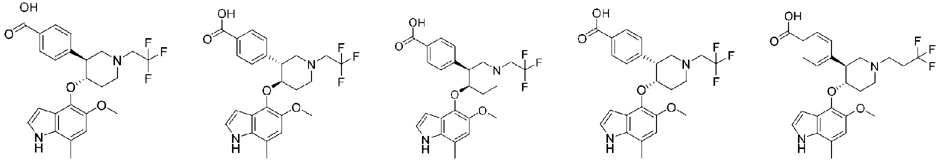 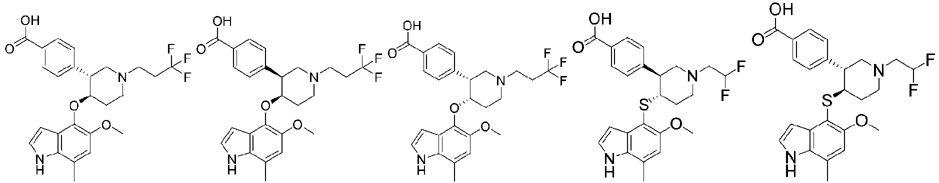 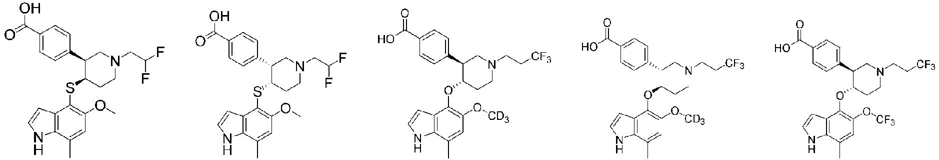 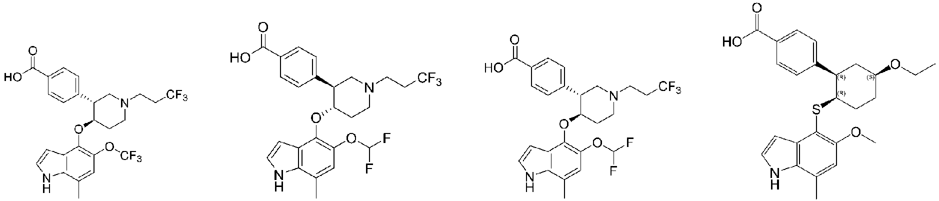 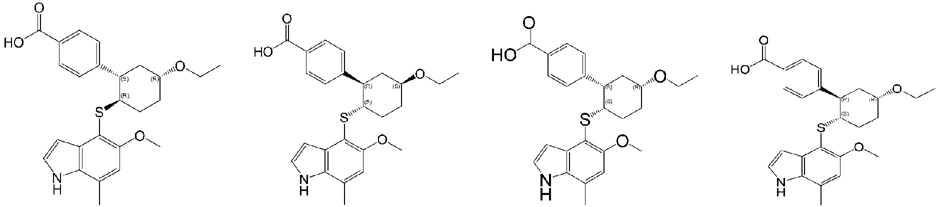 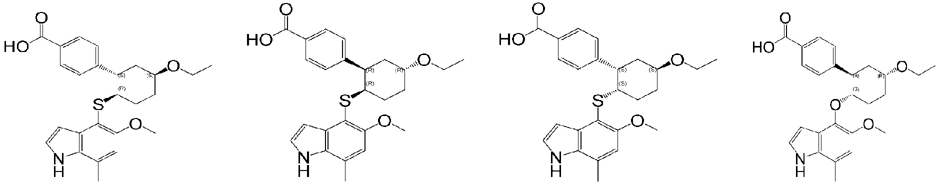 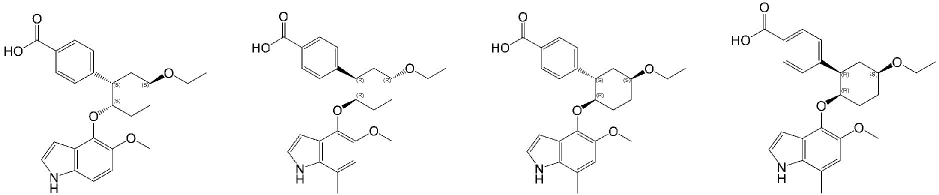 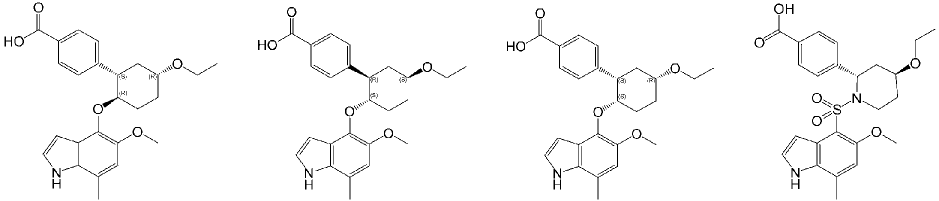 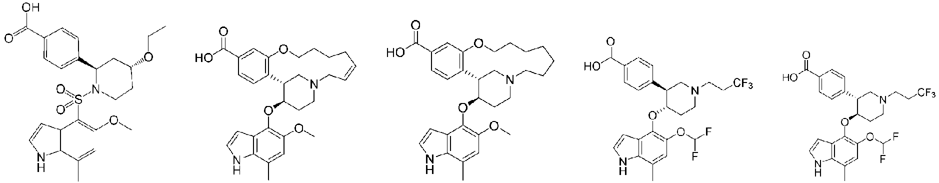 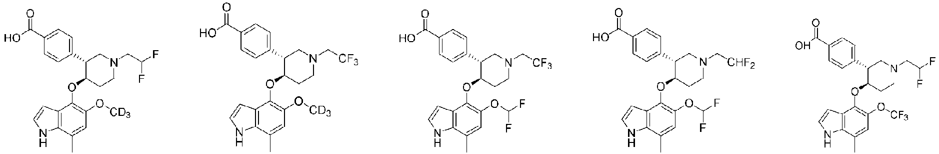 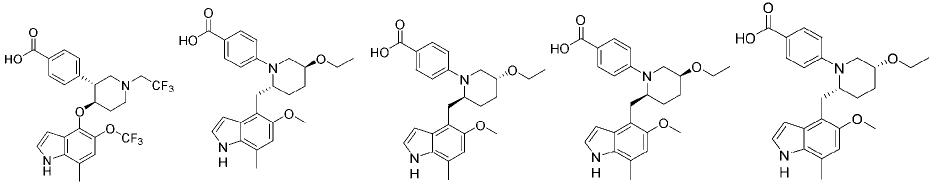 or

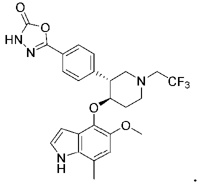

11. A pharmaceutical composition comprising a therapeutically effective amount of the compound or isomer or pharmaceutically acceptable salt thereof according to any one of claims 1-10, and one or more pharmaceutically acceptable carriers.

12. Use of the compound or isomer or pharmaceutically acceptable salt thereof according to any one of claims 1-10, and the pharmaceutical composition according to claim 11 in the manufacture of a medicament for preventing and/or treating a disease or a disease state mediated by complement factor B.

13. The use according to claim 12, wherein, the disease or disease state mediated by complement factor B is selected from one or more of an ophthalmic disease, an autoimmune disease, a disease related to the renal system, a disease of respiratory system, and a cardiovascular disease; and is preferably arthritis.
